(19) Europäisches Patentamt
European Patent Office
Office européen des brevets

(11) EP 0 591 102 B1

(12) **EUROPÄISCHE PATENTSCHRIFT**

(45) Veröffentlichungstag und Bekanntmachung des Hinweises auf die Patenterteilung:
**18.10.2000 Patentblatt 2000/42**

(51) Int. Cl.[7]: **C07D 307/83**, C07C 59/64, C08K 5/15

(21) Anmeldenummer: **93810651.5**

(22) Anmeldetag: **14.09.1993**

(54) **3-(2-Acyloxyethoxyphenyl)benzofuran-2-one als Stabilisatoren**
3-(2-Acyloxyethoxyphenyl)benzofuran-2-ones as stabilizers
3-(2-Acyloxyéthoxyphényl)benzofuran-2-ones en tant que stabilisateurs

(84) Benannte Vertragsstaaten:
**AT BE DE ES FR GB IT NL SE**

(30) Priorität: **23.09.1992 CH 297992**

(43) Veröffentlichungstag der Anmeldung:
**06.04.1994 Patentblatt 1994/14**

(73) Patentinhaber:
**Ciba Specialty Chemicals Holding Inc.**
**4057 Basel (CH)**

(72) Erfinder: **Nesvadba, Peter, Dr.**
**CH-1723 Marly (CH)**

(56) Entgegenhaltungen:
**EP-A- 0 078 241**
**EP-A- 0 415 887**
**WO-A-80/01566**
**GB-A- 2 257 140**
**GB-A- 2 257 141**

Bemerkungen:
Die Akte enthält technische Angaben, die nach dem Eingang der Anmeldung eingereicht wurden und die nicht in dieser Patentschrift enthalten sind.

EP 0 591 102 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft neue 3-(2-Acyloxyethoxyphenyl)benzofuran-2-one, Zusammensetzungen, enthaltend ein organisches Material, bevorzugt ein Polymer und die neuen Stabilisatoren, sowie die Verwendung derselben zum Stabilisieren von organischen Materialien gegen oxidativen, thermischen oder lichtinduzierten Abbau.

Einzelne 3-(Hydroxyphenyl)benzofuran-2-one und 3-(Acetoxyphenyl)benzofuran-2-one wurden beispielsweise von M. H. Hubacher, J. Org. Chem 24, 1949 (1959); J. Gripenberg et al, Acta Chemica Scandinavica 23, 2583 (1969); M. Auger et al, Bull. Soc. Chim. Fr. 1970, 4024 und J. Morvan et al, Bull. Soc. Chim. Fr. 1979, II-575 beschrieben.

Die Verwendung von einigen Benzofuran-2-onen als Stabilisatoren für organische Polymere ist beispielsweise aus WO-A-80/01566 oder EP-A-415 887 bekannt. Die PCT-Anmeldung WO-A-80/01566 führte zu den U.S. Patenten 4,325,863 und 4,338,244.

Es wurde nun gefunden, dass eine ausgewählte Gruppe solcher Benzofuran-2-one sich besonders gut als Stabilisatoren für organische Materialien, die gegen oxidativen, thermischen oder lichtinduzierten Abbau empfindlich sind, eignen.

Die vorliegende Erfindung betrifft daher Verbindungen der Formel (1)

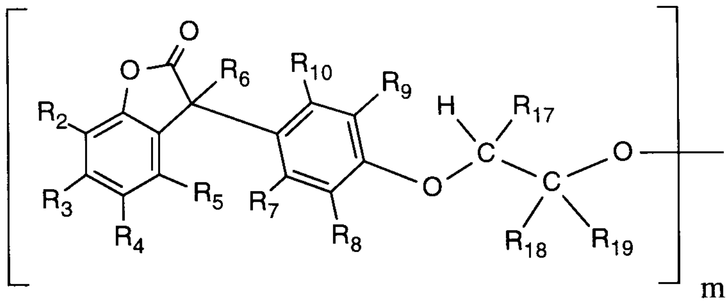

(1)

worin, wenn m 1 ist,

$R_1$ Wasserstoff, $C_1$-$C_{25}$-Alkanoyl, $C_3$-$C_{25}$-Alkenoyl, durch Sauerstoff, Schwefel oder >N-$R_{16}$ unterbrochenes $C_3$-$C_{25}$-Alkanoyl; durch eine Di($C_1$-$C_6$-alkyl)phosphonatgruppe substituiertes $C_2$-$C_{25}$-Alkanoyl; $C_6$-$C_9$-Cycloalkylcarbonyl, Thenoyl, Furoyl, Benzoyl oder durch $C_1$-$C_{12}$-Alkyl substituiertes Benzoyl;

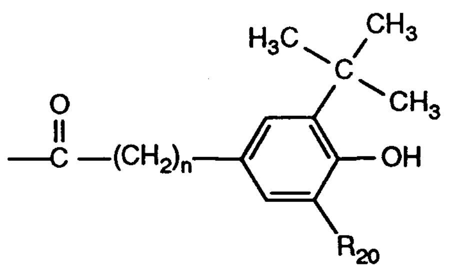

,

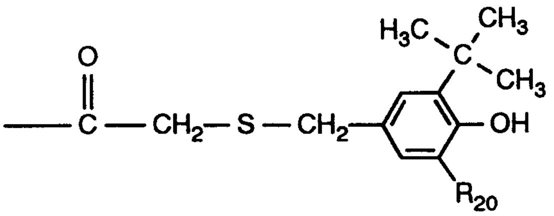 , 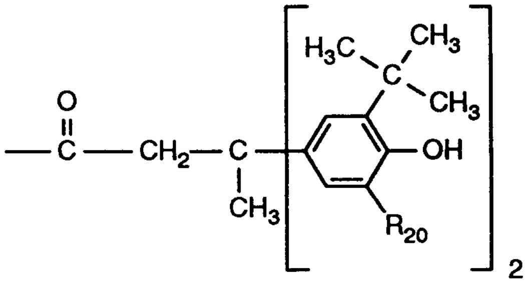 ,

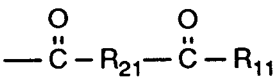 oder 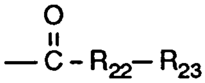

bedeutet, und wenn m 2 ist,
$R_1$

$R_1$ 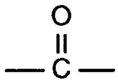 , 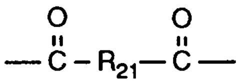 oder 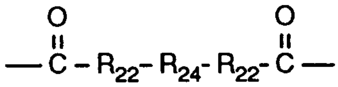

darstellt, und wenn m 3 bedeutet,
$R_1$ $C_4$-$C_{18}$-Alkantricarbonyl, $C_9$-$C_{18}$-Aryltricarbonyl,

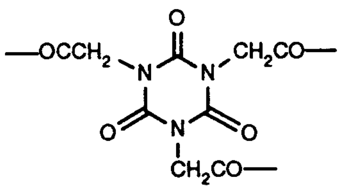

oder

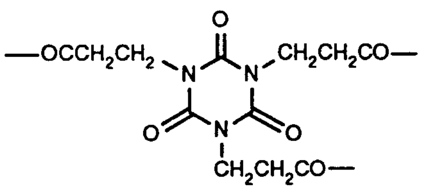

darstellt, und wenn m 4 ist,
$R_1$ $C_6$-$C_{18}$-Alkantetracarbonyl oder $C_{10}$-$C_{18}$-Aryltetracarbonyl bedeutet,
$R_2$, $R_3$, $R_4$ und $R_5$ unabhängig voneinander Wasserstoff, Chlor, $C_1$-$C_{25}$-Alkyl, $C_7$-$C_9$-Phenylalkyl, unsubstituiertes oder durch $C_1$-$C_4$-Alkyl substituiertes Phenyl, unsubstituiertes oder durch C $_1$-$C_4$-Alkyl substituiertes $C_5$-$C_8$-Cyclo-aLkyl; $C_1$-$C_{18}$-Alkoxy, $C_1$-$C_{18}$-Alkylthio, Hydroxy, $C_1$-$C_4$-Alkylamino, Di-($C_1$-$C_4$-alkyl)amino, $C_1$-$C_{25}$-Alkanoyloxy, $C_1$-$C_{25}$-Alkanoylamino, $C_3$-$C_{25}$-Alkenoyloxy, durch Sauerstoff, Schwefel oder $>$N-$R_{16}$ unterbrochenes $C_3$-$C_{25}$-Alkanoyloxy; $C_6$-$C_9$-Cycloalkylcarbonyloxy, Benzoyloxy oder durch $C_1$-$C_{12}$-Alkyl substituiertes Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Phenylring bilden, $R_4$ zusätzlich -$(CH_2)_n$-$COR_{11}$ darstellt, oder wenn $R_3$, $R_5$, $R_6$, $R_7$ und $R_{10}$ Wasserstoff sind, $R_4$ zusätzlich einen Rest der Formel (2)

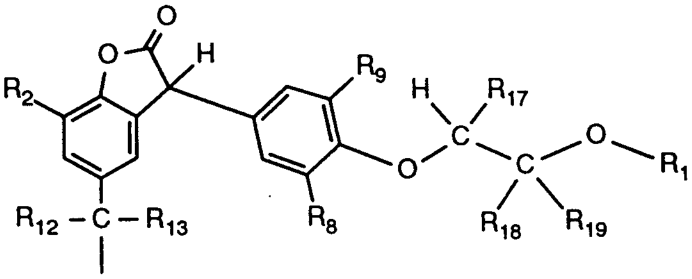

(2)

bedeutet, worin $R_1$ wie oben für m = 1 angegeben definiert ist,
$R_6$ Wasserstoff oder einen Rest der Formel (3)

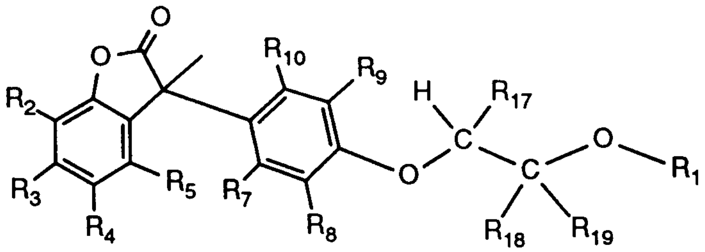

(3)

darstellt, wobei $R_4$ nicht einen Rest der Formel (2) bedeutet und $R_1$ wie oben für m = 1 angegeben definiert ist,
$R_7$, $R_8$, $R_9$ und $R_{10}$ unabhängig voneinander Wasserstoff, $C_1$-$C_4$-Alkyl oder $C_1$-$C_4$-Alkoxy darstellen, mit der Bedingung, dass mindestens einer der Reste $R_7$, $R_8$, $R_9$ und $R_{10}$ Wasserstoff ist,
$R_{11}$ Hydroxy,

$$\left[-O^{\ominus}\ \frac{1}{r}M^{r+}\right],$$

$C_1$-$C_{18}$-Alkoxy oder

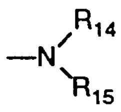

bedeutet,
$R_{12}$ und $R_{13}$ unabhängig voneinander Wasserstoff, $CF_3$, $C_1$-$C_{12}$-Alkyl oder Phenyl darstellen, oder $R_{12}$ und $R_{13}$ zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden;
$R_{14}$ und $R_{15}$ unabhängig voneinander Wasserstoff oder $C_1$-$C_{18}$-Alkyl darstellen,
$R_{16}$ Wasserstoff oder $C_1$-$C_8$-Alkyl bedeutet,
$R_{17}$ Wasserstoff oder $C_1$-$C_4$-Alkyl darstellt,
$R_{18}$ Wasserstoff, unsubstituiertes oder durch $C_1$-$C_4$-Alkyl substituiertes Phenyl;
$C_1$-$C_{25}$-Alkyl, durch Sauerstoff, Schwefel oder $>N-R_{16}$ unterbrochenes $C_2$-$C_{25}$-Alkyl;
unsubstituiertes oder am Phenylrest durch 1 bis 3 $C_1$-$C_4$-Alkyl substituiertes $C_7$-$C_9$-Phenylalkyl; durch Sauerstoff, Schwefel oder $>N-R_{16}$ unterbrochenes unsubstituiertes oder am Phenylrest durch 1 bis 3 $C_1$-$C_4$-Alkyl substituiertes $C_7$-$C_{25}$-Phenylalkyl bedeutet, oder ferner die Reste $R_{17}$ und $R_{18}$ zusammen mit den Kohlenstoffatomen, an die sie gebunden sind, einen unsubstituierten oder durch 1 bis 3 $C_1$-$C_4$-Alkyl substituierten $C_5$-$C_{12}$-Cycloalkylenring bil-

den; oder wenn $R_6$, $R_{17}$ und $R_{19}$ Wasserstoff sind, $R_4$ nicht den Rest der Formel (2) darstellt, m 1 und $R_1$ wie oben für m = 1 angegeben definiert ist, $R_{18}$ zusätzlich einen Rest der Formel (4)

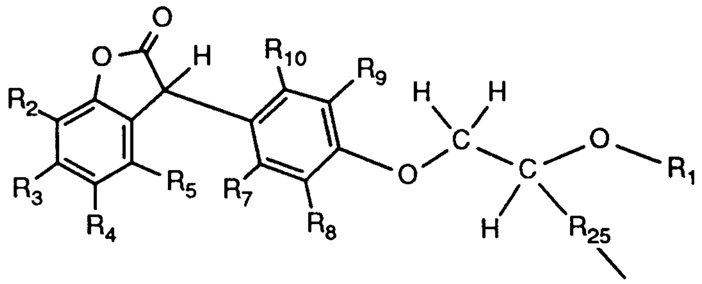

(4)

bedeutet,

$R_{19}$ Wasserstoff oder $C_1$-$C_4$-Alkyl darstellt,

$R_{20}$ Wasserstoff oder $C_1$-$C_4$-Alkyl bedeutet,

$R_{21}$ eine direkte Bindung, $C_1$-$C_{18}$-Alkylen, durch Sauerstoff, Schwefel oder >N-$R_{16}$ unterbrochenes $C_2$-$C_{18}$-Alkylen; $C_2$-$C_{18}$-Alkenylen, $C_2$-$C_{20}$-Alkyliden, $C_7$-$C_{20}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

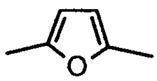

oder

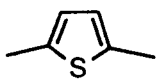

darstellt,

$R_{22}$ Sauerstoff, -NH- oder bedeutet,

$R_{23}$ $C_1$-$C_{18}$-Alkyl oder Phenyl ist,

$R_{24}$ $C_2$-$C_{18}$-Alkylen, $C_5$-$C_8$-Cycloalkylen oder Phenylen darstellt,

$R_{25}$ eine direkte Bindung, $C_1$-$C_{18}$-Alkylen oder durch Sauerstoff, Schwefel oder >N-$R_{16}$ unterbrochenes $C_2$-$C_{18}$-Alkylen bedeutet,

M ein r-wertiges Metallkation ist,

m 1, 2, 3 oder 4 darstellt, wobei, wenn m 2,3 oder 4 ist, $R_6$ Wasserstoff bedeutet;

n 0, 1 oder 2 und

r 1,2 oder 3 darstellt.

Alkanoyl mit bis zu 25 Kohlenstoffatomen bedeutet einen verzweigten oder unverzweigten Rest wie beispielsweise Formyl, Acetyl, Propionyl, Butanoyl, Pentanoyl, Hexanoyl, Heptanoyl, Octanoyl, Nonanoyl, Decanoyl, Undecanoyl, Dodecanoyl, Tridecanoyl, Tetradecanoyl, Pentadecanoyl, Hexadecanoyl, Heptadecanoyl, Octadecanoyl, Eicosanoyl oder Docosanoyl. Eine bevorzugte Bedeutung von $R_1$ ist $C_1$-$C_{18}$-Alkanoyl. Eine speziell bevorzugte Bedeutung von $R_1$ ist $C_2$-$C_4$-Alkanoyl.

Alkenoyl mit 3 bis 25 Kohlenstoffatomen bedeutet einen verzweigten oder unverzweigten Rest wie beispielsweise Propenoyl, 2-Butenoyl, 3-Butenoyl, Isobutenoyl, n-2,4-Pentadienoyl, 3-Methyl-2-butenoyl, n-2-Octenoyl, n-2-Dodecenoyl, iso-Dodecenoyl, Oleoyl, n-2-Octadecenoyl oder n-4-Octadecenoyl.

Durch Sauerstoff, Schwefel oder >N-$R_{16}$ unterbrochenes $C_3$-$C_{25}$-Alkanoyl bedeutet beispielsweise $CH_3$-O-$CH_2CO$-, $CH_3$-S-$CH_2CO$-, $CH_3$-NH-$CH_2CO$-, $CH_3$-N($CH_3$)-$CH_2CO$-, $CH_3$-O-$CH_2CH_2$-O-$CH_2CO$-, $CH_3$-(O-$CH_2CH_2$-)$_2$O-$CH_2CO$-, $CH_3$-(O-$CH_2CH_2$-)$_3$O-$CH_2CO$- oder $CH_3$-(O-$CH_2CH_2$-)$_4$O-$CH_2CO$-. Bevorzugt ist Methoxyacetyl.

Durch eine Di($C_1$-$C_6$-alkyl)phosphonatgruppe substituiertes $C_2$-$C_{25}$-Alkanoyl bedeutet beispielsweise $(CH_3CH_2O)_2POCH_2CO$-, $(CH_3O)_2POCH_2CO$-, $(CH_3CH_2CH_2CH_2O)_2POCH_2CO$-, $(CH_3CH_2O)_2POCH_2CH_2CO$-,

$(CH_3O)_2POCH_2CH_2CO$-, $(CH_3CH_2CH_2CH_2O)_2POCH_2CH_2CO$-, $(CH_3CH_2O)_2PO(CH_2)_4CO$-, $(CH_3CH_2O)_2PO(CH_2)_8CO$- oder $(CH_3CH_2O)_2PO(CH_2)_{17}CO$-.

$C_6$-$C_9$-Cycloalkylcarbonyl bedeutet beispielsweise Cyclopentylcarbonyl, Cyclohexylcarbonyl, Cycloheptylcarbonyl oder Cyclooctylcarbonyl. Cyclohexylcarbonyl ist bevorzugt.

Durch $C_1$-$C_{12}$-Alkyl substituiertes Benzoyl bedeutet beispielsweise o-, m- oder p-Methylbenzoyl, 2,3-Dimethylbenzoyl, 2,4-Dimethylbenzoyl, 2,5-Dimethylbenzoyl, 2,6-Dimethylbenzoyl, 3,4-Dimethylbenzoyl, 3,5-Dimethylbenzoyl, 2-Methyl-6-ethylbenzoyl, 4-tert-Butylbenzoyl, 2-Etyhlbenzoyl, 2,4,6-Trimethylbenzoyl, 2,6-Dimethyl-4-tert-butylbenzoyl oder 3,5-Di-tert-butylbenzoyl.

$C_4$-$C_{18}$-Alkantricarbonyl bedeutet einen verzweigten oder unverzweigten Rest wie beispielsweise Methantricarbonyl, 1,1 ,2-Ethantricarbonyl, 1,2,3-Propantricarbonyl oder 1,2,3-Butantricarbonyl.

$C_9$-$C_{18}$-Aryltricarbonyl bedeutet beispielsweise 1,2,4-Benzoltricarbonyl (abgleitet von Trimellitsäure) oder 1,3,5-Benzoltricarbonyl (abgeleitet von Trimesinsäure).

$C_6$-$C_{18}$-Alkantetracarbonyl bedeutet einen verzweigten oder unverzweigten Rest wie beispielsweise 1,1,3,3-Propantetracarbonyl oder 1,2,3,4-Butantetracarbonyl.

$C_{10}$-$C_{18}$-Aryltetracarbonyl bedeutet beispielsweise 1,2,4,5-Benzoltetracarbonyl (abgeleitet von Pyromellitsäure).

Alkyl mit bis zu 25 Kohlenstoffatomen bedeutet einen verzweigten oder unverzweigten Rest wie beispielsweise Methyl, Ethyl, Propyl, Isopropyl, n-Butyl, sec-Butyl, Isobutyl, tert-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1 ,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl oder Docosyl. Eine der bevorzugten Bedeutungen von $R_2$ und $R_4$ ist beispielweise $C_1$-$C_{18}$-Alkyl. Eine besonders bevorzugte Bedeutung von $R_4$ ist $C_1$-$C_4$-Alkyl.

$C_7$-$C_9$-Phenylalkyl bedeutet beispielsweise Benzyl, $\alpha$-Methylbenzyl, $\alpha,\alpha$-Dimethylbenzyl oder 2-Phenylethyl. Benzyl ist bevorzugt.

Durch $C_1$-$C_4$-Alkyl substituiertes Phenyl, das vorzugsweise 1 bis 3, insbesondere 1 oder 2 Alkylgruppen enthält, bedeutet beispielsweise o-, m- oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 4-tert-butylphenyl, 2-Ethylphenyl oder 2,6-Diethylphenyl.

Unsubstituiertes oder durch $C_1$-$C_4$-Alkyl substituiertes $C_5$-$C_8$-Cycloalkyl bedeutet beispielsweise Cyclopentyl, Methylcyclopentyl, Dimethylcyclopentyl, Cyclohexyl, Methylcyclohexyl, Dimethylcyclohexyl, Trimethylcyclohexyl, tert-Butylcyclohexyl, Cycloheptyl oder Cyclooctyl. Bevorzugt ist Cyclohexyl und tert-Butylcyclohexyl.

Alkoxy mit bis zu 18 Kohlenstoffatomen bedeutet einen verzweigten oder unverzweigten Rest wie beispielsweise Methoxy, Ethoxy, Propoxy, Isopropoxy, n-Butoxy, Isobutoxy, Pentoxy, Isopentoxy, Hexoxy, Heptoxy, Octoxy, Decyloxy, Tetradecyloxy, Hexadecyloxy oder Octadecyloxy.

Alkylthio mit bis zu 18 Kohlenstoffatomen bedeutet einen verzweigten oder unverzweigten Rest wie beispielsweise Methylthio, Ethylthio, Propylthio, Isopropylthio, n-Butylthio, Isobutylthio, Pentylthio, Isopentylthio, Hexylthio, Heptylthio, Octylthio, Decylthio, Tetradecylthio, Hexadecylthio oder Octadecylthio.

Alkylamino mit bis zu 4 Kohlenstoffatomen bedeutet einen verzweigten oder unverzweigten Rest wie beispielsweise Methylamino, Ethylamino, Propylamino, Isopropylamino, n-Butylamino, Isobutylamino oder tert-Butylamino.

Di-($C_1$-$C_4$-alkyl)amino bedeutet auch, dass die beiden Reste unabhängig voneinander verzweigt oder unverzweigt sind wie beispielsweise Dimethylamino, Methylethylamino, Diethylamino, Methyl-n-propylamino, Methylisopropylamino, Methyl-n-butylamino, Methylisobutylamino, Ethylisopropylamino, Ethyl-n-butylamino, Ethylisobutylamino, Ethyl-tert-butylamino, Diethylamino, Diisopropylamino, Isopropyl-n-butylamino, Isopropylisobutylamino, Di-n-butylamino oder Di-isobutylamino.

Alkanoyloxy mit bis zu 25 Kohlenstoffatomen bedeutet einen verzweigten oder unverzweigten Rest wie beispielsweise Formyloxy, Acetyloxy, Propionyloxy, Butanoyloxy, Pentanoyloxy, Hexanoyloxy, Heptanoyloxy, Octanoyloxy, Nonanoyloxy, Decanoyloxy, Undecanoyloxy, Dodecanoyloxy, Tridecanoyloxy, Tetradecanoylxoy, Pentadecanoyloxy, Hexadecanoyloxy, Heptadecanoyloxy, Octadecanoyloxy, Eicosanoyloxy oder Docosanoyloxy.

Alkanoylamino mit bis zu 25 Kohlenstoffatomen bedeutet einen verzweigten oder unverzweigten Rest wie beispielsweise Formylamino, Acetylamino, Propionylamino, Butanoylamino, Pentanoylamino, Hexanoylamino, Heptanoylamino, Octanoylamino, Nonanoylamino, Decanoylamino, Undecanoylamino, Dodecanoylamino, Tridecanoylamino, Tetradecanoylamino, Pentadecanoylamino, Hexadecanoylamino, Heptadecanoylamino, Octadecanoylamino, Eicosanoylamino oder Docosanoylamino.

Alkenoyloxy mit 3 bis 25 Kohlenstoffatomen bedeutet einen verzweigten oder unverzweigten Rest wie beispielsweise Propenoyloxy, 2-Butenoyloxy, 3-Butenoyloxy, Isobutenoyloxy, n-2,4-Pentadienoyloxy, 3-Methyl-2-butenoyloxy, n-2-Octenoyloxy, n-2-Dodecenoyloxy, iso-Dodecenoyloxy, Oleoyloxy, n-2-Octadecenoyloxy oder n-4-Octadecenoyloxy.

Durch Sauerstoff, Schwefel oder >N-$R_{16}$ unterbrochenes $C_3$-$C_{25}$-Alkanoyloxy bedeutet beispielsweise $CH_3$-O-$CH_2$COO-, $CH_3$-S-$CH_2$COO-, $CH_3$-NH-$CH_2$COO-, $CH_3$-N($CH_3$)-$CH_2$COO-, $CH_3$-O-$CH_2CH_2$-O-$CH_2$COO-, $CH_3$-(O-$CH_2CH_2$-$)_2$O-$CH_2$COO-, $CH_3$-(O-$CH_2CH_2$-$)_3$O-$CH_2$COO- oder $CH_3$-(O-$CH_2CH_2$-$)_4$O-$CH_2$COO-.

$C_6$-$C_9$-Cycloalkylcarbonyloxy bedeutet beispielsweise Cyclopentylcarbonyloxy, Cyclohexylcarbonyloxy, Cycloheptylcarbonyloxy oder Cyclooctylcarbonyloxy. Cyclohexylcarbonyloxy ist bevorzugt.

Durch $C_1$-$C_{12}$-Alkyl substituiertes Benzoyloxy bedeutet beispielsweise o-, m- oder p-Methylbenzoyloxy, 2,3-Dimethylbenzoyloxy, 2,4-Dimethylbenzoyloxy, 2,5-Dimethylbenzoyloxy, 2,6-Dimethylbenzoyloxy, 3,4-Dimethylbenzoyloxy, 3,5-Dimethylbenzoyloxy, 2-Methyl-6-ethylbenzoyloxy, 4-tert-Butylbenzoyloxy, 2-Ethylbenzoyloxy, 2,4,6-Trimethylbenzoyloxy, 2,6-Dimethyl-4-tert-butylbenzoyloxy oder 3,5-Di-tert-butylbenzoyloxy.

Durch $C_1$-$C_4$-Alkyl substituierter $C_5$-$C_8$-Cycloalkylidenring, der vorzugsweise 1 bis 3, insbesondere 1 oder 2 verzweigte oder unverzweigte Alkylgruppen-Reste enthält, bedeutet beispielsweise Cyclopentyliden, Methylcyclopentyliden, Dimethylcyclopentyliden, Cyclohexyliden, Methylcyclohexyliden, Dimethylcyclohexyliden, Trimethylcyclohexyliden, tert-Butylcyclohexyliden, Cycloheptyliden oder Cyclooctyliden. Bevorzugt ist Cyclohexyliden und tert-Butylcyclohexyliden.

Durch $C_1$-$C_4$-Alkyl substituierter $C_5$-$C_{12}$-Cycloalkylenring, der vorzugsweise 1 bis 3, insbesondere 1 oder 2 verzweigte oder unverzweigte Alkylgruppen-Reste enthält, bedeutet beispielsweise Cyclopentylen, Methylcyclopentylen, Dimethylcyclopentylen, Cyclohexylen, Methylcyclohexylen, Dimethylcyclohexylen, Trimethylcyclohexylen, tert-Butylcyclohexylen, Cycloheptylen, Cyclooctylen oder Cyclodecylen. Bevorzugt ist Cyclohexylen und tert-Butylcyclohexylen.

Durch Sauerstoff, Schwefel oder >N-$R_{16}$ unterbrochenes $C_2$-$C_{25}$-Alkyl bedeutet beispielsweise $CH_3$-O-$CH_2$-, $CH_3$-S-$CH_2$-, $CH_3$-NH-$CH_2$-, $CH_3$-N($CH_3$)-$CH_2$-, $CH_3$-O-$CH_2CH_2$-O-$CH_2$-, $CH_3$-(O-$CH_2CH_2$-$)_2$O-$CH_2$-, $CH_3$-(O-$CH_2CH_2$-$)_3$O-$CH_2$- oder $CH_3$-(O-$CH_2CH_2$-$)_4$O-$CH_2$-.

Unsubstituiertes oder am Phenylrest durch 1 bis 3 $C_1$-$C_4$-Alkyl substituiertes $C_7$-$C_9$-Phenylalkyl bedeutet beispielsweise Benzyl, α-Methylbenzyl, α,α-Dimethylbenzyl, 2-Phenylethyl, 2-Methylbenzyl, 3-Methylbenzyl, 4-Methylbenzyl, 2,4-Dimethylbenzyl, 2,6-Dimethylbenzyl oder 4-tert-Butylbenzyl. Benzyl ist bevorzugt.

Durch Sauerstoff, Schwefel oder >N-$R_{16}$ unterbrochenes unsubstituiertes oder am Phenylrest durch 1 bis 3 $C_1$-$C_4$-Alkyl substituiertes $C_7$-$C_{25}$-Phenylalkyl bedeutet einen verzweigten oder unverzweigten Rest wie beispielsweise Phenoxymethyl, 2-Methyl-phenoxymethyl, 3-Methyl-phenoxymethyl, 4-Methyl-phenoxymethyl, 2,4-Dimethyl-phenoxymethyl, 2,3-Dimethyl-phenoxymethyl, Phenylthiomethyl, N-Methyl-N-phenyl-methyl, N-Ethyl-N-phenyl-methyl, 4-tert-Butyl-phenoxymethyl, 4-tert-Butyl-phenoxyethoxymethyl, 2,4-Di-tert-butyl-phenoxymethyl, 2,4-Di-tert-butyl-phenoxyethoxymethyl, Phenoxyethoxyethoxyethoxymethyl, Benzyloxymethyl, Benzyloxyethoxymethyl, N-BenzylN-ethyl-methyl oder N-Benzyl-N-isopropyl-methyl.

$C_1$-$C_{18}$-Alkylen bedeutet einen verzweigten oder unverzweigten Rest wie beispielsweise Methylen, Ethylen, Propylen, Butylen, Pentamethylen, Hexamethylen, Heptamethylen, Octamethylen, Decamethylen, Dodecamethylen oder Octadecamethylen. Bevorzugt ist $C_1$-$C_8$-Alkylen.

Durch Sauerstoff, Schwefel oder >N-$R_{16}$ unterbrochenes $C_2$-$C_{18}$-Alkylen bedeutet beispielsweise -$CH_2$-O-$CH_2$-, -$CH_2$-S-$CH_2$-, -$CH_2$-NH-$CH_2$-, -$CH_2$-N($CH_3$)-$CH_2$ -, -$CH_2$-O-$CH_2CH_2$-O-$CH_2$-, -$CH_2$-(O-$CH_2CH_2$-$)_2$O-$CH_2$-, -$CH_2$-(O-$CH_2CH_2$-$)_3$O-$CH_2$-, -$CH_2$-(O-$CH_2CH_2$-$)_4$O-$CH_2$- oder-$CH_2CH_2$-S-$CH_2CH_2$-.

$C_2$-$C_{18}$-Alkenylen bedeutet beispielsweise Vinylen, Methylvinylen, Octenylethylen oder Dodecenylethylen. Bevorzugt ist $C_2$-$C_8$-Alkenylen.

Alkyliden mit 2 bis 20 Kohlenstoffatomen bedeutet beispielsweise Ethyliden, Propyliden, Butyliden, Pentyliden, 4-Methylpentyliden, Heptyliden, Nonyliden, Tridecyliden, Nonadecyliden, 1-Methylethyliden, 1-Ethylpropyliden oder 1-Ethylpentyliden. Bevorzugt ist $C_2$-$C_8$-Alkyliden.

Phenylalkyliden mit 7 bis 20 Kohlenstoffatomen bedeutet beispielsweise Benzyliden, 2-Phenylethyliden oder 1-Phenyl-2-hexyliden. Bevorzugt ist $C_7$-$C_9$-Phenylalkyliden.

$C_5$-$C_8$-Cycloalkylen bedeutet eine gesättigte Kohlenwasserstoffgruppe mit zwei freien Valenzen und mindestens einer Ringeinheit und ist beispielsweise Cyclopentylen, Cyclohexylen, Cycloheptylen oder Cyclooctylen. Bevorzugt ist Cyclohexylen.

$C_7$-$C_8$-Bicycloalkylen bedeutet beispielsweise Bicycloheptylen oder Bicyclooctylen.

Unsubstituiertes oder durch $C_1$-$C_4$-Alkyl substituiertes Phenylen bedeutet beispielsweise 1,2-, 1,3- oder 1,4-Phenylen.

$C_2$-$C_{18}$-Alkylen bedeutet einen verzweigten oder unverzweigten Rest wie beispielsweise Ethylen, Propylen, Butylen, Pentamethylen, Hexamethylen, Heptamethylen, Octamethylen, Decamethylen, Dodecamethylen oder Octadecamethylen. Bevorzugt ist $C_2$-$C_8$-Alkylen.

Ein ein-, zwei- oder drei-wertiges Metallkation ist vorzugsweise ein Alkalimetall-, Erdalkalimetall-oder Aluminium-Kation, beispielsweise Na+, $K^+$, $Mg^{++}$, $Ca^{++}$ oder $Al^{+++}$.

Bevorzugt sind Verbindungen der Formel (1), worin
wenn m 1 ist,

$R_1$ Wasserstoff, $C_1$-$C_{18}$-Alkanoyl, $C_3$-$C_{18}$-Alkenoyl, durch Sauerstoff, Schwefel oder >N-$R_{16}$ unterbrochenes $C_3$-$C_{18}$-Alkanoyl; durch eine Di($C_1$-$C_6$-alkyl)phosphonatgruppe substituiertes $C_2$-$C_{18}$-Alkanoyl; $C_6$-$C_9$-Cydoalkylcarbonyl, Thenoyl, Furoyl, Benzoyl oder durch $C_1$-$C_8$-Alkyl substituiertes Benzoyl;

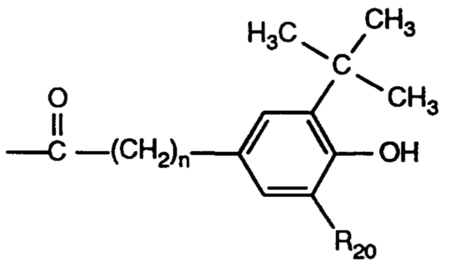 ,

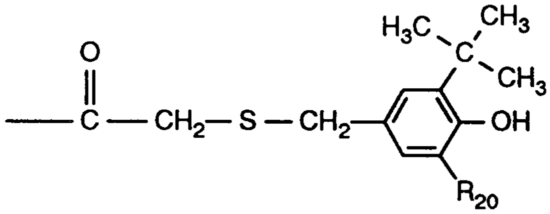 , 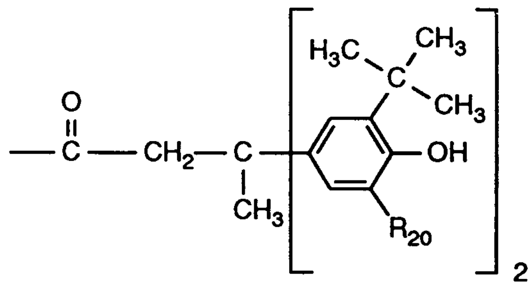 ,

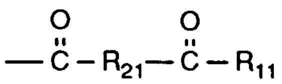 oder 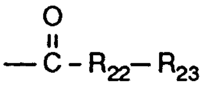

bedeutet,

$R_2$, $R_3$, $R_4$ und $R_5$ unabhängig voneinander Wasserstoff, Chlor, $C_1$-$C_{18}$-Alkyl, Benzyl, Phenyl, $C_5$-$C_8$-Cycloalkyl, $C_1$-$C_{18}$-Alkoxy, $C_1$-$C_{18}$-Alkylthio, $C_1$-$C_{18}$-Alkanoyloxy, $C_1$-$C_{18}$-Alkanoylamino, $C_3$-$C_{18}$-Alkenoyloxy oder Benzoyloxy darstellen, oder ferner die Reste $R_2$ und $R_3$ oder die Reste $R_4$ und $R_5$ zusammen mit den Kohlenstoffatomen, an die sie gebunden sind, einen Phenylring bilden, oder wenn $R_3$, $R_5$, $R_6$, $R_7$ und $R_{10}$ Wasserstoff sind, $R_4$ zusätzlich einen Rest der Formel (2) bedeutet,

$R_7$, $R_8$, $R_9$ und $R_{10}$ unabhängig voneinander Wasserstoff oder $C_1$-$C_4$-Alkyl darstellen, mit der Bedingung, dass mindestens einer der Reste $R_7$, $R_8$, $R_9$ und $R_{10}$ Wasserstoff ist, $R_{12}$ und $R_{13}$ Methylgruppen sind oder zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden,

$R_{18}$ Wasserstoff, Phenyl, $C_1$-$C_{18}$-Alkyl, durch Sauerstoff, Schwefel oder >N-$R_{16}$ unterbrochenes $C_2$-$C_{18}$-Alkyl; Benzyl, durch Sauerstoff, Schwefel oder >N-$R_{16}$ unterbrochenes $C_7$-$C_{18}$-Phenylalkyl bedeutet, oder ferner die Reste $R_{17}$ und $R_{18}$ zusammen mit den Kohlenstoffatomen, an die sie gebunden sind einen unsubstituierten oder durch 1 bis 3 $C_1$-$C_4$-Alkyl substituierten $C_5$-$C_8$-Cycloalkylenring bilden,

$R_{21}$ eine direkte Bindung, $C_1$-$C_{12}$-Alkylen, durch Sauerstoff, Schwefel oder >N-$R_{16}$ unterbrochenes $C_2$-$C_{12}$-Alkylen; $C_2$-$C_{12}$-Alkenylen, $C_2$-$C_{12}$-Alkyliden, $C_7$-$C_{12}$-Phenylalkyliden, $C_5$-$C_8$-Cycloalkylen, $C_7$-$C_8$-Bicycloalkylen oder Phenylen darstellt, $R_{24}$ $C_2$-$C_{12}$-Alkylen, $C_5$-$C_8$-Cycloalkylen oder Phenylen bedeutet, und $R_{25}$ eine direkte Bindung, $C_1$-$C_{12}$-Alkylen oder durch Sauerstoff, Schwefel oder >N-$R_{16}$ unterbrochenes $C_2$-$C_{12}$-Alkylen darstellt.

Bevorzugt sind auch Verbindungen der Formel (1), worin mindestens zwei der Reste $R_2$, $R_3$, $R_4$ und $R_5$ Wasserstoff sind.

Ebenfalls bevorzugt sind Verbindungen der Formel (1), worin $R_3$ und $R_5$ Wasserstoff sind.

Besonders bevorzugt sind Verbindungen der Formel (1), worin $R_2$, $R_3$, $R_4$ und $R_5$ unabhängig voneinander Wasserstoff, Chlor, $C_1$-$C_{18}$-Alkyl, $C_5$-$C_6$-Cycloalkyl oder $C_1$-$C_4$-Alkoxy darstellen, oder ferner die Reste $R_2$ und $R_3$ zusammen mit den Kohlenstoffatomen, an die sie gebunden sind, einen Phenylring bilden.

Ebenfalls besonders bevorzugt sind Verbindungen der Formel (1), worin m 1 oder 2 bedeutet.

Von besonderem Interesse sind Verbindungen der Formel (1), worin $R_{18}$ Wasserstoff, $C_1$-$C_{12}$-Alkyl, durch

Sauerstoff oder Schwefel unterbrochenes $C_2$-$C_{12}$-Alkyl; durch Sauerstoff oder Schwefel unterbrochenes $C_7$-$C_{12}$-Phenylalkyl bedeutet, oder ferner die Reste $R_{17}$ und $R_{18}$ zusammen mit den Kohlenstoffatomen, an die sie gebunden sind, einen $C_5$-$C_8$-Cycloalkylenring bilden.

Auch von besonderem Interesse sind Verbindungen der Formel (1), worin, wenn m 1 ist,

$R_1$ Wasserstoff, $C_1$-$C_{18}$-Alkanoyl, C3-C12-Alkenoyl, durch Sauerstoff unterbrochenes $C_3$-$C_{12}$-Alkanoyl; durch eine Di($C_1$-$C_6$-alkyl)phosphonatgruppe substituiertes $C_2$-$C_{12}$-Alkanoyl; $C_6$-$C_9$-Cycloalkylcarbonyl, Benzoyl,

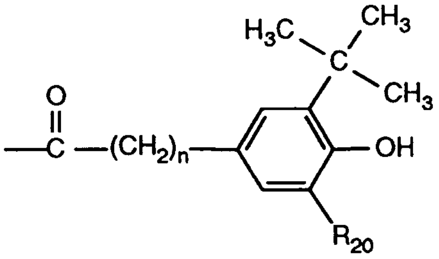 , 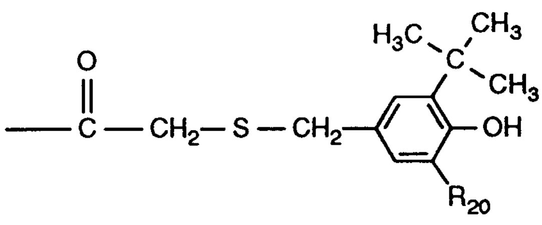 ,

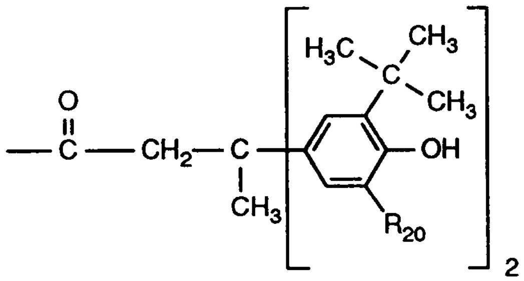 , 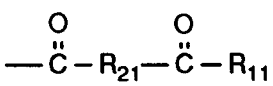 oder 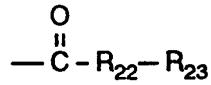

bedeutet,

$R_2$, $R_3$, $R_4$ und $R_5$ unabhängig voneinander Wasserstoff, $C_1$-$C_{18}$-Alkyl, $C_5$-$C_7$-Cycloalkyl, $C_1$-$C_{12}$-Alkoxy, $C_1$-$C_{12}$-Alkanoyloxy oder Benzoyloxy darstellen, oder ferner die Reste $R_2$ und $R_3$ zusammen mit den Kohlenstoffatomen, an die sie gebunden sind, einen Phenylring bilden, oder wenn $R_3$, $R_5$, $R_6$, $R_7$ und $R_{10}$ Wasserstoff sind, $R_4$ zusätzlich einen Rest der Formel (2) bedeutet,

$R_{12}$ und $R_{13}$ Methylgruppen sind oder zusammen mit dem C-Atom, an das sie gebunden sind, einen $C_5$-$C_8$-Cycloalkylidenring bilden,

$R_{18}$ Wasserstoff, $C_1$-$C_{12}$-Alkyl, durch Sauerstoff oder Schwefel unterbrochenes $C_2$-$C_{12}$-Alkyl; durch Sauerstoff oder Schwefel unterbrochenes $C_7$-$C_{12}$-Phenylalkyl bedeutet, oder ferner die Reste $R_{17}$ und $R_{18}$ zusammen mit den Kohlenstoffatomen, an die sie gebunden sind, einen $C_5$-$C_8$-Cycloalkylenring bilden,

$R_{21}$ $C_1$-$C_{12}$-Alkylen, Phenylen oder durch Sauerstoff oder Schwefel unterbrochenes $C_2$-$C_{12}$-Alkylen darstellt,

$R_{23}$ $C_1$-$C_{12}$-Alkyl bedeutet,

$R_{24}$ $C_2$-$C_{12}$-Alkylen, oder Phenylen darstellt,

$R_{25}$ $C_1$-$C_8$-Alkylen oder durch Sauerstoff unterbrochenes $C_2$-$C_8$-Alkylen bedeutet, und m 1, 2 oder 3 darstellt.

Speziell von besonderem Interesse sind Verbindungen der Formel (1), worin, wenn m 1 ist,

$R_1$ Wasserstoff, $C_1$-$C_{18}$-Alkanoyl, $C_3$-$C_4$-Alkenoyl, durch eine Di($C_1$-$C_4$-alkyl)phosphonatgruppe substituiertes $C_2$-$C_4$-Alkanoyl; Cyclohexylcarbonyl, Benzoyl,

, 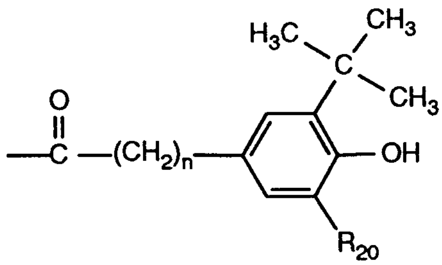 , 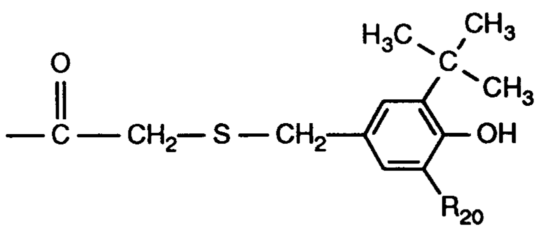

oder

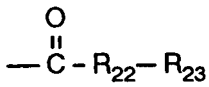

bedeutet, und wenn m 2 ist,
$R_1$

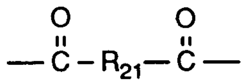

oder

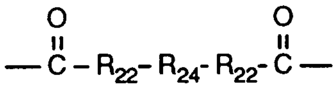

darstellt,
$R_2$ Wasserstoff, $C_1$-$C_{18}$-Alkyl oder Cyclohexyl bedeutet,
$R_3$ Wasserstoff ist, oder ferner die Reste $R_2$ und $R_3$ zusammen mit den Kohlenstoffatomen, an die sie gebunden sind, einen Phenylring bilden,
$R_4$ $C_1$-$C_4$-Alkyl oder Cyclohexyl darstellt, oder wenn $R_3$, $R_5$, $R_6$, $R_7$ und $R_{10}$ Wasserstoff sind, $R_4$ zusätzlich einen Rest der Formel (2) bedeutet, worin $R_1$ wie oben für m = 1 angegeben definiert ist,
$R_5$ Wasserstoff bedeutet,
$R_6$ Wasserstoff oder einen Rest der Formel (3) darstellt, wobei $R_4$ nicht einen Rest der Formel (2) bedeutet und $R_1$ wie oben für m = 1 angegeben definiert ist,
$R_7$ Wasserstoff bedeutet,
$R_8$ und $R_9$ unabhängig voneinander Wasserstoff, $C_1$-$C_4$-Alkyl oder $C_1$-$C_4$-Alkoxy darstellen,
$R_{10}$ Wasserstoff bedeutet,
$R_{12}$ und $R_{13}$ Methylgruppen sind oder zusammen mit dem C-Atom, an das sie gebunden sind, einen Cyclohexylidenring bilden,
$R_{17}$ Wasserstoff darstellt,
$R_{18}$ Wasserstoff, $C_1$-$C_4$-Alkyl, durch Sauerstoff unterbrochenes $C_2$-$C_8$-Alkyl; oder durch Sauerstoff unterbrochenes $C_7$-$C_9$-Phenylalkyl bedeutet, oder ferner die Reste $R_{17}$ und $R_{18}$ zusammen mit den Kohlenstoffatomen, an die sie gebunden sind, einen Cyclohexylenring bilden,
$R_{19}$ Wasserstoff darstellt,
$R_{20}$ Wasserstoff oder $C_1$-$C_4$-Alkyl ist,
$R_{21}$ $C_1$-$C_8$-Alkylen, durch Schwefel unterbrochenes $C_2$-$C_6$-Alkylen; oder Phenylen darstellt,
$R_{22}$ -NH- oder

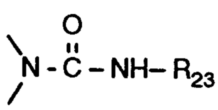

bedeutet,
$R_{23}$ $C_1$-$C_4$-Alkyl darstellt,
$R_{24}$ $C_4$-$C_8$-Alkylen bedeutet,
m 1 oder 2 darstellt, und
n 0 oder 2 bedeutet.

Die erfindungsgemässen Verbindungen der Formel (1) können auf an sich bekannte Weise hergestellt werden.

Beispielsweise, und dies ist bevorzugt, wird ein Phenol der Formel (5),

(5) 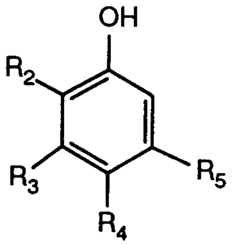 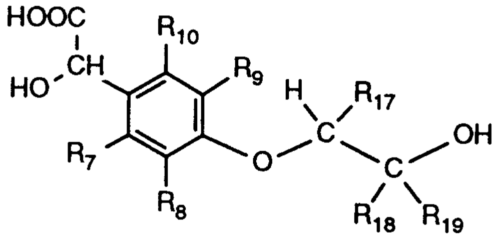 (6)

worin $R_2$, $R_3$, $R_4$ und $R_5$ die angegebenen Bedeutungen haben, mit einem am Phenylring substituierten Mandelsäure-Derivat der Formel (6), worin $R_7$, $R_8$, $R_9$, $R_{10}$, $R_{17}$, $R_{18}$ und $R_{19}$ die angegebenen Bedeutungen haben, und wenn $R_{17}$ und $R_{19}$ Wasserstoff sind, $R_{18}$ zusätzlich einen Rest der Formel (10)

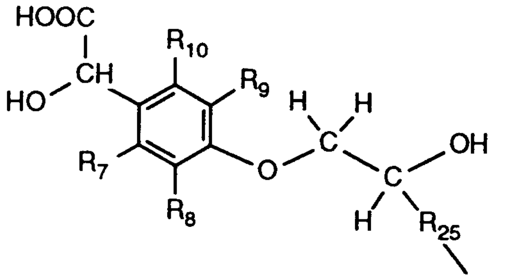 (10)

bedeutet, worin $R_7$, $R_8$, $R_9$, $R_{10}$ und $R_{25}$ die angegebenen Bedeutungen haben, bei erhöhter Temperatur, insbesondere Temperaturen von 130 bis 200°C in der Schmelze oder in einem Lösungsmittel gegebenenfalls unter leichtem Vakuum, zu Verbindungen der Formel (7)

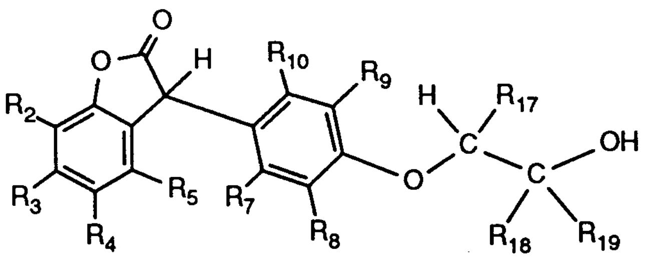 (7)

umgesetzt. Bevorzugt wird die Reaktion in einem Lösungsmittel wie beispielsweise Essigsäure, Propionsäure oder Ameisensäure in einem Temperaturbereich von 50 bis 130°C durchgeführt. Die Reaktion kann durch Zusatz einer Säure wie Salzsäure, Schwefelsäure oder Methansulfonsäure katalysiert werden. Die Umsetzung kann z.B. in der Weise durchgeführt werden, wie sie in den in der Beschreibungseinleitung angegebenen Literaturstellen beschrieben ist.

Die durch diese Umsetzung erhaltenen Alkohole der Formel (7), können nach allgemein bekannten Veresterungsmethoden, z.B. gemäss Organikum 1986, Seite 402-408, beispielsweise durch Acylierung mit einem Säurechlorid oder Säureanhydrid der Formel $R_1^1Cl$ bzw. $R_1^1$-O-$R_1^1$, worin $R_1^1$ für $R_1$ mit Ausnahme von Wasserstoff steht, zu den Verbindungen der Formel (1) verestert werden. Wird als Reagens anstelle eines Säurechlorids ein Isocyanat der Formel $R_{23}$-N=C=O verwendet, werden die entsprechenden Carbamate der Formel (1) erhalten, worin $R_1$ einen Rest

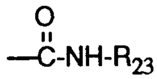

bedeutet.

Die erfindungsgemässen Verbindungen der Formel (1) können in verschiedenen Kristallmodifikationen vorliegen.

Die Alkohole der Formel (7) können ebenfalls nach allgemein bekannten Umeresterungsmethoden, z.B. gemäss Organikum 1986, Seite 388, beispielsweise durch Umesterung mit

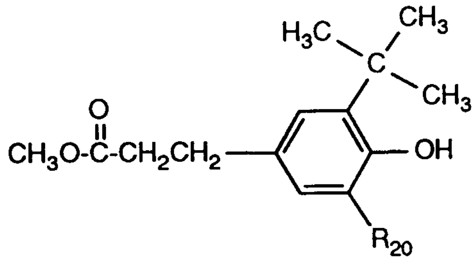

zu den Verbindungen der Formel (1) umgesetzt werden. Das bei der Reaktion entstehende Methanol wird kontinuierlich abdestilliert.

Die 3-(2-Hydroxyethoxyphenyl)benzofuran-2-one der Formel (7) können auch ohne Isolierung oder Reinigung direkt mit Säurechloriden oder Säureanhydriden zu den 3-(2-Acyloxyethoxyphenyl)benzofuran-2-onen der Formel (1) umgesetzt werden.

Die Umsetzung der Verbindungen der Formel (5) und (6) erfolgt bevorzugt durch Kochen der beiden Komponenten in einem Carbonsäure Lösungsmittel wie beispielsweise Essigsäure oder Propionsäure. Das Reaktionswasser wird durch Destillation, vorteilhaft durch azeotrope Destillation oder durch Zugabe des bezüglich Lösungsmittel entsprechenden Säurechlorids, beispielsweise Acetylchlorid oder Propionylchlorid, oder Säureanhydrids, beispielsweise Essigsäureanhydrid oder Propionsäureanhydrid, entfernt. Als Produkte fallen dann die entsprechenden 3-(2-Acyloxyethoxyphenyl)benzofuran-2-one der Formel (1) an.

Die 3-(2-Hydroxyethoxyphenyl)benzofuran-2-one der Formel (7) sind auch durch Hydrolyse oder Alkoholyse der 3-(2-Acyloxyethoxyphenyl)benzofuran-2-one der Formel (1) erhälflich. Die Reaktion wird bevorzugt in rückflussierendem Methanol, dem konzentrierte Salzsäure zugegeben wird, durchgeführt.

Die Phenole der Formel (5) sind bekannt oder können nach an sich bekannten Verfahren erhalten werden.

Bisphenolverbindungen der Formel (8)

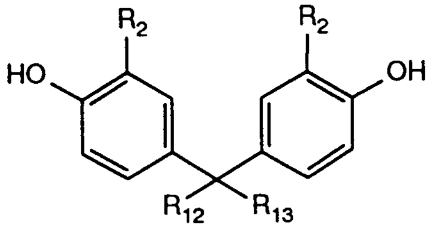

(8)

können gemäss Houben-Weyl, Methoden der organischen Chemie, Band 6/1c, 1030, hergestellt werden.

4-(2-Hydroxyethoxy)mandelsäuren sind in der Literatur weitgehend unbekannt. Einzig die unsubstituierte 4-(2-Hydroxyethoxy)mandelsäure ist in EP-A-146 269 und EP-A-397 170 beschrieben.

Die vorliegende Erfindung betrifft daher auch Verbindungen der Formel (9)

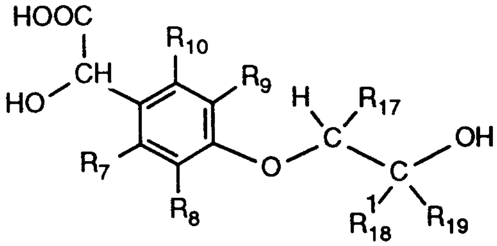

(9)

worin $R_7$, $R_8$, $R_9$ und $R_{10}$ unabhängig voneinander Wasserstoff, $C_1$-$C_4$-Alkyl oder $C_1$-$C_4$-Alkoxy darstellen, mit der Bedingung, dass mindestens einer der Reste $R_7$, $R_8$, $R_9$ und $R_{10}$ Wasserstoff ist, und, wenn $R_7$, $R_8$, $R_9$ und $R_{10}$ gleichzeitig Wasserstoff sind,

entweder $R_{17}$, $R^1_{18}$ oder $R_{19}$ von Wasserstoff verschieden ist,

$R_{16}$ Wasserstoff oder $C_1$-$C_8$-Alkyl bedeutet,

$R_{17}$ Wasserstoff oder $C_1$-$C_4$-Alkyl darstellt,

$R^1_{18}$ Wasserstoff, unsubstituiertes oder durch $C_1$-$C_4$-Alkyl substituiertes Phenyl; $C_1$-$C_{25}$-Alkyl, durch Sauerstoff oder Schwefel unterbrochenes $C_2$-$C_{25}$-Alkyl; unsubstituiertes oder am Phenylring durch 1 bis 3 $C_1$-$C_4$-Alkyl substituiertes $C_7$-$C_9$-Phenylalkyl; durch Sauerstoff, Schwefel oder >N-$R_{16}$ unterbrochenes unsubstituiertes oder am Phenylring durch 1 bis 3 $C_1$-$C_4$-Alkyl substituiertes $C_7$-$C_{25}$-Phenylalkyl bedeutet, oder ferner die Reste $R_{17}$ und $R^1_{18}$ zusammen mit den Kohlenstoffatomen, an die sie gebunden sind einen unsubstituierten oder durch 1 bis 3 $C_1$-$C_4$-Alkyl substituierten $C_5$-$C_{12}$-Cycloalkylenring bilden; oder wenn $R_{17}$ und $R_{19}$ Wasserstoff sind, $R^1_{18}$ zusätzlich einen Rest der Formel (10)

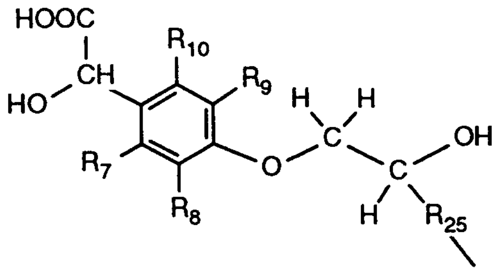

(10)

bedeutet,

$R_{19}$ Wasserstoff oder $C_1$-$C_4$-Alkyl darstellt, und

$R_{25}$ eine direkte Bindung, $C_1$-$C_{18}$-Alkylen oder durch Sauerstoff, Schwefel oder >N-$R_{16}$ unterbrochenes $C_2$-$C_{18}$-Alkylen bedeutet.

Bevorzugt sind Verbindungen der Formel (9), worin

$R^1_{18}$ Wasserstoff, Phenyl, $C_1$-$C_{18}$-Alkyl, durch Sauerstoff oder Schwefel unterbrochenes $C_2$-$C_{18}$-Alkyl; Benzyl, durch Sauerstoff, Schwefel oder >N-$R_{16}$ unterbrochenes $C_7$-$C_{18}$-Phenylalkyl bedeutet, oder ferner die Reste $R_{17}$ und $R^1_{18}$ zusammen mit den Kohlenstoffatomen, an die sie gebunden sind einen unsubstituierten oder durch 1 bis 3 $C_1$-$C_4$-Alkyl substituierten $C_5$-$C_8$-Cycloalkylenring bilden, und

$R_{25}$ eine direkte Bindung, $C_1$-$C_{12}$-Alkylen oder durch Sauerstoff, Schwefel oder >N-$R_{16}$ unterbrochenes $C_2$-$C_{12}$-Alkylen bedeutet.

Ebenfalls bevorzugt sind Verbindungen der Formel (9), worin $R_7$ und $R_{10}$ Wasserstoff sind.

Besonders bevorzugt sind Verbindungen der Formel (9), worin

$R_{17}$ Wasserstoff darstellt,

$R^1_{18}$ Wasserstoff, $C_1$-$C_{12}$-Alkyl, durch Sauerstoff oder Schwefel unterbrochenes $C_2$-$C_{12}$-Alkyl; durch Sauerstoff oder Schwefel unterbrochenes $C_7$-$C_{12}$-Phenylalkyl bedeutet, oder ferner die Reste $R_{17}$ und $R^1_{18}$ zusammen mit den Kohlenstoffatomen, an die sie gebunden sind, einen $C_5$-$C_8$-Cycloalkylenring bilden, und

$R_{25}$ $C_1$-$C_8$-Alkylen oder durch Sauerstoff unterbrochenes $C_2$-$C_8$-Alkylen bedeutet.

Speziell von besonderem Interesse sind Verbindungen der Formel (9), worin

$R_7$, $R_{10}$, $R_{17}$ und $R_{19}$ Wasserstoff darstellen, und
$R^1_{18}$ Wasserstoff, $C_1$-$C_4$-Alkyl, durch Sauerstoff unterbrochenes $C_2$-$C_8$-Alkyl; durch Sauerstoff unterbrochenes $C_7$-$C_9$-Phenylalkyl bedeutet, oder ferner die Reste $R_{17}$ und $R^1_{18}$ zusammen mit den Kohlenstoffatomen, an die sie gebunden sind, einen Cyclohexylenring bilden.

Die Verbindungen der Formeln (6) und (9) können auf an sich bekannte Weise hergestellt werden. Sowohl EP-A-146 269 als auch EP-A-397 170 beschreiben die Alkylierung der 4-Hydroxymandelsäure der Formel (11), worin $R_7$, $R_8$, $R_9$ und $R_{10}$ Wasserstoff bedeutet, mit Bromethanol unter basischen Bedingungen zur 4-(2-Hydroxyethoxy)mandelsäure.

(11) 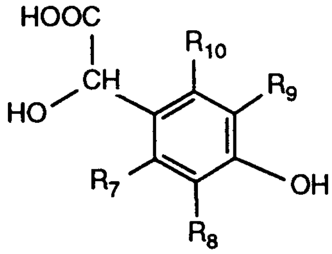

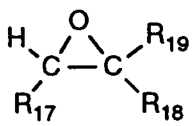 (12)

Des weiteren wurde nun gefunden, dass die Umsetzung von 4-Hydroxymandelsäuren der Formel (11) mit Epoxiden der Formel (12) sehr leicht und in guten Ausbeuten zu den 4-(2-Hydroxyethoxyphenyl)mandelsäuren der Formeln (6) und (9) erfolgt.

Ein weiterer Gegenstand der Erfindung ist daher auch ein neues Verfahren zur Herstellung von Verbindungen der Formel (6),

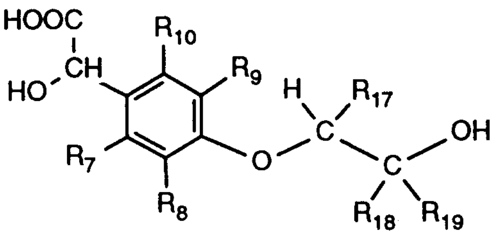 (6)

worin die allgemeinen Symbole wie in Formel (1) definiert sind und wenn $R_{17}$ und $R_{19}$ Wasserstoff sind, $R_{18}$ zusätzlich einen Rest der Formel (10)

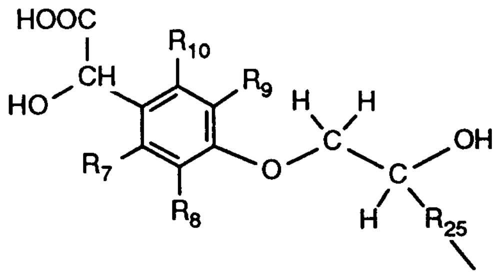 (10)

bedeutet, dadurch gekennzeichnet, dass eine 4-Hydroxymandelsäure der Formel (11) mit einem Epoxid der Formel (12),

(11) 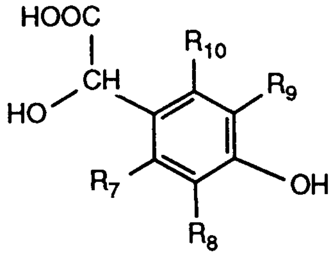 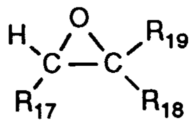 (12)

worin die Reste $R_7$, $R_8$, $R_9$, $R_{10}$, $R_{17}$, $R_{18}$ und $R_{19}$ die gleiche Bedeutung haben wie für Formel (1) beschrieben, und wenn $R_{17}$ und $R_{19}$ Wasserstoff sind, $R_{18}$ in Formel (12) zusätzlich einen Rest der Formel (10) oder einen Rest der Formel (16)

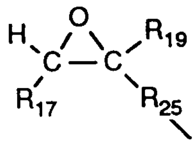 (16)

bedeutet, zu Verbindungen der Formel (6) umgesetzt wird.

Von besonderem Interesse ist ein Verfahren zur Herstellung von Verbindungen der Formel (6), worin $R_{18}$ die gleiche Bedeutung hat wie für Formel (1) beschrieben.

Die bevorzugten Reste $R_7$, $R_8$, $R_9$, $R_{10}$, $R_{17}$, $R_{18}$ und $R_{19}$ im Verfahren zur Herstellung der Verbindungen der Formel (6) sind die gleichen wie sie für die Verbindung der Formel (1) beschrieben sind.

Die Reaktion wird bevorzugt in Gegenwart einer Base in einem Temperaturbereich von 20 bis 200°C, insbesondere 50 bis 150°C, und unter leichtem Druck durchgeführt.

Die eingesetzte Base, beispielsweise Natriumhydroxid, wird in equimolaren Mengen bezüglich eingesetzter 4-Hydroxymandelsäure oder einem leichten Ueberschuss, insbesondere einem Ueberschuss von 1 bis 30 %, verwendet. Wird die 4-Hydroxymandelsäure in Form ihrer Salze eingesetzt, insbesondere Natriumsalze, wird entsprechend weniger Base verwendet.

Die Reaktion kann mit oder ohne Lösungsmittel durchgeführt werden. Die Verwendung eines Lösungsmittels, insbesondere Wasser, ist jedoch bevorzugt.

Ein besonders bevorzugtes Epoxid der Formel (12) ist Ethylenoxid.

In einem besonders bevorzugten Verfahren wird das Epoxid in einem molaren Ueberschuss von 1 bis 80 %, insbesondere 10 bis 60 %, bezüglich der eingesetzten 4-Hydroxymandelsäure der Formel (10) verwendet.

Die am Phenylring substituierten Mandelsäuren der Formel (11) sind in der Literatur bekannt oder können beispielsweise gemäss W. Bradley et al, J. Chem. Soc. 1956, 1622; EP-A-146269, EP-B-182507 (Beispiel 1, Seite 4) oder DE-A-2 944 295 in analoger Weise hergestellt werden.

Die Epoxide der Formel (12) sind in der Literatur bekannt oder können leicht durch Oxidation der entsprechenden Olefine mit Persäuren erhalten werden. Das besonders bevorzugte Ethylenoxid wird grosstechnisch hergestellt. Ebenfalls bevorzugt ist die Alkylierung eines Alkohols oder Phenols $R^2_{18}$ OH mit Epichlorhydrin (1-Chlor-2,3-epoxy-propan) zu den Epoxiden der Formel

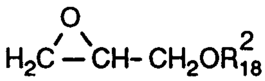 ,

wobei der Rest $-CH_2OR^2_{18}$ in den Definitionsbereich von $R_{18}$ fällt.

Selbstverständlich können auch die am Phenylring substituierten 4-Hydroxymandelsäuren der Formel (11) bei erhöhter Temperatur, insbesondere Temperaturen von 130 bis 200°C in der Schmelze oder in einem Lösungsmittel gegebenenfalls unter leichtem Vakuum, zuerst zu Verbindungen der Formel (13)

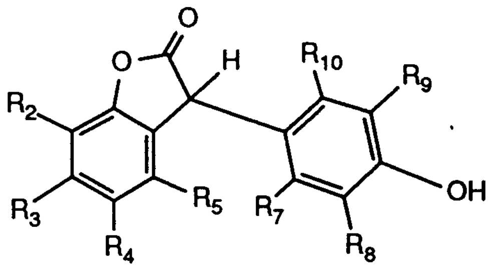 (13)

umgesetzt werden. Bevorzugt wird die Reaktion in einem Lösungsmittel wie beispielsweise Essigsäure, Propionsäure oder Ameisensäure in einem Temperaturbereich von 50 bis 130°C durchgeführt. Die Reaktion kann durch Zusatz einer Säure wie Salzsäure, Schwefelsäure oder Methansulfonsäure katalysiert werden. Die Umsetzung kann z.B. in der Weise durchgeführt werden, wie sie in den in der Beschreibungseinleitung angegebenen Literaturstellen beschrieben ist.

Die Umsetzung der Verbindungen der Formel (13) mit entweder den Epoxiden der Formel (12) oder Haloethanol (Bromethanol oder Chlorethanol) liefert anlog wie oben beschrieben die 3-(2-Hydroxyethoxyphenyl)benzofuran-2-one der Formel (7).

(14)

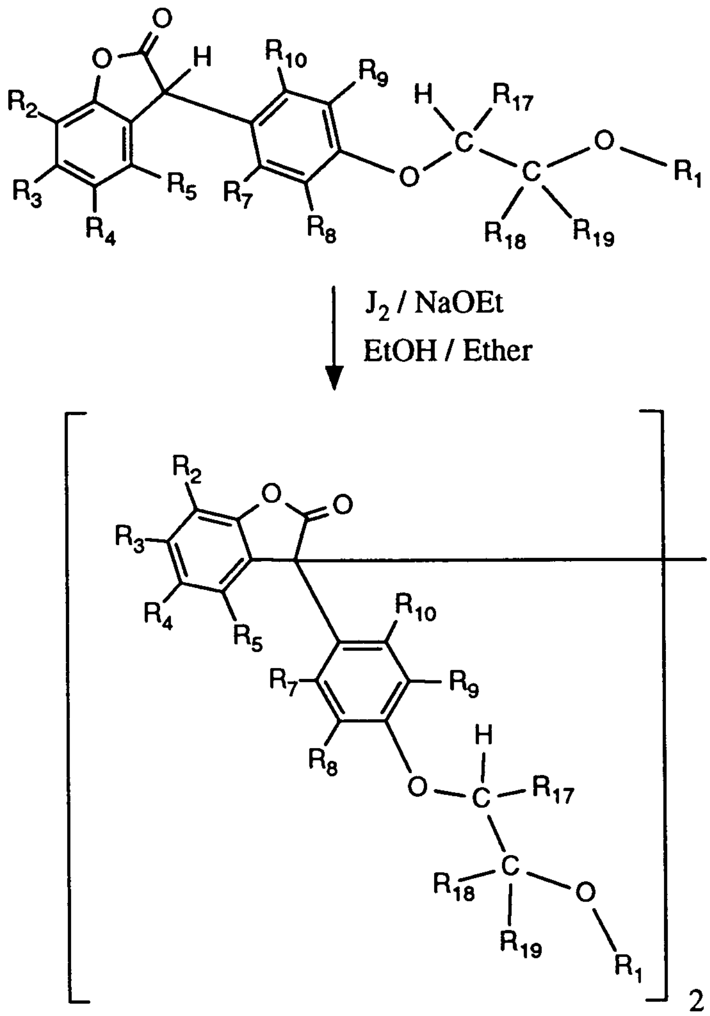


(15)

Die Dimerisierung der Verbindungen der Formel (14) zur Herstellung von Verbindungen der Formel (1), worin $R_6$ eine Gruppe der Formel (3) ist [Verbindungen der Formel (15)] erfolgt durch Oxidation mit beispielsweise Jod unter basischen Bedingungen in einem organischen Lösungsmittel bei Raumtemperatur. Als Base eignet sich besonders Natriumethylat, als Lösungsmittel Ethanol und Diethylether.

Die erfindungsgemässen Verbindungen der Formel (1) eignen sich zum Stabilisieren von organischen Materialien gegen thermischen, oxidativen oder lichtinduzierten Abbau.

Beispiele für derartige Materialien sind:

1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).

Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:

a) radikalisch (gewöhnlich bei hohem Druck und hoher Temperatur).

b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide,

Alkoholate, Ester, Ether, Amine, Alkyle, Alkenyle und/oder Aryle, die entweder π- oder σ-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten- 1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.

4. Kohlenwasserstoffharze (z.B. $C_5$-$C_9$) inklusive hydrierte Modifikationen davon (z.B. Klebrigmacherharze) und Mischungen von Polyalkylenen und Stärke.

5. Polystyrol, Poly-(p-methylstyrol), Poly-(α-methylstyrol).

6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.

8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo-und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.

10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-VinylhalogenidCopolymere oder Acrylnitril-Alkylmethacrylat-Butadien-Terpolymere.

11. Polymere, die sich von ungesättigten Alkoholen und Aminen bzw. deren Acylderivaten oder Acetalen ableiten, wie Polyvinylalkohol, Polyvinylacetat, -stearat, - benzoat, -maleat, Polyvinylbutyral, Polyallylphthalat, Polyallylmelamin; sowie deren Copolymere mit in Punkt 1 genannten Olefinen.

12. Homo- und Copolymere von cyclischen Ethern, wie Polyalkylenglykole, Polyethylenoxyd, Polypropylenoxyd oder deren Copolymere mit Bisglycidylethern.

13. Polyacetale, wie Polyoxymethylen, sowie solche Polyoxymethylene, die Comonomere, wie z.B. Ethylenoxid,

enthalten; Polyacetale, die mit thermoplastischen Polyurethanen, Acrylaten oder MBS modifiziert sind.

14. Polyphenylenoxide und -sulfide und deren Mischungen mit Styrolpolymeren oder Polyamiden.

15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

17. Polyharnstoffe, Polyimide, Polyamid-imide und Polybenzimidazole.

18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

19. Polycarbonate und Polyestercarbonate.

20. Polysulfone, Polyethersulfone und Polyetherketone.

21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

22. Trocknende und nicht-trocknende Alkydharze.

23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

24. Vernetzbare Acrylharze, die sich von substituierten Acrylsäureestern ableiten, wie z.B. von Epoxyacrylaten, Urethan-acrylaten oder Polyester-acrylaten.

25. Alkydharze, Polyesterharze und Acrylatharze, die mit Melaminharzen, Harnstoffharzen, Polyisocyanaten oder Epoxidharzen vernetzt sind.

26. Vernetzte Epoxidharze, die sich von Polyepoxiden ableiten, z.B. von Bis-glycidylethern oder von cycloaliphatischen Diepoxiden.

27. Natürliche Polymere, wie Cellulose, Naturkautschuk, Gelatine, sowie deren polymerhomolog chemisch abgewandelte Derivate, wie Celluloseacetate, -propionate und - butyrate, bzw. die Celluloseether, wie Methylcellulose; sowie Kolophoniumharze und Derivate.

28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

29. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

30. Wässrige Emulsionen natürlicher oder synthetischer Kautschuke, wie z.B. Naturkautschuk-Latex oder Latices von carboxylierten Styrol-Butadien-Copolymeren.

Weitere Gegenstände der Erfindung sind daher auch Zusammensetzungen enthaltend ein dem oxidativen, thermischen oder lichtinduzierten Abbau unterworfenes organisches Material und mindestens eine Verbindung der Formel (1).

Bevorzugte organische Materialien sind Polymere, z.B. synthetische Polymere, insbesondere thermoplastische Polymere. Besonders bevorzugt sind Polyacetale oder Polyolefine, z.B. Polypropylen oder Polyethylen.

Besonders hervorzuheben ist die Wirkung der erfindungsgemässen Verbindungen gegen thermischen und oxidativen Abbau, vor allem bei thermischer Belastung, wie sie bei der Verarbeitung von Thermoplasten auftritt. Die erfindungsgemässen Verbindungen sind daher hervorragend als Verarbeitungsstabilisatoren einzusetzen.

Vorzugsweise werden die Verbindungen der Formel (1) dem zu stabilisierenden Material in Mengen von 0,0005 bis 5 %, insbesondere 0,001 bis 2 %, beispielsweise 0,01 bis 2 %, zugesetzt, bezogen auf das Gewicht des zu stabilisierenden organischen Materials.

Zusätzlich zu den Verbindungen der Formel (1) können die erfindungsgemässen Zusammensetzungen weitere Costabilisatoren enthalten, wie beispielsweise die folgenden:

1. Antioxidantien

1.1. Alkylierte Monophenole, z.B. 2,6-Di-tert-butyl-4-methylphenol, 2-butyl-4,6-di-methylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclo-hexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyl-oxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.

1.4. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3-,6-di-sec.-amylphenol), 4,4'-Bis-(2,6-di-methyl-4-hydroxyphenyl)-disulfid.

1.5. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphend], 2,2'-Methylen-bis-[6-(α,α-di-methylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.

1.6. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-di-thioterephthalat, Bis-(3,5-di-tert-butyl-4-hydroxybenzyl)-sulfid, Isooctyl-3,5-di-tert-butyl-4-hydroxybenzyl-mercaptoacetat.

1.7. Hydroxybenzylierte Malonate, z.B. Dioctadecyl-2,2-bis-(3,5-di-tert-butyl-2-hydroxy-benzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxy-benzyl)-malonat.

1.8. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.

1.9. Triazinverbindungen, z.B. 2,4-Bis-octylmercapto-6-(3,5-di-tert-butyl4-hydroxy-anilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin,1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin,1,3,5-Tris-(3,5-dicyclohexy-4-hydroxybenzyl)-isocyanurat.

1.10. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

1.11. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.12. Ester der β-(3.5-Di-tert-butyl-4-hydroxyphenyl)-propionsäure mit ein- oder mehr wertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.13. Ester der β-(5-tert-Butyl-4-hydroxy-3-methylphenyl)-propionsäure mit ein-oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(3,5-Dicyclohexyl-4-hydroxyphenyl)-propionsaüre mit ein-oder mehr wertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der 3,5-Di-tert-butyl-4-hydroxyphenylessigsäure mit ein-oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Amide der β-(3,S-Di-tert-butyl-4-hydroxyphenyl)-propionsäure, wie z.B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

2. UV-Absorber und Lichtschutzmittel

2.1.-Hydroxyphenyl)-benztriazole, wie z.B. 2-(2'-Hydroxy-5'-methylphenyl)-benztriazol, 2-(3',5'-Di-tert-butyl-2'-hydroxyphenyl)-benztriazol, 2-(5'-tert-Butyl-2'-hydroxyphenyl)-benztriazol, 2-(2'-Hydroxy-5'-(1,1,3,3-tetramethylbutyl)phenyl)-benztriazol, 2-(3',5'-Di-tert-butyl-2'-hydroxyphenyl)-5-chlor-benztriazol, 2-(3'-tert-Butyl- 2'-hydroxy-5'-methylphenyl)-5-chlor-benztriazol, 2-(3'-sec-Butyl-5'-tert-butyl-2'-hydroxyphenyl)-benztriazol, 2-(2'-Hydroxy-4'-octoxyphenyl)-benztriazol, 2-(3',5'-Di-tert-amyl-2' -hydroxyphenyl)-benztriazol, 2-(3',5'-Bis-(α,α-dimethylbenzyl)-2'-hydroxyphenyl)-benztriazol, Mischung aus 2-(3'-tert-Butyl-2'-hydroxy-5'-(2-octyloxycarbonylethyl)-phenyl)-5-chlor-benztriazol, 2-(3'-tert-Butyl-5'-[2-(2-ethylhexyloxy)-carbonylethyl]-2'-hydroxyphenyl)-5-chlor-benztriazol, 2-(3'-tert-Butyl-2'-hydroxy-5'-(2-methoxycarbonylethyl)phenyl)-5-chlor-benztriazol, 2-(3'-tert-Butyl-2'-hydroxy-5'-(2-methoxycarbonylethyl)phenyl)-benztriazol, 2-(3'-tert-Butyl-2'-hydroxy-5'-(2-octyloxycarbonylethyl) phenyl)-benztriazol, 2-(3'-tert-Butyl-5'-[2-(2-ethylhexyloxy)carbonylethyl]-2'-hydroxyphenyl)-benztriazol, 2-(3'-Dodecyl-2'-hydroxy-5'-methylphenyl)-benztriazol, und 2-(3' tert-Butyl-2'-hydroxy-5'-(2-isooctyloxycarbonylethyl)phenyl-benztriazol, 2,2'-Methylen-bis[4-(1,1,3,3-tetramethylbutyl)-6-benztriazol-2-yl-phenol]; Umesterungsprodukt von 2-[3'-tert-Butyl-5'-(2-methoxycarbonylethyl)-2'-hydroxy-phenyl]-benztriazol mit Polyethylenglycol 300; [R-$CH_2CH_2$-COO($CH_2$) mit R = 3'-tert-Butyl-4'-hydroxy-5'-2H-benztriazol-2-yl-phenyl.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.

2.4. Acrylate, wie z.B. α-Cyan-β,β-diphenylacrylsäure-ethylester bzw. - isooctylester, α-Carbomethoxy-zimtsäuremethylester, α-Cyano-,β-methyl-p-methoxy-zimtsäuremethylester bzw. -butylester, α-Carbomethoxy-p-methoxy-zimtsäure-methylester, N-(β-Carbomethoxy-,β-cyanovinyl)-2-methyl-indolin.

2.5. Nickelverbindungen, wie z.B. Nickelkomplexe des 2,2'-Thio-bis-[4-(1,1,3,3-tetra-methylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim , Nickelkomplexe des 1-Phenyl-4-lauroyl-5-hydroxy-pyrazols, gegebenenfalls mit zusätzlichen Liganden.

2.6. Sterisch behinderte Amine, wie z.B. Bis-(2,2,6,6-tetramethyl-piperidyl)-sebacat, Bis-(2,2,6,6-tetramethylpiperidyl)-succinat, Bis-(1,2,2,6,6-pentamethylpiperidyl)-sebacat, n-Butyl-3,5-di-tert-butyl-4-hydroxybenzylmalonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl- 1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.

2.7. Oxalsäurediamide, wie z.B. 4,4'-Di-octyloxy-oxanilid, 2,2'-Di-octyloxy-5,5'-di-tert-butyl-oxanilid, 2,2'-Di-dodecyloxy-5,5,di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-di-benz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.

5. Peroxidzerstörende Verbindunnen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.

8. Nukleierungsmittel, wie z.B. 4-tert-Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.

10. Sonstige Zusätze, wie z.B. Weichmacher, Gleitmittel, Emulgatoren, Pigmente, Optische Aufheller, Flammschutzmittel, Antistatika, Treibmittel.

Die Costabilisatoren werden beispielsweise in Konzentrationen von 0,01 bis 10 %, bezogen auf das Gesamtgewicht des zu stabilisierenden Materials, zugesetzt.

Die erfindungsgemässen Verbindungen der Formel (1) können insbesondere zusammen mit phenolischen Antioxidantien eingesetzt werden. Die erfindungsgemässen Zusammensetzungen enthalten daher vorzugsweise neben Verbindungen der Formel (1) phenolische Antioxidantien, insbesondere solche, wie sie in den obigen Punkten

1.1 bis 1.16 aufgelistet sind.

Andere bevorzugte Zusammensetzungen enthalten neben den Verbindungen der Formel (1) mindestens ein organisches Phosphit oder Phosphonit.

Die Einarbeitung der Verbindungen der Formel (1) sowie gegebenenfalls weiterer Additive in das polymere, organische Material erfolgt nach bekannten Methoden, beispielsweise vor oder während der Formgebung oder auch durch Aufbringen der gelösten oder dispergierten Verbindungen auf das polymere, organische Material, gegebenenfalls unter nachträglichem Verdunsten des Lösungsmittels. Die Verbindungen der Formel (1) können auch in Form eines Masterbatches, der diese beispielsweise in einer Konzentration von 2,5 bis 25 Gew.-% enthält, den zu stabilisierenden Materialien zugesetzt werden.

Die Verbindungen der Formel (1) können auch vor oder während der Polymerisation oder vor der Vernetzung zugegeben werden.

Die Verbindungen der Formel (1) können in reiner Form oder in Wachsen, Oelen oder Polymeren verkapselt in das zu stabilisierende Material eingearbeitet werden.

Die Verbindungen der Formel (1) können auch auf das zu stabilisierende Polymer aufgesprüht werden. Sie sind in der Lage, andere Zusätze (z.B. die oben angegebenen herkömmlichen Additive) bzw. deren Schmelzen zu verdünnen, so dass sie auch zusammen mit diesen Zusätzen auf das zu stabilisierende Polymer aufgesprüht werden können. Besonders vorteilhaft ist die Zugabe durch Aufsprühen während der Desaktivierung der Polymerisationskatalysatoren, wobei z.B. der zur Desaktivierung verwendete Dampf zum Versprühen verwendet werden kann.

Bei kugelförmig polymerisierten Polyolefinen kann es z.B. vorteilhaft sein, die Verbindungen der Formel (1), gegebenenfalls zusammen mit anderen Additiven, durch Aufsprühen zu applizieren.

Eine bevorzugte Ausführungsform der vorliegenden Erfindung ist daher die Verwendung von Verbindungen der Formel (1) zum Stabilisieren von organischen Materialien gegen oxidativen, thermischen oder lichtinduzierten Abbau.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Die vorliegende Erfindung betrifft auch ein Verfahren zum Stabilisieren eines organischen Materials gegen oxidativen, thermischen oder lichtinduzierten Abbau, das dadurch gekennzeichnet ist, dass man diesem mindestens eine Verbindung der Formel (1) einverleibt oder auf dieses aufbringt.

Wie bereits hervorgehoben, werden die erfindungsgemässen Verbindungen besonders vorteilhaft als Stabilisatoren in Polyolefinen eingesetzt, vor allem als Thermostabilisatoren. Ausgezeichnete Stabilisierung wird z.B. dann erhalten, wenn man sie in Kombination mit organischen Phosphiten oder Phosphoniten einsetzt. Dabei weisen die erfindungsgemässen Verbindungen den Vorteil auf, dass sie bereits in ausserordentlich geringen Mengen wirksam sind. Sie werden z.B. in Mengen von 0,0001 bis 0,015, insbesondere 0,0001 bis 0,008 Gew. % bezogen auf das Polyolefin, eingesetzt. Das organische Phosphit oder Phosphonit wird zweckmässig in einer Menge von 0,01 bis 2, insbesondere 0,01 bis 1 Gew. %, ebenfalls bezogen auf das Polyolefin, eingesetzt. Als organische Phosphite bzw. Phosphonite werden vorzugsweise solche eingesetzt, wie sie in DE-A- 4 202 276 beschrieben sind. Siehe dort insbesondere die Patentansprüche, die Beispiele sowie die Seiten 5, letzter Absatz bis Seite 8. Besonders zweckmässige Phosphite und Phosphonite sind auch Punkt 4 der obigen Auflistung von Costabilisatoren zu entnehmen.

Die folgenden Beispiele erläutern die Erfindung weiter. Angaben in Teilen oder Prozenten beziehen sich auf das Gewicht.

<u>Beispiel 1</u>: Herstellung von 3-[4-(2-Acetoxyethoxy)phenyl]-5,7-di-tert-butyl-benzofuran-2-on (Verbindung (101), Tabelle 1).

Eine unter Stickstoffatmosphäre gerührte Suspension von 154,7 g (0,75 Mol) 2,4-Di-tert-butyl-phenol und 106,1 g (0,50 Mol) 4-(2-Hydroxyethoxy)-mandelsäure (Verbindung (201), Beispiel 10, Tabelle 2) in 200 ml mit Salzsäure-Gas gesättigter Essigsäure wird während 8 Stunden unter Rückfluss gekocht. Die Essigsäure wird anschliessend am Vakuumrotationsverdampfer abdestilliert, der Rückstand mit 15 ml (0,21 Mol) Acetylchlorid versetzt und während 20 Minuten bei 120°C gehalten. Das Reaktionsgemisch wird erneut am Vakuumrotationsverdampfer eingeengt, der Rückstand mit 400 ml Methanol versetzt und bei ca. -8°C stehen gelassen. Die ausgefallenen Kristalle werden filtriert, mit 250 ml kaltem Methanol gewaschen und getrocknet. Es resultieren 176,3 g (83 %) 3-[4-(2-Acetoxyethoxy)phenyl]-5,7-di-tert-butyl-benzofuran-2-on, Smp. 93-96°C (Verbindung (101), Tabelle 1). Umkristallisation aus Ligroin liefert die Verbindung (101) in zwei Kristallformen. Kristallform A: Smp. 75-78°C, Schmelzenthalpie 62,4 Joule/g. Kristallform B: Smp. 93-96°C, Schmelzenthalpie 118,2 Joule/g.

In Analogie zu Beispiel 1 werden aus den entsprechenden Phenolen (beispielsweise 4-tert-Butyl-phenol, 1-Naphtol, 2-(Hexadec-2-yl)-4-tert-butyl-phenol oder 2,4-Dicyclohexyl-phenol), Mandelsäuren (Beispiele 10 und 11), Carbonsäure-Lösungsmittel (beispielsweise Ameisensäure, Essigsäure oder Propionsäure) und Säurechlonden die

Verbindungen (102), (103), (104), (112), (118), (123), (127), (128), (136), (137), (138), (139) und (140) hergestellt (vgl. Tabelle 1). Die Verbindung (123) wird in Ameisensäure anstelle von Essigsäure ohne Zugabe eines Säurechlorids hergestellt.

Beispiel 2: Herstellung von 3-[4-(2-Hydroxyethoxy)phenyl]-5-methyl-benzofuran-2-on (Verbindung (119), Tabelle 1).

Eine Suspension von 8,5 g (40,0 mMol) 4-(2-Hydroxyethoxy)-mandelsäure (Verbindung (201), Beispiel 10, Tabelle 2) und 12,0 g (110 mMol) p-Kresol wird unter Stickstoffatmosphäre während 75 Minuten bei 180°C gehalten, wobei das gebildete Wasser abdestilliert. Das überschüssige p-Kresol wird anschliessend am Vakuumrotationsverdampfer abdestilliert. Chromatographie des Rückstandes an Kieselgel mit dem Laufmittelsystem Dichlormethan/Ethylacetat = 9:1 liefert 6,6 g (58 %) 3-[4-(2-Hydroxyethoxy)phenyl]-5-methylbenzofuran-2-on, gelbliches Harz (Verbindung (119), Tabelle 1).

In Analogie zu Beispiel 2 werden aus den entsprechenden Phenolen und Mandelsäuren (Beispiel 12) die Verbindungen (113) und (114) hergestellt (vgl. Tabelle 1).

Beispiel 3: Herstellung von 5,7-Di-tert-butyl-3-[4-(2-hydroxyethoxy)phenyl]-benzofuran-2-on (Verbindung (105), Tabelle 1).

a) Durch Hydrolyse von 3-[4-(2-Acetoxyethoxy)phenyl]-5,7-di-tert-butyl-benzofuran-2-on (Beispiel 1, Verbindung (101), Tabelle 1).

Eine Lösung von 170 g (0,40 Mol) 3-[4-(2-Acetoxyethoxy)phenyl]-5,7-di-tert-butyl-benzofuran-2-on (Beispiel 1) in 1000 ml Methanol und 40 ml konzentrierter Salzsäure wird während 15 Stunden unter Rückfluss gekocht. Das Reaktionsgemisch wird anschliessend durch Abdestillieren von ca. 600 ml Methanol aufkonzentriert und im Kühlschrank stehen gelassen. Die ausgefallenen Kristalle werden filtriert, mit 200 ml kaltem Methanol gewaschen und getrocknet. Es resultieren 137,5 g (90 %) 5,7-Di-tert-butyl-3-[4-(2-hydroxyethoxy)phenyl]-benzofuran-2-on, Smp. 132-135 (Verbindung (105), Tabelle 1).

In Analogie zu Beispiel 3a wird aus Verbindung (104) (Beispiel 1) das 5,7-Di-tert-butyl-3-[3,5-dimethyl-4-(2-hydroxyethoxy)phenyl]-benzofuran-2-on (Verbindung (106), Tabelle 1) hergestellt.

b) Durch Hydroxyethylierung von 5,7-Di-tert-butyl-3-(4-hydroxyphenyl)-benzofuran-2-on.

Zu einer auf 80°C gewärmten Lösung von 3,38 g (10,0 mMol) 5,7-Di-tert-butyl-3-(4-hydroxyphenyl)-benzofuran-2-on in 30 ml 1N Natriumhydroxid-Lösung wird 1,0 ml (15,0 mMol) 2-Chlorethanol gegeben. Das Reaktionsgemisch wird anschliessend noch 2 Stunden bei 80°C gehalten, dann mit 50 ml 1N Salzsäure versetzt, 1 Stunde nachgerührt, abgekühlt und mit Dichlormethan extrahiert. Die organischen Phasen werden mit Wasser gewaschen, vereinigt, über Natriumsulfat getrocknet und am Vakuumrotationsverdampfer eingeengt. Kristallisation des Rückstandes aus 8 ml Ethanol und 2 ml Wasser liefert 2,34 g (61 %) 5,7-Di-tert-butyl-3-[4-(2-hydroxyethoxy)phenyl]-benzofuran-2-on Smp. 132-135°C (Verbindung (105), Tabelle 1).

Das als Ausgangsmaterial benötigte 5,7-Di-tert-butyl-3-(4-hydroxyphenyl)-benzofuran-2-on wird folgendermassen hergestellt:

Ein Gemisch von 103,2 g (0,50 Mol) 2,4-Di-tert-butylphenol und 102,4 g (0,55 Mol) 4-Hydroxymandelsäure Monohydrat in 100 ml Essigsäure wird unter Stickstoffatmosphäre während 24 Stunden am Rückfluss gekocht. Das Reaktionsgemisch wird anschliessend mit 140 ml 50 % wässriger Essigsäure verdünnt, abgekühlt und der ausgefallene Niederschlag abfiltriert. Der Rückstand wird mit weiteren 200 ml 50 % wässriger Essigsäure gewaschen und anschliessend getrocknet. Es resultieren 95,9 g (57 %) 5,7-Di-tert-butyl-3-(4-hydroxyphenyl)benzofuran-2-on, Smp. 187-190°C.

Beispiel 4: Herstellung von 1,7-Di-tert-butyl-3-[4-(2-stearoyloxyethoxy)phenyl]-benzofuran-2-on (Verbindung (107), Tabelle 1).

Eine Suspension von 11,4 g (30 mMol) 5,7-Di-tert-butyl-3-[4-(2-hydroxyethoxy)phenyl]-benzofuran-2-on (Verbindung (105), Beispiel 3) und 9,4 g (31 mMol) Stearoylchlorid in 60 ml Toluol wird während 4 Stunden unter Rückfluss gekocht. Das Reaktionsgemisch wird anschliessend am Vakuumrotationsverdampfer eingeengt und der Rückstand aus Methanol umkristallisiert. Es resultieren 17,3 g (89 %) 5,7-Di-tert-butyl-3-[4-(2-stearoyloxyethoxy)phenyl]-benzofuran-2-on, Smp. 54-60°C (Verbindung (107), Tabelle 1).

In Analogie zu Beispiel 4 werden aus den entsprechenden Benzofuranonen und Säurechloriden die Verbindungen (108), (121), (122), (124), (125), (133), (134) und (141) hergestellt.

Beispiel 5: Herstellung des Terephtalsäureester-Derivats (Verbindung (111), Tabelle 1).

Eine Suspension von 4,0 g (20 mMol) Terephtalsäuredimethylester, 16,0 g (42 mMol) 5,7-Di-tert-butyl-3-[4-(2-hydroxyethoxy)phenyl]-benzofuran-2-on (Verbindung (105), Beispiel 3) und 300 mg Dibutylzinnoxid wird unter Stickstoffatmosphäre während 30 Minuten bei 170°C gerührt, wobei das entstehende Methanol abdestilliert. Anschliessend wird die Temperatur auf 240°C erhöht und noch 1,5 Stunden unter leichtem Vakuum (50 mbar) weitergerührt. Nach dem Entfernen des Heizbades werden durch den Kühler zur Schmelze 20 ml Chlorbenzol und danach 75 ml Isopropanol gegossen. Das Reaktionsgemisch wird mit Eis/Wasser abgekühlt. Das ausgefallene Produkt wird filtriert, mit kaltem Isopropanol gewaschen und getrocknet. Es resultieren 15,6 g (87 %) der Verbindung (111) (Tabelle 1), Smp. 248-251°C.

In Analogie zu Beispiel 5 werden aus den entsprechenden Estern und Benzofuranonen die Verbindungen (109), (110), (115), (117), (120), (142), (143) und (144) hergestellt.

Beispiel 6: Herstellung des Bernsteinsäureester-Derivats (Verbindung (116), Tabelle 1).

Eine Suspension von 7,65 g (20,0 mMol) 5,7-Di-tert-butyl-3-[4-(2-hydroxyethoxy)phenyl]-benzofuran-2-on (Verbindung (105), Beispiel 3), 1,0 g (10,0 mMol) Bernsteinsäureanhydrid und 1 Tropfen Methansulfonsäure wird unter Stickstoffatmosphäre während 30 Minuten bei 150°C gehalten. Anschliessend wird unter leichtem Vakuum (50 mbar) noch 2 Stunden bei 150°C weitergerührt. Das Reaktionsgemisch wird abgekühlt und an Kieselgel mit dem Laufmittelsystem Dichlormethan/Hexan = 19:1 chromatographiert. Kristallisation der reinen Fraktionen aus Ethanol liefert 6,5 g (77 %) der Verbindung (116) (Tabelle 1), Smp. 145-163°C.

In Analogie zu Beispiel 6 wird ausgehend von Thiodipropionsäure anstelle von Bernsteinsäureanhydrid die Verbindung (131) hergestellt.

Beispiel 7: Herstellung von 5,7-Di-tert-butyl-3-[4-(2-methylaminocarboxyethoxy)-phenyl]-benzofuran-2-on (Verbindung (129), Tabelle 1).

Eine Suspension von 3,83 g (10,0 mMol) 5,7-Di-tert-butyl-3-[4-(2-hydroxyethoxy)phenyl]-benzofuran-2-on (Verbindung (105), Beispiel 3), 0,60 ml (10,0 mMol) Methylisocyanat und 100 mg Dibutylzinnoxid wird während 3 Stunden bei Raumtemperatur gerührt und anschliessend am Vakuumrotationsverdampfer eingeengt. Kristallisation des Rückstandes aus 10 ml Ethanol liefert 2,3 g (52 %) 5,7-Di-tert-butyl-3-[4-(2-methylaminocarboxyethoxy)phenyl]-benzofuran-2-on, Smp. 115-121°C (Verbindung (129), Tabelle 1).

In Analogie zu Beispiel 7 wird ausgehend von einem halben Aequivalent Hexamethylendiisocyanat anstelle von Methylisocyanat die Verbindung (132) hergestellt.

Beispiel 8: Herstellung der Verbindung (130) (Tabelle 1).

Eine Suspension von 11,5 g (30,0 mMol) 5,7-Di-tert-butyl-3-[4-(2-hydroxyethoxy)phenyl]-benzofuran-2-on (Verbindung (105), Beispiel 3), 5,3 ml (90,0 mMol) Methylisocyanat und 200 mg Dibutylzinnoxid in 25 ml Toluol wird während 30 Minuten unter Rückfluss gekocht. Das Reaktionsgemisch wird anschliessend am Vakuumrotationsverdampfer eingeengt. Zweimalige Umkristallisation des Rückstandes aus jeweils 25 ml Methanol liefert 8,9 g (59 %) der Verbindung (130), Smp. 142-144°C.

Beispiel 9: Herstellung von 3,3'-Bis-[5,7-di-tert-butyl-3-(4-[2-hydroxyethoxy]phenyl)benzofuran-2-on] (Verbindung (135), Tabelle 1).

Zu einer Natriumethylat-Lösung, hergestellt durch Zugabe von 0,69 g (30,0 mMol) Natrium in 40 ml absolutem Ethanol, wird unter Stickstoffatmosphare 11,48 g (50 mMol) 5,7-Di-tert-butyl-3-[4-(2-hydroxyethoxy)phenyl]-benzofuran-2-on (Verbindung (105), Beispiel 3) gegeben. Anschliessend wird bei Raumtemperatur während ca. 10 Minuten eine Lösung von 3,8 g (15,0 mMol) Jod in 40 ml Diethylether zugetropft. Das Reaktionsgemisch wird noch 30 Minuten nachgerührt, danach mit 200 ml Wasser verdünnt und dreimal mit je 50 ml Diethylether extrahiert Die organischen Phasen werden abgetrennt, mit Wasser gewaschen, vereinigt, über Magnesiumsulfat getrocknet und am Vakuumrotationsverdampfer eingeengt. Kristallisation des Rückstandes aus Ligroin/Dichlormethan liefert 10,3 g (90 %) des 3,3'-Bis-[5,7-di-tert-butyl-3-(4-[2-hydroxyethoxy]phenyl)-benzofuran-2-on], Smp. 212-218°C (Verbindung (135), Tabelle 1).

Die strukturelle Abkürzung der Formeln in Tabelle 1 und 2, wie beispielsweise

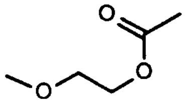 oder 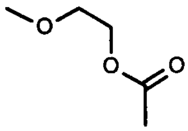 , bedeutet $-OCH_2CH_2O\overset{O}{\overset{\|}{C}}-$ .

Tabelle 1:

| Nr. | Verbindung | Smp. (°C) | C (%), H (%) (berechnet/gefunden) | Ausbeute (%) |
|---|---|---|---|---|
| 101 | 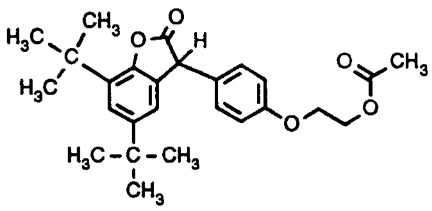 | 93-96 | 73,56 7,60<br>73,54 7,60 | 83 |
| 102 | 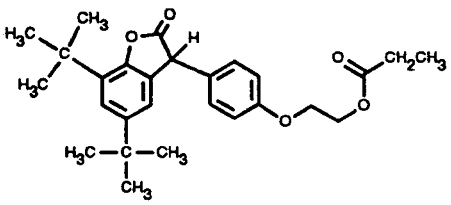 | 55-60 | 73,95 7,81<br>73,84 7,88 | 87 |
| 103 | 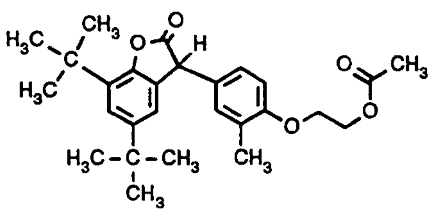 | 124-128 | 73,95 7,81<br>73,84 7,82 | 70 |
| 104 | 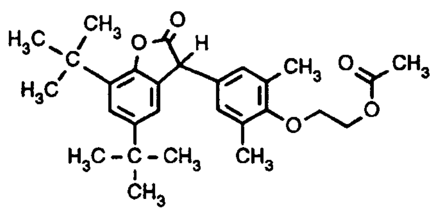 | 86-92 | 74,31 8,02<br>74,26 8,16 | 49 |

Tabelle 1: (Fortsetzung)

| Nr. | Verbindung | Smp. (°C) | C (%), H (%) (berechnet/gefunden) | Ausbeute (%) |
|---|---|---|---|---|
| 105 | 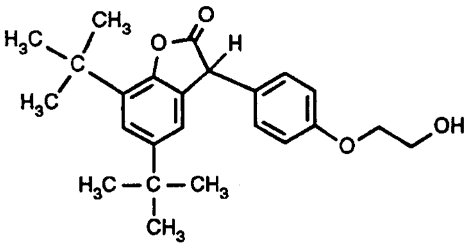 | 132-135 | 75,36 7,91<br>75,05 7,90 | 90 |
| 106 | 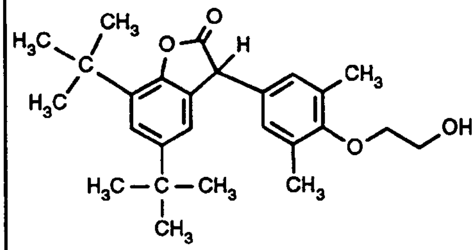 | Harz | 76,06 8,35<br>76,00 8,35 | ~100 |
| 107 | 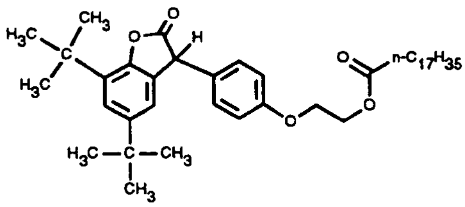 | 54-60 | 77,73 9,94<br>77,75 9,96 | 89 |
| 108 | 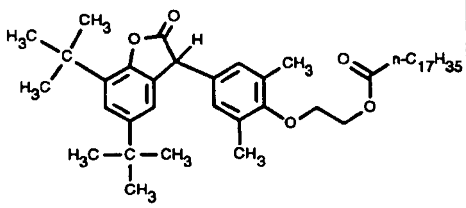 | Harz | 78,06 10,12<br>78,11 10,16 | 80 |

Tabelle 1: (Fortsetzung)

| Nr. | Verbindung | Smp. (°C) | C (%), H (%) (berechnet/gefunden) | Ausbeute (%) |
|---|---|---|---|---|
| 109 | 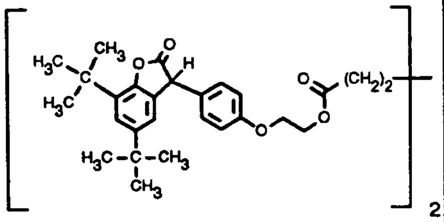 | 130-142 | 74,12 7,60<br>74,02 7,67<br>Diastereomeren-Gemisch | 58 |
| 110 | 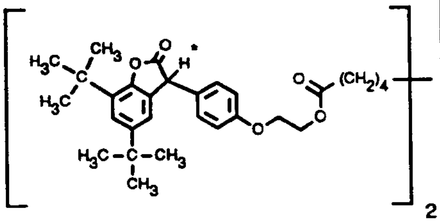 | Harz | Charakterisiert durch $^{1}$H-NMR ($CDCl_3$)<br>$\delta (H^{*}) = 4{,}78$ ppm<br>Diastereomeren-Gemisch | 47 |
| 111 | 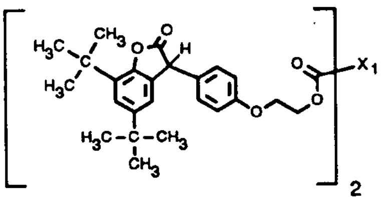<br>$X_1$ = 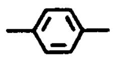 | 248-251 | 75,14 6,98<br>75,04 7,04<br>Diastereomeren-Gemisch | 87 |
| 112 | 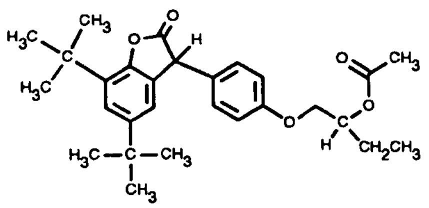 | Harz | 74,31 8,02<br>74,29 8,12<br>Diastereomeren-Gemisch | 32 |

Tabelle 1: (Fortsetzung)

| Nr. | Verbindung | Smp. (°C) | C (%), H (%) (berechnet/gefunden) | Ausbeute (%) |
|---|---|---|---|---|
| 113 | 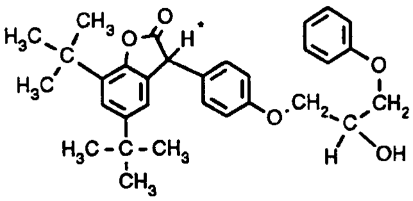 | Harz | Charakterisiert durch $^1$H-NMR ($CDCl_3$) $\delta$ ($H^*$) = 4,78 ppm<br>Diastereomeren-Gemisch | 43 |
| 114 | 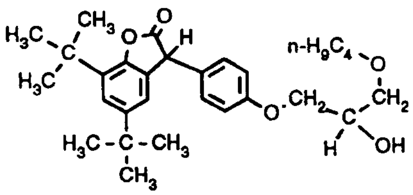 | Harz | 74,33 8,60<br>74,28 8,58<br>Diastereomeren-Gemisch | 23 |
| 115 | 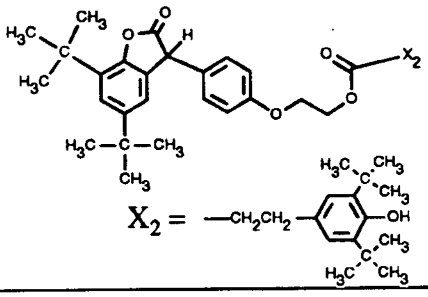 | 137-140 | 76,60 8,47<br>76,59 8,50 | 65 |
| 116 | 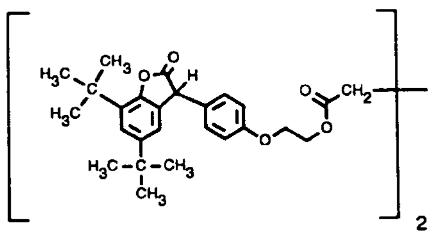 | 145-163 | 73,73 7,38<br>73,70 7,40<br>Diastereomeren-Gemisch | 77 |

Tabelle 1: (Fortsetzung)

| Nr. | Verbindung | Smp. (°C) | C (%), H (%) (berechnet/gefunden) | Ausbeute (%) |
|---|---|---|---|---|
| 117 | 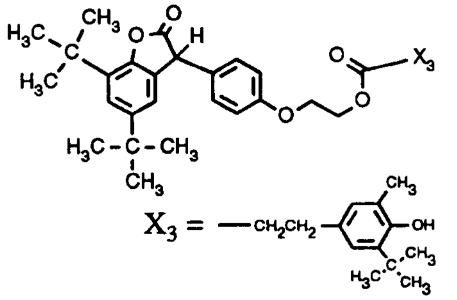 $X_3$ = | 127-129 | 75,97 8,05<br>76,01 8,00 | 75 |
| 118 | 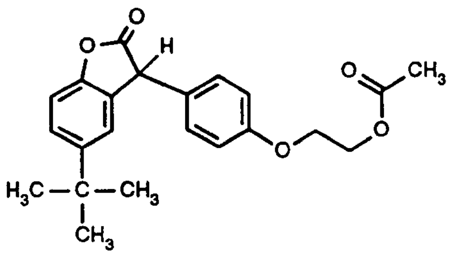 | 98-100 | 71,72 6,57<br>71,53 6,71 | 43 |
| 119 | 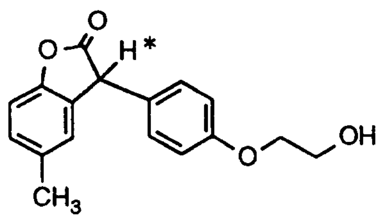 | Harz | Charakterisiert durch $^1$H-NMR ($CDCl_3$) $\delta$ (H*) = 4,79 ppm | 58 |
| 120 | 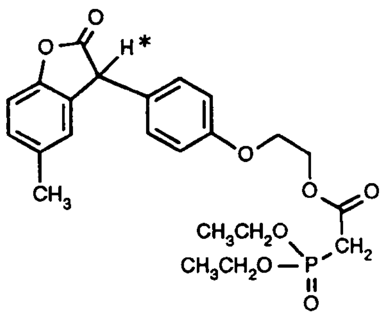 | Harz | Charakterisiert durch $^1$H-NMR ($CDCl_3$) $\delta$ (H*) = 4,80 ppm | 83 |

Tabelle 1: (Fortsetzung)

| Nr. | Verbindung | Smp. (°C) | C (%), H (%) (berechnet/gefunden) | Ausbeute (%) |
|---|---|---|---|---|
| 121 | 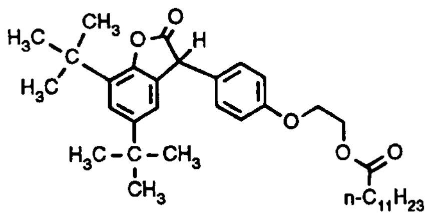 | Oel | 76,56 9,28<br>76,41 9,38 | 86 |
| 122 | 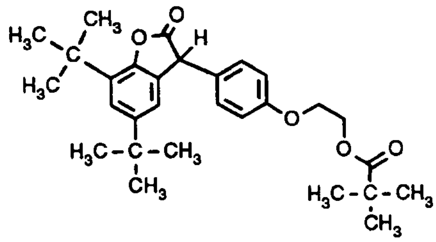 | 102-105 | 74,65 8,21<br>74,78 8,21 | 91 |
| 123 | 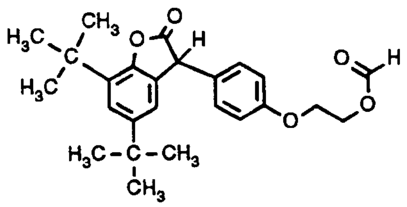 | 85-89 | 73,15 7,37<br>73,13 7,38 | 45 |
| 124 | 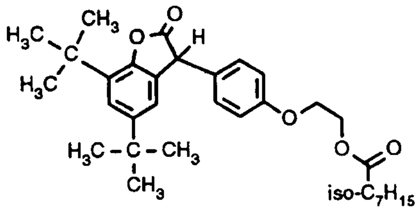 | Harz | 75,11 9,26<br>75,43 8,77 | 81 |

Tabelle 1: (Fortsetzung)

| Nr. | Verbindung | Smp. (°C) | C (%), H (%) (berechnet/gefunden) | Ausbeute (%) |
|---|---|---|---|---|
| 125 | 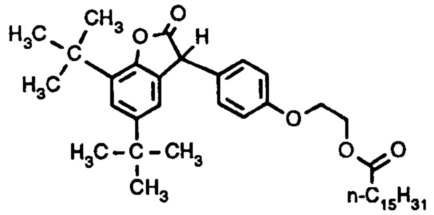 | 54-57 | 77,38 9,74<br>77,31 9,74 | 88 |
| 126 | 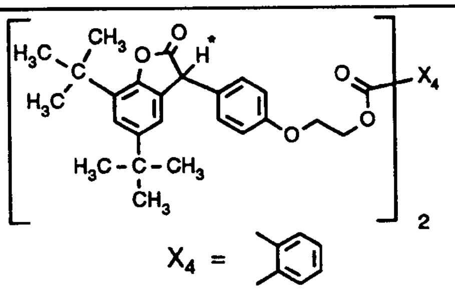 | 93-98 | Charakterisiert durch $^1$H-NMR ($CDCl_3$) δ ($H^*$) = 4,77 ppm Diastereomeren-Gemisch | 22 |
| 127 | 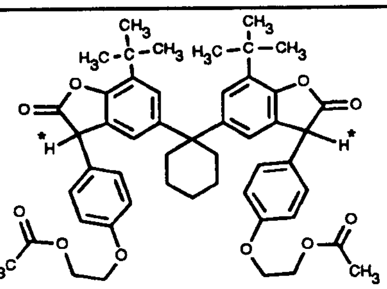 | Harz | Charakterisiert durch $^1$H-NMR ($CDCl_3$) δ ($H^*$) = 4,76 ppm Diastereomeren-Gemisch | 28 |
| 128 | 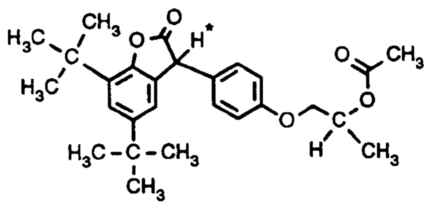 | Harz | Charakterisiert durch $^1$H-NMR ($CDCl_3$) δ ($H^*$) = 4,78 ppm Diastereomeren-Gemisch | 70 |

Tabelle 1: (Fortsetzung)

| Nr. | Verbindung | Smp. (°C) | C (%), H (%), N (%) (berechnet/gefunden) | Ausbeute (%) |
|---|---|---|---|---|
| 129 | 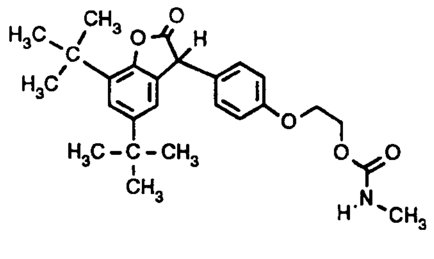 | 115-121 | 71,05 7,57 3,19<br>70,81 7,71 2,77 | 52 |
| 130 | 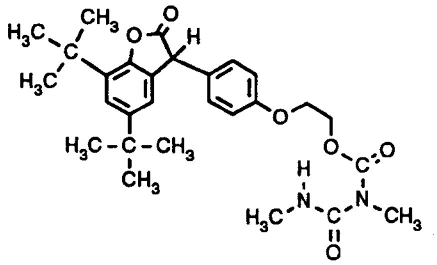 | 142-144 | 67,58 7,49 5,63<br>67,54 7,31 5,60 | 59 |
| 131 | 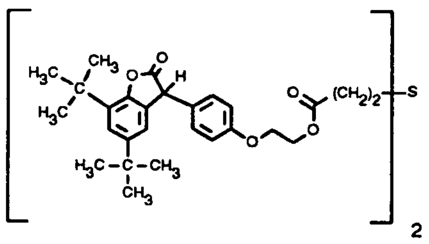 | Harz | C (%), H (%), S (%) (berechnet/gefunden)<br>71,50 7,33 3,53<br>71,28 7,35 3,48<br>Diastereomeren-Gemisch | 88 |
| 132 | 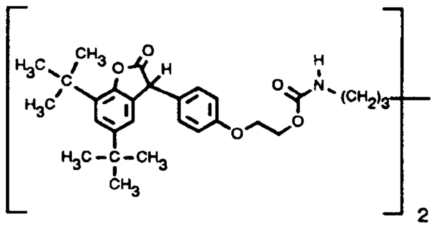 | 67-82 | 72,08 7,78<br>72,11 7,80<br>Diastereomeren-Gemisch | 66 |

Tabelle 1: (Fortsetzung)

| Nr. | Verbindung | Smp. (°C) | C (%), H (%) (berechnet/gefunden) | Ausbeute (%) |
|---|---|---|---|---|
| 133 | 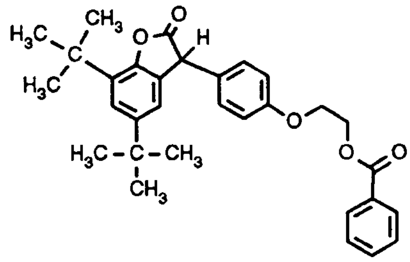 | 127-132 | 76,52 7,04<br>76,36 7,03 | 56 |
| 134 | 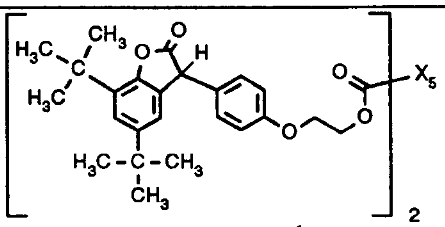<br>$X_5$ = 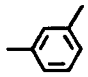 | 94-97 | 75,14 6,98<br>74,95 7,07<br>Diastereomeren-Gemisch | 91 |
| 135 | 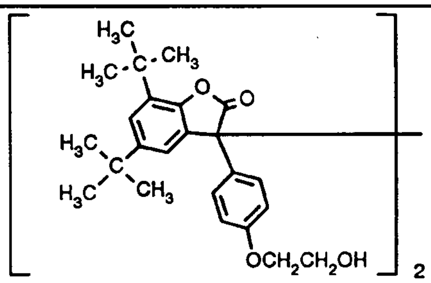 | 212-218 | 75,56 7,66<br>75,39 7,71<br>Diastereomeren-Gemisch | 90 |
| 136 | 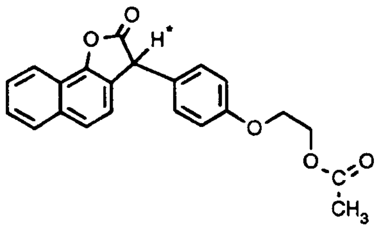 | Harz | Charakterisiert durch $^1$H-NMR ($CDCl_3$)<br>δ ($H^*$) = 5,02 ppm | 69 |

Tabelle 1: (Fortsetzung)

| Nr. | Verbindung | Smp. (°C) | C (%), H (%) (berechnet/gefunden) | Ausbeute (%) |
|---|---|---|---|---|
| 137 | 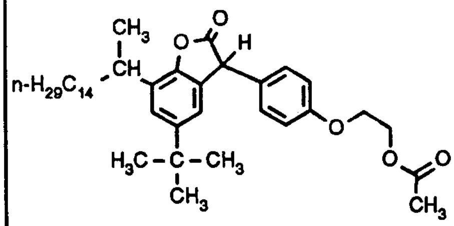 | 68-71 | 76,85 9,67<br>76,64 9,70<br>Diastereomeren-Gemisch | 81 |
| 138 | 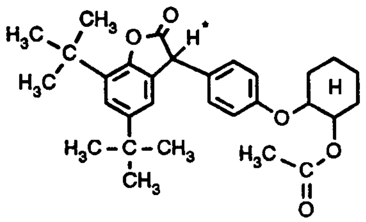 | Harz | Charakterisiert durch $^{1}$H-NMR ($CDCl_3$)<br>δ ($H^*$) = 4,77 ppm<br>Diastereomeren-Gemisch | 58 |
| 139 | 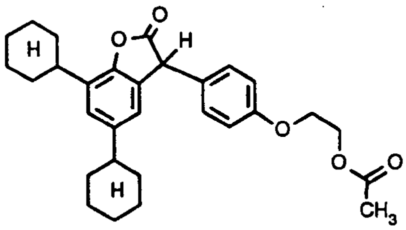 | 88-96 | 75,60 7,61<br>75,53 7,66 | 49 |
| 140 | 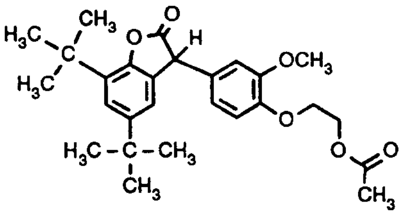 | 94-97 | 71,34 7,54<br>71,26 7,61 | 34 |

Tabelle 1: (Fortsetzung)

| Nr. | Verbindung | Smp. (°C) | C (%), H (%), S (%) (berechnet/gefunden) | Ausbeute (%) |
|---|---|---|---|---|
| 141 | 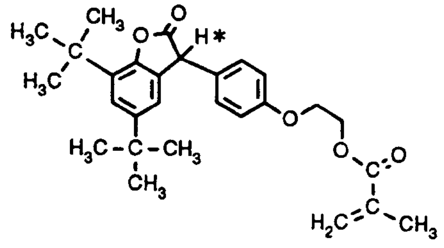 | Harz | Charakterisiert durch $^1$H-NMR ($CDCl_3$) δ (H*) = 4,78 ppm | 86 |
| 142 | 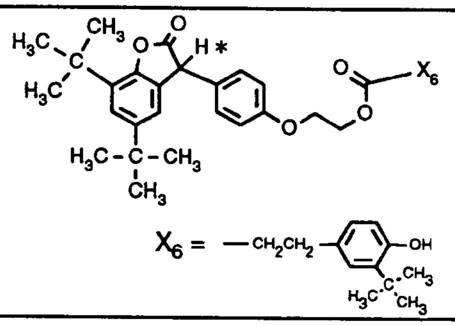 | Harz | Charakterisiert durch $^1$H-NMR ($CDCl_3$) δ (H*) = 4,80 ppm | 89 |
| 143 | 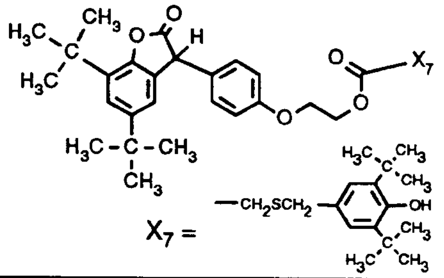 | Harz | 72,96 8,06 4,75<br>72,83 8,13 4,75 | 63 |
| 144 | 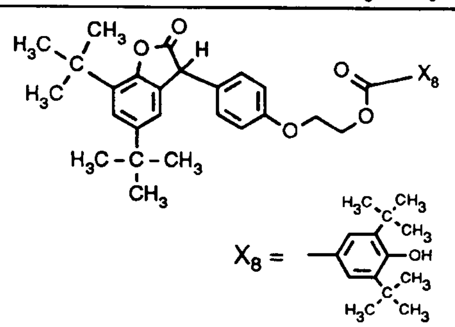 | 114-115 | 76,19 8,20<br>76,13 8,32 | 50 |

<u>Beispiel 10:</u> Herstellung von 4-(2-Hydroxyethoxy)mandelsäure (Verbindung (201), Tabelle 2).

In einen 6,3 Liter fassenden Autoklaven werden 1040,8 g (5,00 Mol) 4-Hydroxymandelsäure Natriumsalz Monohydrat, 10,0 g (0,25 Mol) Natriumhydroxid und 1000 ml Wasser gegeben. Der Autoklav wird mit Stickstoff gespült und danach werden 330,4 g (7,50 Mol) Ethylenoxid zugepresst. Der Inhalt wird unter Rühren während 2 Stunden langsam auf 95°C aufgeheizt und bei dieser Temperatur noch 2 Stunden nachgerührt. Das noch warme Reaktionsgemisch wird umgegossen, bei 95°C mit 540 ml (ca. 5,5 Mol) 32 %iger Salzsäure angesäuert und durch langsames Abkühlen auf ca. + 10°C auskristallisiert. Das ausgefallene Produkt wird filtriert, mit 1000 ml kaltem Wasser gewaschen und

getrocknet. Es resultieren 948 g (89 %) 4-(2-Hydroxyethoxy)mandelsäure, Smp. 162-164°C (Verbindung (201), Tabelle 2).

In Analogie zu Beispiel 10 wird ausgehend von 3,5-Dimethyl-4-hydroxy-mandelsäure Natriumsalz (Beispiel 13) die Verbindung (202) (Tabelle 2) hergestellt. Wird anstelle von Ethylenoxid Propylenoxid oder Cyclohexenoxid verwendet, werden die Verbindungen (206) und (207) (Tabelle 2) erhalten.

Beispiel 11: Herstellung von 4-(2-Hydroxyethoxy)-3-methyl-mandelsäure (Verbindung (203), Tabelle 2).

Zu einer auf 70°C erwärmten Lösung von 18,2 g (100 mMol) 4-Hydroxy-3-methyl-mandelsäure (Beispiel 13), 4,0 g (100 mMol) Natriumhydroxid und 13,4 ml (200 mMol) 2-Chlorethanol in 60 ml Wasser wird während einer Stunde eine Lösung von 8,0 g (200 mMol) Natriumhydroxid in 15 ml Wasser zugetropft. Anschliessend werden nochmals 6,7 ml (100 mMol) 2-Chlorethanol und 4,0 g (100 mMol) Natriumhydroxid in 10 ml Wasser zugegeben. Nach weiteren 15 Minuten wird das Reaktionsgemisch mit konzentrierter Salzsäure angesäuert und zweimal mit Ethylacetat extrahiert. Die organischen Phasen werden mit Wasser gewaschen, vereinigt, über Magnesiumsulfat getrocknet und am Vakuumrotationsverdampfer eingeengt. Es werden 23,0 g (~100 %) 4-(2-Hydroxyethoxy)-3-methyl-mandelsäure als gelbliches Harz erhalten (Verbindung (203), Tabelle 2).

In Analogie zu Beispiel 11 wird ausgehend von 4-Hydroxy-3-methoxy-mandelsäure (Beilstein, 10 IV, 2034) die Verbindung (208) erhalten (Tabelle 2).

Beispiel 12: Herstellung von 4-(2-Hydroxy-3-phenoxypropoxy)-mandelsäure (Verbindung (204), Tabelle 2).

Zu einer Suspension von 10,4 g (50 mMol) 4-Hydroxymandelsäure Natriumsalz Monohydrat und 300 mg (5,0 mMol) Kaliumhydroxid in 25 ml Methanol wird 7,5 g (50 mMol) 2,3-Epoxypropyl-phenylether (Phenylglycidether) gegeben und während 8 Stunden unter Rückfluss gekocht. Anschliessend wird das homogene Reaktionsgemisch mit 300 ml Wasser verdünnt, mit 25 ml konzentrierter Salzsäure angesäuert und dreimal mit Dichlormethan extrahiert. Die organischen Phasen werden mit Wasser gewaschen, vereinigt, über Magnesiumsulfat getrocknet und am Vakuumrotationsverdampfer eingeengt. Es resultieren 8,4 g (53 %) 4-(2-Hydroxy-3-phenoxypropoxy)-mandelsäure als gelbliches Harz (Verbindung (204), Tabelle 2).

In Analogie zu Beispiel 12 wird ausgehend von 1,2-Butylenoxid anstelle von 2,3-Epoxypropyl-phenylether die Verbindung (205) (Tabelle 2) erhalten.

Beispiel 13: Herstellung substituierter 4-Hydroxy-mandelsäuren:

0,30 Mol Ausgangsphenol (beispielsweise 2,6-Dimethyl-phenol, o-Kresol, 2-tert-Butylphenol oder 2-Isopropyl-3-methyl-phenol) wird in 150 ml 2N Natriumhydroxid-Lösung unter Stickstoffatmosphäre gelöst. Nach Abkühlen auf +5°C werden 4,8 g (0,12 Mol) Natriumhydroxid und 13,3 ml (0,12 Mol) 50 %wässrige Glyoxylsäure zugegeben und das Reaktionsgemisch während 4 Stunden bei Raumtemperatur gerührt. Nach jeweils 4 Stunden werden zweimal weitere 0,12 Mol Natriumhydroxid und Glyoxylsäure zugegeben (total 0,36 Mol). Das Reaktionsgemisch wird anschliessend noch 12 Stunden gerührt, dann mit konzentrierter Salzsäure neutralisiert und mit zweimal 75 ml Petrolether gewaschen. Die wässrige Phase wird nun mit konzentrierter Salzsäure angesäuert und mit Ether mehrmals extrahiert. Die organischen Phasen werden vereinigt, über Magnesiumsulfat getrocknet und am Vakuumrotationsverdampfer eingeengt. Es werden so die folgenden Präparate erhalten: 3,5-Dimethyl-4-hydroxy-mandelsäure, Smp. 132-135°C (85 %); 4-Hydroxy-3-methyl-mandelsäure, Smp. 115-120°C, Ausbeute 55 %; 4-Hydroxy-3-tert-butyl-mandelsäure, Smp. 156-158°C, Ausbeute 26 %; und 3-Isopropyl-4-hydroxy-2-methyl-mandelsäure, Smp. 114-119°C, Ausbeute 20 %.

Tabelle 2:

| Nr. | Verbindung | Smp. (°C) | C (%), H (%) (berechnet/gefunden) | Ausbeute (%) |
|---|---|---|---|---|
| 201 | 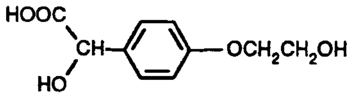 | 162-164 | 56,60 5,70<br>56,55 5,73 | 89 |
| 202 | 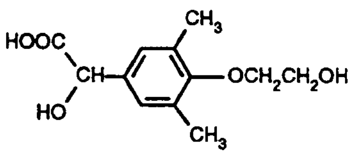 | 145-148 | 59,99 6,71<br>60,21 6,75 | 57 |
| 203 | 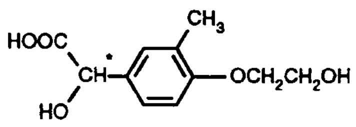 | Harz | Charakterisiert durch $^1$H-NMR (DMSO-$d_6$) $\delta(H^*) = 4,91$ ppm | ~100 |
| 204 | 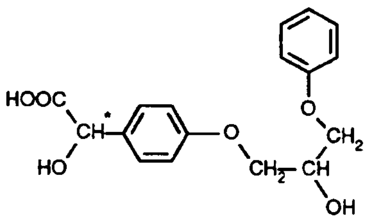 | Harz | Charakterisiert durch $^1$H-NMR (DMSO-$d_6$) $\delta(H^*) = 4,95$ ppm Diastereomeren-Gemisch | 53 |

Tabelle 2: (Fortsetzung)

| Nr. | Verbindung | Smp. (°C) | C (%), H (%) (berechnet/gefunden) | Ausbeute (%) |
|---|---|---|---|---|
| 205 | 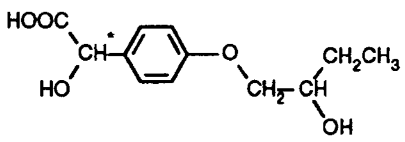 | 150-205 | Charakterisiert durch $^1$H-NMR (DMSO-$d_6$) δ(H*) = 4,83 ppm Diastereomeren-Gemisch | 40 |
| 206 | 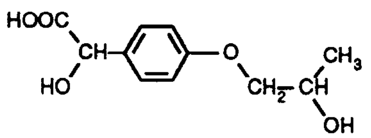 | 138-145 | 58,40 6,24<br>58,27 6,21<br>Diastereomeren-Gemisch | 71 |
| 207 | 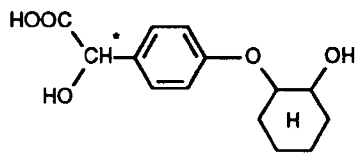 | 150-160 | Charakterisiert durch $^1$H-NMR (DMSO-$d_6$) δ(H*) = 4,93 ppm Diastereomeren-Gemisch | 69 |
| 208 | 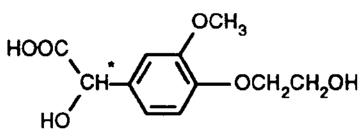 | Harz | Charakterisiert durch $^1$H-NMR (DMSO-$d_6$) δ(H*) = 4,94 ppm | 30 |

Beispiel 14: Stabilisierung von Polypropylen bei Mehrfachextrusion.

1,3 kg Polypropylenpulver (Profax 6501), das mit 0,025 % Irganox® 1076 (3-[3,5-di-tert-butyl-4-hydroxyphenyl]propionsäure-n-octadecylester) vorstabilisiert wurde, (mit einem bei 230°C und mit 2,16 kg gemessenen Schmelzindex von 3,2) werden gemischt mit 0,05 % Irganox® 1010 (Pentaerythrit-tetrakis-[3-(3,5-di-tert-butyl-4-hydroxyphenyl)-propionat), 0,05 % Calciumstearat, 0,03 % DHT 4A® (Kyowa Chemical Industry Co., Ltd., [$Mg_{4.5}Al_2(OH)_{13}CO_3 \cdot 3,5\ H_2O$]) und 0,015 % Verbindung aus Tabelle 1. Diese Mischung wird in einem Extruder mit

einem Zylinderdurchmesser von 20 mm und einer Länge von 400 mm mit 100 Umdrehungen pro Minute extrudiert, wobei die 3 Heizzonen auf die folgenden Temperaturen eingestellt werden: 260, 270, 280°C. Das Extrudat wird zur Kühlung durch ein Wasserbad gezogen und anschliessend granuliert Dieses Granulat wird wiederholt extrudiert. Nach 3 Extrusionen wird der Schmelzindex gemessen (bei 230°C mit 2,16 kg). Grosse Zunahme des Schmelzindex bedeutet starken Kettenabbau, also schlechte Stabilisierung. Die Resultate sind in Tabelle 3 zusammengefasst

Tabelle 3

| Verbindung aus Tabelle 1 | Schmelzindex nach 3 Extrusionen | | Verbindung aus Tabelle 1 | Schmelzindex nach 3 Extrusionen |
|---|---|---|---|---|
| - | 17,1 | | - | 17,1 |
| 101 | 6,1 | | 121 | 5,6 |
| 103 | 5,6 | | 122 | 5,8 |
| 104 | 5,7 | | 123 | 5,6 |
| 105 | 5,4 | | 124 | 5,8 |
| 106 | 6,0 | | 126 | 6,0 |
| 109 | 5,4 | | 128 | 5,7 |
| 111 | 5,7 | | 133 | 6,0 |
| 114 | 5,5 | | 134 | 5,9 |
| 116 | 5,7 | | 139 | 5,7 |
| 117 | 5,6 | | 141 | 5,9 |
| 118 | 5,3 | | | |

Beispiel 15: Stabilisierung von Polyethylen während der Verarbeitung.

100 Teile Polyethylenpulver (Lupolen® 5260 Z) werden mit 0,05 Teilen Pentaerythrittetrakis-[3-(3,5-di-tert-butyl-4-hydroxyphenyl)-propionat], 0,05 Teilen Tris-(2,4-di-tert-butyl-phenyl)-phosphit und 0,05 Teilen Verbindung aus Tabelle 1 gemischt und in einem Brabender Plastographen bei 220°C und 50 Umdrehungen pro Minute geknetet. Während dieser Zeit wird der Knetwiderstand als Drehmoment kontinuierlich registriert. Im Verlauf der Knetzeit beginnt das Polymere nach längerer Konstanz zu vernetzen, was anhand der raschen Zunahme des Drehmoments festgestellt werden kann. In der Tabelle 4 ist die Zeit bis zur merklichen Zunahme des Drehmoments als Mass der Stabilisatorwirkung angegeben. Je länger diese Zeit ist, desto besser ist die Stabilisatorwirkung.

Tabelle 4

| Verbindung aus Tabelle 1 | Zeit bis zum Anstieg von Drehmoment (Min) | | Verbindung aus Tabelle 1 | Zeit bis zum Anstieg von Drehmoment (Min) |
|---|---|---|---|---|
| - | 9,5 | | - | 9,5 |
| 101 | 25,0 | | 123 | 29,0 |
| 103 | 24,5 | | 124 | 27,5 |
| 105 | 28,0 | | 125 | 26,5 |
| 107 | 28,5 | | 126 | 28,5 |
| 109 | 26,0 | | 128 | 25,5 |
| 111 | 30,0 | | 130 | 26,0 |
| 113 | 29,0 | | 132 | 25,0 |
| 114 | 28,0 | | 133 | 28,0 |
| 115 | 35,5 | | 134 | 29,0 |

Tabelle 4 (fortgesetzt)

| Verbindung aus Tabelle 1 | Zeit bis zum Anstieg von Drehmoment (Min) | | Verbindung aus Tabelle 1 | Zeit bis zum Anstieg von Drehmoment (Min) |
|---|---|---|---|---|
| 116 | 27,0 | | 135 | 27,5 |
| 121 | 29,0 | | 137 | 27,0 |
| 122 | 29,5 | | 141 | 27,0 |

## Patentansprüche

**1.** Verbindungen der Formel (1)

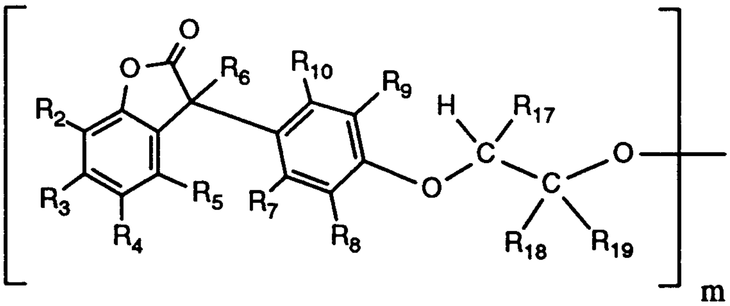

(1)

worin, wenn m 1 ist,

$R_1$ Wasserstoff, $C_1$-$C_{25}$-Alkanoyl, $C_3$-$C_{25}$-Alkenoyl, durch Sauerstoff, Schwefel oder >N-$R_{16}$ unterbrochenes $C_3$-$C_{25}$-Alkanoyl; durch eine Di($C_1$-$C_6$-alkyl)phosphonatgruppe substituiertes $C_2$-$C_{25}$-Alkanoyl; $C_6$-$C_9$-Cycloalkylcarbonyl, Thenoyl, Furoyl, Benzoyl oder durch $C_1$-$C_{12}$-Alkyl substituiertes Benzoyl;

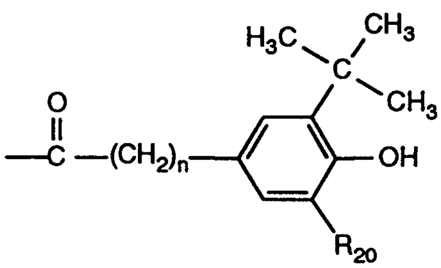

,

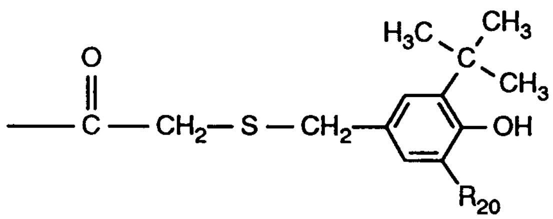 , 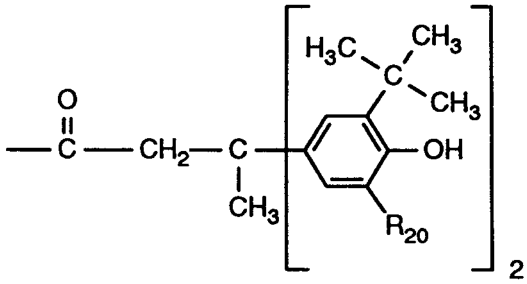 ,

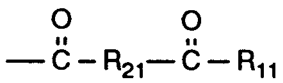 oder 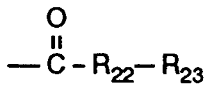

bedeutet, und wenn m 2 ist,
$R_1$

$R_1$ 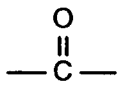 , 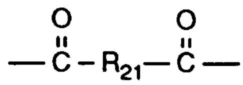 oder 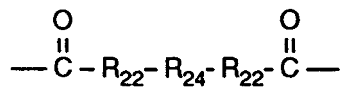

darstellt, und wenn m 3 bedeutet,
$R_1$ $C_4$-$C_{18}$-Alkantricarbonyl, $C_9$-$C_{18}$-Aryltricarbonyl,

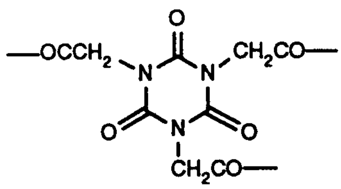

oder

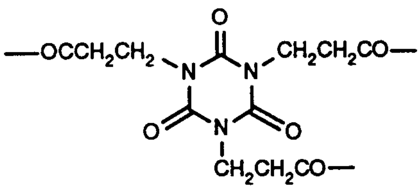

darstellt, und wenn m 4 ist,
$R_1$ $C_6$-$C_{18}$-Alkantetracarbonyl oder $C_{10}$-$C_{18}$-Aryltetracarbonyl bedeutet,
$R_2$, $R_3$, $R_4$ und $R_5$ unabhängig voneinander Wasserstoff, Chlor, $C_1$-$C_{25}$-Alkyl, $C_7$-$C_9$-Phenylalkyl, unsubstituiertes oder durch $C_1$-$C_4$-Alkyl substituiertes Phenyl, unsubstituiertes oder durch $C_1$-$C_4$-Alkyl substituiertes $C_5$-$C_8$-Cycloalkyl; $C_1$-$C_{18}$-Alkoxy, $C_1$-$C_{18}$-Alkylthio, Hydroxy, $C_1$-$C_4$-Alkylamino, Di-($C_1$-$C_4$-alkyl)amino, $C_1$-$C_{25}$-Alkanoyloxy, $C_1$-$C_{25}$-Alkanoylamino, $C_3$-$C_{25}$-Alkenoyloxy, durch Sauerstoff, Schwefel oder >N-$R_{16}$ unterbrochenes $C_3$-$C_{25}$-Alkanoyloxy; $C_6$-$C_9$-Cycloalkylcarbonyloxy, Benzoyloxy oder durch $C_1$-$C_{12}$-Alkyl substituiertes Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Phenylring bilden, $R_4$ zusätzlich -$(CH_2)_n$-$COR_{11}$ darstellt, oder wenn $R_3$, $R_5$, $R_6$, $R_7$ und $R_{10}$ Wasserstoff sind, $R_4$ zusätzlich einen Rest der Formel (2)

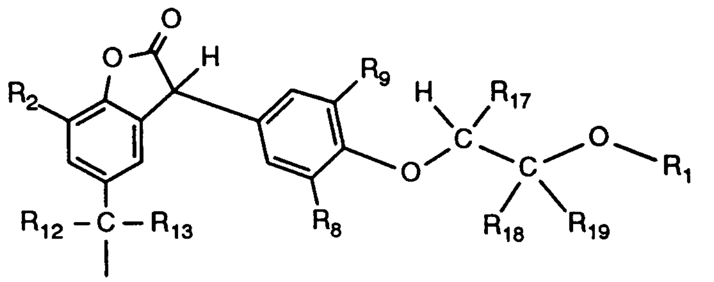

(2)

bedeutet, worin $R_1$ wie oben für m = 1 angegeben definiert ist,
$R_6$ Wasserstoff oder einen Rest der Formel (3)

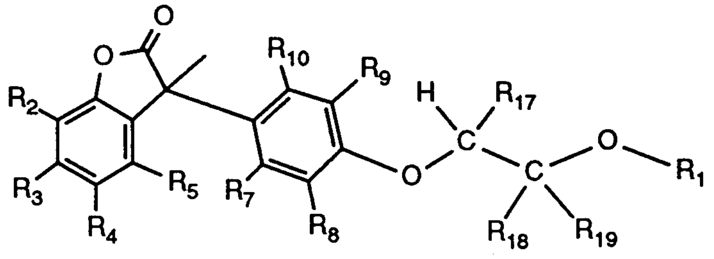

(3)

darstellt, wobei $R_4$ nicht einen Rest der Formel (2) bedeutet und $R_1$ wie oben für m = 1 angegeben definiert ist,
$R_7$, $R_8$, $R_9$ und $R_{10}$ unabhängig voneinander Wasserstoff, $C_1$-$C_4$-Alkyl oder $C_1$-$C_4$-Alkoxy darstellen, mit der Bedingung, dass mindestens einer der Reste $R_7$, $R_8$, $R_9$ und $R_{10}$ Wasserstoff ist,
$R_{11}$ Hydroxy,

$$\left[-O^{\ominus}\ \frac{1}{r} M^{r+}\right],$$

$C_1$-$C_{18}$-Alkoxy oder

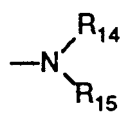

bedeutet,
$R_{12}$ und $R_{13}$ unabhängig voneinander Wasserstoff, $CF_3$, $C_1$-$C_{12}$-Alkyl oder Phenyl darstellen, oder $R_{12}$ und $R_{13}$ zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden;
$R_{14}$ und $R_{15}$ unabhängig voneinander Wasserstoff oder $C_1$-$C_{18}$-Alkyl darstellen,
$R_{16}$ Wasserstoff oder $C_1$-$C_8$-Alkyl bedeutet,
$R_{17}$ Wasserstoff oder $C_1$-$C_4$-Alkyl darstellt,
$R_{18}$ Wasserstoff, unsubstituiertes oder durch $C_1$-$C_4$-Alkyl substituiertes Phenyl; $C_1$-$C_{25}$-Alkyl, durch Sauerstoff, Schwefel oder $>$N-$R_{16}$ unterbrochenes $C_2$-$C_{25}$-Alkyl; unsubstituiertes oder am Phenylrest durch 1 bis 3 $C_1$-$C_4$-Alkyl substituiertes $C_7$-$C_9$-Phenylalkyl; durch Sauerstoff, Schwefel oder $>$N-$R_{16}$ unterbrochenes unsubstituiertes oder am Phenylrest durch 1 bis 3 $C_1$-$C_4$-Alkyl substituiertes $C_7$-$C_{25}$-Phenylalkyl bedeutet, oder ferner die Reste $R_{17}$ und $R_{18}$ zusammen mit den Kohlenstoffatomen, an die sie gebunden sind, einen unsubstituierten oder durch 1 bis 3 $C_1$-$C_4$-Alkyl substituierten $C_5$-$C_{12}$-Cycloalkylenring bilden; oder wenn $R_6$, $R_{17}$ und $R_{19}$ Wasserstoff sind, $R_4$ nicht den Rest der Formel (2) darstellt, m 1 und $R_1$ wie oben für m = 1 ange-

geben definiert ist, $R_{18}$ zusätzlich einen Rest der Formel (4)

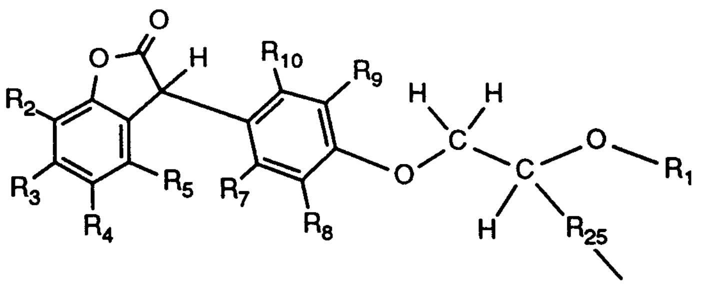

(4)

bedeutet,

$R_{19}$ Wasserstoff oder $C_1$-$C_4$-Alkyl darstellt,

$R_{20}$ Wasserstoff oder $C_1$-$C_4$-Alkyl bedeutet,

$R_{21}$ eine direkte Bindung, $C_1$-$C_{18}$-Alkylen, durch Sauerstoff, Schwefel oder >N-$R_{16}$ unterbrochenes $C_2$-$C_{18}$-Alylen; $C_2$-$C_{18}$-Alkenylen, $C_2$-$C_{20}$-Alkyliden, $C_7$-$C_{20}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

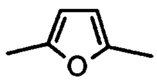

oder

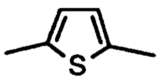

darstellt,

$R_{22}$ Sauerstoff, -NH- oder

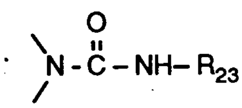

bedeutet,

$R_{23}$ $C_1$-$C_{18}$-Alkyl oder Phenyl ist,

$R_{24}$ $C_2$-$C_{18}$-Alkylen, $C_5$-$C_8$-Cycloalkylen oder Phenylen darstellt,

$R_{25}$ eine direkte Bindung, $C_1$-$C_{18}$-Alkylen oder durch Sauerstoff, Schwefel oder >N-$R_{16}$ unterbrochenes $C_2$-$C_{18}$-Alkylen bedeutet,

M ein r-wertiges Metallkation ist,

m 1, 2, 3 oder 4 darstellt, wobei, wenn m 2,3 oder 4 ist, $R_6$ Wasserstoff bedeutet;

n 0, 1 oder 2 und

r 1,2 oder 3 darstellt.

**2.** Verbindungen gemäss Anspruch 1, worin, wenn m 1 ist,

$R_1$ Wasserstoff, $C_1$-$C_{18}$-Alkanoyl, $C_3$-$C_{18}$-Alkenoyl, durch Sauerstoff, Schwefel oder >N-$R_{16}$ unterbrochenes $C_3$-$C_{18}$-Alkanoyl; durch eine Di($C_1$-$C_6$-alkyl)phosphonatgruppe substituiertes $C_2$-$C_{18}$-Alkanoyl; $C_6$-$C_9$-Cycloalkylcarbonyl, Thenoyl, Furoyl, Benzoyl oder durch $C_1$-$C_8$-Alkyl substituiertes Benzoyl;

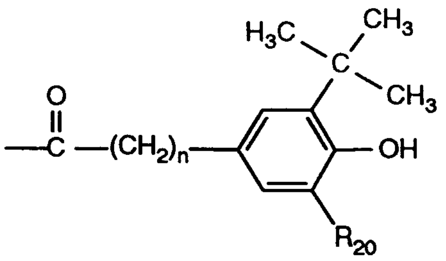

,

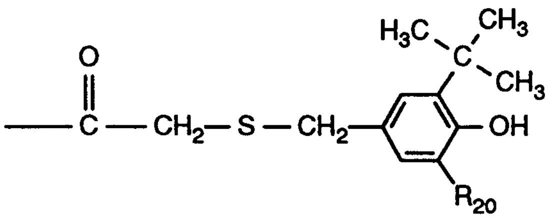

,

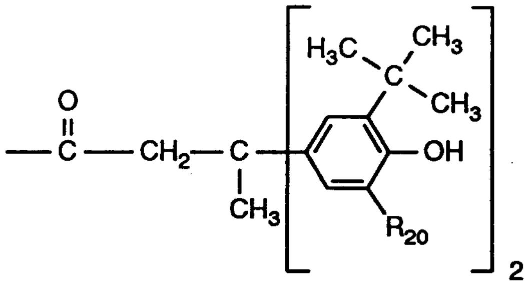

,

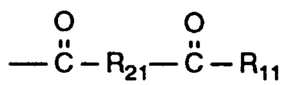 oder 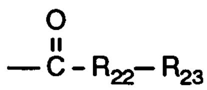

bedeutet,

$R_2$, $R_3$, $R_4$ und $R_5$ unabhängig voneinander Wasserstoff, Chlor, $C_1$-$C_{18}$-Alkyl, Benzyl, Phenyl, $C_5$-$C_8$-Cycloalkyl, $C_1$-$C_{18}$-Alkoxy, $C_1$-$C_{18}$-Alkylthio, $C_1$-$C_{18}$-Alkanoyloxy, $C_1$-$C_{18}$-Alkanoylamino, $C_3$-$C_{18}$-Alkenoyloxy oder Benzoyloxy darstellen, oder ferner die Reste $R_2$ und $R_3$ oder die Reste $R_4$ und $R_5$ zusammen mit den Kohlenstoffatomen, an die sie gebunden sind, einen Phenylring bilden, oder wenn $R_3$, $R_5$, $R_6$, $R_7$ und $R_{10}$ Wasserstoff sind, $R_4$ zusätzlich einen Rest der Formel (2) bedeutet,

$R_7$, $R_8$, $R_9$ und $R_{10}$ unabhängig voneinander Wasserstoff oder $C_1$-$C_4$-Alkyl darstellen, mit der Bedingung, dass mindestens einer der Reste $R_7$, $R_8$, $R_9$ und $R_{10}$ Wasserstoff ist, $R_{12}$ und $R_{13}$ Methylgruppen sind oder zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden,

$R_{18}$ Wasserstoff, Phenyl, $C_1$-$C_{18}$-Alkyl, durch Sauerstoff, Schwefel oder $>$N-$R_{16}$ unterbrochenes $C_2$-$C_{18}$-Alkyl; Benzyl, durch Sauerstoff, Schwefel oder $>$N-$R_{16}$ unterbrochenes $C_7$-$C_{18}$-Phenylalkyl bedeutet, oder ferner die Reste $R_{17}$ und $R_{18}$ zusammen mit den Kohlenstoffatomen, an die sie gebunden sind einen unsubstituierten oder durch 1 bis 3 $C_1$-$C_4$-Alkyl substituierten $C_5$-$C_8$-Cycloalkylenring bilden, $R_{21}$ eine direkte Bindung, $C_1$-$C_{12}$-Alkylen, durch Sauerstoff, Schwefel oder $>$N-$R_{16}$ unterbrochenes $C_2$-$C_{12}$-Alkylen; $C_2$-$C_{12}$-Alkenylen, $C_2$-$C_{12}$-Alkyliden, $C_7$-$C_{12}$-Phenylalkyliden, $C_5$-$C_8$-Cycloalkylen, $C_7$-$C_8$-Bicycloalkylen oder Phenylen darstellt,

$R_{24}$ $C_2$-$C_{12}$-Alkylen, $C_5$-$C_8$-Cycloalkylen oder Phenylen bedeutet, und

$R_{25}$ eine direkte Bindung, $C_1$-$C_{12}$-Alkylen oder durch Sauerstoff, Schwefel oder $>$N-$R_{16}$ unterbrochenes $C_2$-$C_{12}$-Alkylen darstellt.

**3.** Verbindungen gemäss Anspruch 1, worin mindestens zwei der Reste $R_2$, $R_3$, $R_4$ und $R_5$ Wasserstoff sind.

**4.** Verbindungen gemäss Anspruch 1, worin $R_3$ und $R_5$ Wasserstoff sind.

**5.** Verbindungen gemäss Anspruch 1, worin $R_2$, $R_3$, $R_4$ und $R_5$ unabhängig voneinander Wasserstoff, Chlor, $C_1$-$C_{18}$-Alkyl, $C_5$-$C_6$-Cycloalkyl oder $C_1$-$C_4$-Alkoxy darstellen, oder ferner die Reste $R_2$ und $R_3$ zusammen mit den Kohlenstoffatomen, an die sie gebunden sind, einen Phenylring bilden.

**6.** Verbindungen gemäss Anspruch 1, worin m 1 oder 2 bedeutet.

**7.** Verbindungen gemäss Anspruch 1, worin $R_{18}$ Wasserstoff, $C_1$-$C_{12}$-Alkyl, durch Sauerstoff oder Schwefel unterbrochenes $C_2$-$C_{12}$-Alkyl; durch Sauerstoff oder Schwefel unterbrochenes $C_7$-$C_{12}$-Phenylalkyl bedeutet, oder ferner die Reste $R_{17}$ und $R_{18}$ zusammen mit den Kohlenstoffatomen, an die sie gebunden sind, einen $C_5$-$C_8$-Cycloalkylenring bilden.

**8.** Verbindungen gemäss Anspruch 1, worin,
wenn m 1 ist,

$R_1$ Wasserstoff, $C_1$-$C_{18}$-Alkanoyl, $C_3$-$C_{12}$-Alkenoyl, durch Sauerstoff unterbrochenes $C_3$-$C_{12}$-Alkanoyl; durch eine Di($C_1$-$C_6$-alkyl)phosphonatgruppe substituiertes $C_2$-$C_{12}$-Alkanoyl; $C_6$-$C_9$-Cycloalkylcarbonyl, Benzoyl,

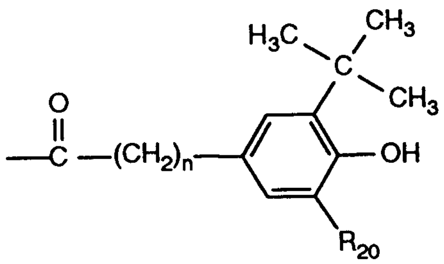 , 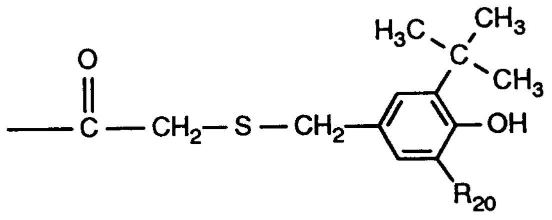 ,

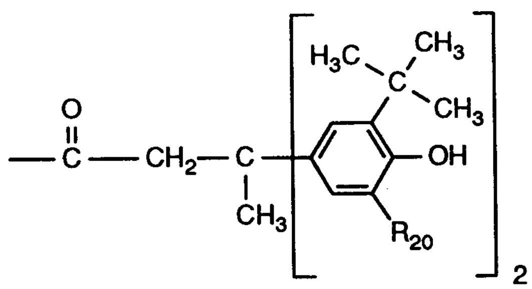 , 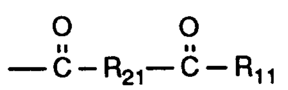 oder 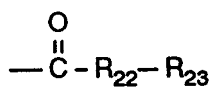

bedeutet,
$R_2$, $R_3$, $R_4$ und $R_5$ unabhängig voneinander Wasserstoff, $C_1$-$C_{18}$-Alkyl, $C_5$-$C_7$-Cycloalkyl, $C_1$-$C_{12}$-Alkoxy, $C_1$-$C_{12}$-Alkanoyloxy oder Benzoyloxy darstellen, oder ferner die Reste
$R_2$ und $R_3$ zusammen mit den Kohlenstoffatomen, an die sie gebunden sind, einen Phenylring bilden, oder wenn $R_3$, $R_5$, $R_6$, $R_7$ und $R_{10}$ Wasserstoff sind, $R_4$ zusätzlich einen Rest der Formel (2) bedeutet,
$R_{12}$ und $R_{13}$ Methylgruppen sind oder zusammen mit dem C-Atom, an das sie gebunden sind, einen $C_5$-$C_8$-Cycloalkylidenring bilden,
$R_{18}$ Wasserstoff, $C_1$-$C_{12}$-Alkyl, durch Sauerstoff oder Schwefel unterbrochenes $C_2$-$C_{12}$-Alkyl; durch Sauerstoff oder Schwefel unterbrochenes $C_7$-$C_{12}$-Phenylalkyl bedeutet, oder ferner die Reste $R_{17}$ und $R_{18}$ zusammen mit den Kohlenstoffatomen, an die sie gebunden sind, einen $C_5$-$C_8$-Cycloalkylenring bilden,
$R_{21}$ $C_1$-$C_{12}$-Alkylen, Phenylen oder durch Sauerstoff oder Schwefel unterbrochenes $C_2$-$C_{12}$-Alkylen darstellt,
$R_{23}$ $C_1$-$C_{12}$-Alkyl bedeutet,
$R_{24}$ $C_2$-$C_{12}$-Alkylen, oder Phenylen darstellt,
$R_{25}$ $C_1$-$C_8$-Alkylen oder durch Sauerstoff unterbrochenes $C_2$-$C_8$-Alkylen bedeutet, und
m 1, 2 oder 3 darstellt.

**9.** Verbindungen gemäss Anspruch 1, worin,
wenn m 1 ist,

$R_1$ Wasserstoff, $C_1$-$C_{18}$-Alkanoyl, $C_3$-$C_4$-Alkenoyl, durch eine Di($C_1$-$C_4$-alkyl)phosphonatgruppe substituiertes $C_2$-$C_4$-Alkanoyl; Cyclohexylcarbonyl, Benzoyl,

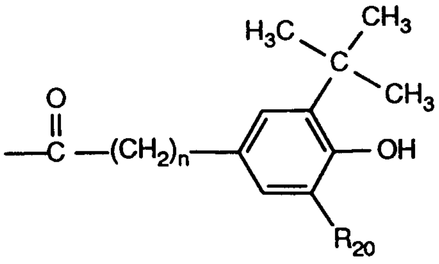
,
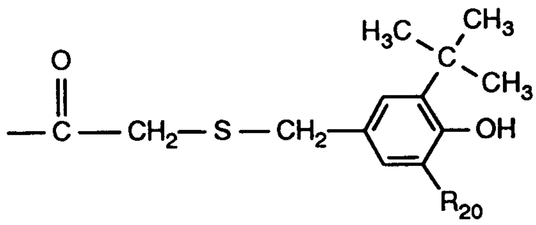

oder

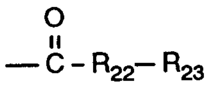

bedeutet, und
wenn m 2 ist, $R_1$

$R_1$ 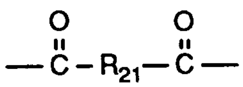 oder 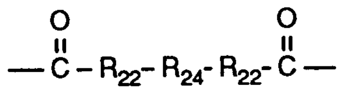

darstellt,

$R_2$ Wasserstoff, $C_1$-$C_{18}$-Alkyl oder Cyclohexyl bedeutet,

$R_3$ Wasserstoff ist, oder ferner die Reste $R_2$ und $R_3$ zusammen mit den Kohlenstoffatomen, an die sie gebunden sind, einen Phenylring bilden,

$R_4$ $C_1$-$C_4$-Alkyl oder Cyclohexyl darstellt, oder wenn $R_3$, $R_5$, $R_6$, $R_7$ und $R_{10}$ Wasserstoff sind, $R_4$ zusätzlich einen Rest der Formel (2) bedeutet, worin $R_1$ wie oben für m = 1 angegeben definiert ist,

$R_5$ Wasserstoff bedeutet,

$R_6$ Wasserstoff oder einen Rest der Formel (3) darstellt, wobei $R_4$ nicht einen Rest der Formel (2) bedeutet und $R_1$ wie oben für m = 1 angegeben definiert ist,

$R_7$ Wasserstoff bedeutet,

$R_8$ und $R_9$ unabhängig voneinander Wasserstoff, $C_1$-$C_4$-Alkyl oder $C_1$-$C_4$-Alkoxy darstellen,

$R_{10}$ Wasserstoff bedeutet,

$R_{12}$ und $R_{13}$ Methylgruppen sind oder zusammen mit dem C-Atom, an das sie gebunden sind, einen Cyclohexylidenring bilden,

$R_{17}$ Wasserstoff darstellt,

$R_{18}$ Wasserstoff, $C_1$-$C_4$-Alkyl, durch Sauerstoff unterbrochenes $C_2$-$C_8$-Alkyl; oder durch Sauerstoff unterbrochenes $C_7$-$C_9$-Phenylalkyl bedeutet, oder ferner die Reste $R_{17}$ und $R_{18}$ zusammen mit den Kohlenstoffatomen, an die sie gebunden sind, einen Cyclohexylenring bilden,

$R_{19}$ Wasserstoff darstellt,

$R_{20}$ Wasserstoff oder $C_1$-$C_4$-Alkyl ist,

$R_{21}$ $C_1$-$C_8$-Alkylen, durch Schwefel unterbrochenes $C_2$-$C_6$-Alkylen; oder Phenylen darstellt,

$R_{22}$ -NH- oder

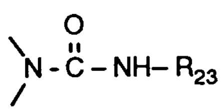

bedeutet,

$R_{23}$ $C_1$-$C_4$-Alkyl darstellt,

$R_{24}$ $C_4$-$C_8$-Alkylen bedeutet,
m 1 oder 2 darstellt, und
n 0 oder 2 bedeutet.

**10.** Zusammensetzung enthaltend

a) ein dem oxidativen, thermischen oder lichtinduzierten Abbau unterworfenes organisches Material und
b) mindestens eine Verbindung der Formel (1) gemäss Anspruch 1.

**11.** Zusammensetzung gemäss Anspruch 10, worin die Komponente a) ein synthetisches Polymer ist.

**12.** Zusammensetzung gemäss Anspruch 10, worin die Komponente b) in einer Menge von 0,0005 bis 5 % bezogen auf das Gewicht der Komponente a) vorliegt.

**13.** Zusammensetzungen gemäss Anspruch 10, enthaltend zusätzlich ein organisches Phosphit oder Phosphonit.

**14.** Verwendung der Verbindungen der in Anspruch 1 definierten Formel (1) als Stabilisatoren für organische Materialien gegen oxidativen, thermischen oder lichtinduzierten Abbau.

**15.** Verfahren zum Stabilisieren eines organischen Materials gegen oxidativen, thermischen oder lichtinduzierten Abbau, dadurch gekennzeichnet, dass man diesem mindestens eine Verbindung der in Anspruch 1 definierten Formel (1) einverleibt oder auf dieses aufbringt.

**16.** Verbindungen der Formel (9)

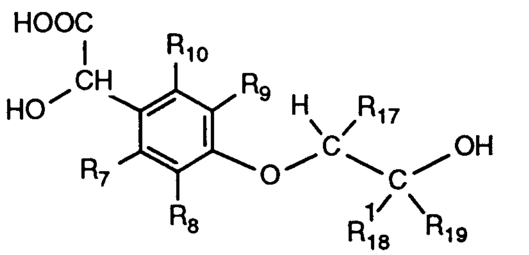

(9)

worin $R_7$, $R_8$, $R_9$ und $R_{10}$ unabhängig voneinander Wasserstoff, $C_1$-$C_4$ - Alkyl oder $C_1$-$C_4$-Alkoxy darstellen, mit der Bedingung, dass mindestens einer der Reste $R_7$, $R_8$, $R_9$ und $R_{10}$ Wasserstoff ist, und, wenn $R_7$, $R_8$, $R_9$ und $R_{10}$ gleichzeitig Wasserstoff sind, entweder $R_{17}$, $R^1_{18}$ oder $R_{19}$ von Wasserstoff verschieden ist,
$R_{16}$ Wasserstoff oder $C_1$-$C_8$-Alkyl bedeutet,
$R_{17}$ Wasserstoff oder $C_1$-$C_4$-Alkyl darstellt,
$R^1_{18}$ Wasserstoff, unsubstituiertes oder durch $C_1$-$C_4$-Alkyl substituiertes Phenyl; $C_1$-$C_{25}$-Alkyl, durch Sauerstoff oder Schwefel unterbrochenes $C_2$-$C_{25}$-Alkyl; unsubstituiertes oder am Phenylring durch 1 bis 3 $C_1$-$C_4$-Alkyl substituiertes $C_7$-$C_9$-Phenylalkyl; durch Sauerstoff, Schwefel oder $>N$-$R_{16}$ unterbrochenes unsubstituiertes oder am Phenylring durch 1 bis 3 $C_1$-$C_4$-Alkyl substituiertes $C_7$-$C_{25}$-Phenylalkyl bedeutet, oder ferner die Reste $R_{17}$ und $R^1_{18}$ zusammen mit den Kohlenstoffatomen, an die sie gebunden sind einen unsubstituierten oder durch 1 bis 3 $C_1$-$C_4$-Alkyl substituierten $C_5$-$C_{12}$-Cycloalkylenring bilden; oder wenn $R_{17}$ und $R_{19}$ Wasserstoff sind, $R^1_{18}$ zusätzlich einen Rest der Formel (10)

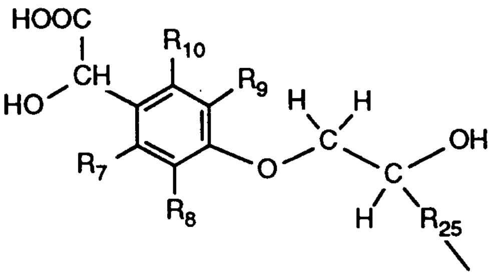

(10)

bedeutet,

$R_{19}$ Wasserstoff oder $C_1$-$C_4$-Alkyl darstellt, und

$R_{25}$ eine direkte Bindung, $C_1$-$C_{18}$-Alkylen oder durch Sauerstoff, Schwefel oder $>$N-$R_{16}$ unterbrochenes $C_2$-$C_{18}$-Alkylen bedeutet.

**17.** Verbindungen gemäss Anspruch 16, worin

$R_{18}^1$ Wasserstoff, Phenyl, $C_1$-$C_{18}$-Alkyl, durch Sauerstoff oder Schwefel unterbrochenes $C_2$-$C_{18}$-Alkyl; Benzyl, durch Sauerstoff, Schwefel oder $>$N-$R_{16}$ unterbrochenes $C_7$-$C_{18}$-Phenylalkyl bedeutet, oder ferner die Reste $R_{17}$ und $R_{18}^1$ zusammen mit den Kohlenstoffatomen, an die sie gebunden sind einen unsubstituierten oder durch 1 bis 3 $C_1$-$C_4$-Alkyl substituierten $C_5$-$C_8$-Cycloalkylenring bilden, und

$R_{25}$ eine direkte Bindung, $C_1$-$C_{12}$-Alkylen oder durch Sauerstoff, Schwefel oder $>$N-$R_{16}$ unterbrochenes $C_2$-$C_{12}$-Alkylen bedeutet.

**18.** Verbindung gemäss Anspruch 16, worin $R_7$ und $R_{10}$ Wasserstoff sind.

**19.** Verbindung gemäss Anspruch 16, worin

$R_{17}$ Wasserstoff darstellt,

$R_{18}^1$ Wasserstoff, $C_1$-$C_{12}$-Alkyl, durch Sauerstoff oder Schwefel unterbrochenes $C_2$-$C_{12}$-Alkyl; durch Sauerstoff oder Schwefel unterbrochenes $C_7$-$C_{12}$-Phenylalkyl bedeutet, oder ferner die Reste $R_{17}$ und $R_{18}^1$ zusammen mit den Kohlenstoffatomen, an die sie gebunden sind, einen $C_5$-$C_8$-Cycloalkylenring bilden, und

$R_{25}$ $C_1$-$C_8$-Alkylen oder durch Sauerstoff unterbrochenes $C_2$-$C_8$-Alkylen bedeutet.

**20.** Verbindung gemäss Anspruch 16, worin

$R_7$, $R_{10}$, $R_{17}$ und $R_{19}$ Wasserstoff darstellen, und

$R_{18}^1$ Wasserstoff, $C_1$-$C_4$-Alkyl, durch Sauerstoff unterbrochenes $C_2$-$C_8$-Alkyl; durch Sauerstoff unterbrochenes $C_7$-$C_9$-Phenylalkyl bedeutet, oder ferner die Reste $R_{17}$ und

$R_{18}^1$ zusammen mit den Kohlenstoffatomen, an die sie gebunden sind, einen Cyclohexylenring bilden.

**21.** Verfahren zur Herstellung von Verbindungen der Formel (6),

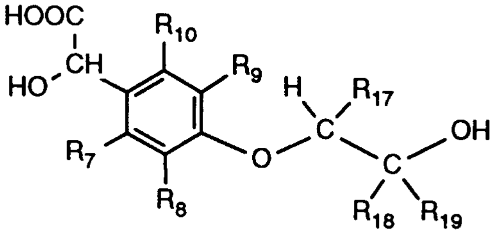

(6)

worin die allgemeinen Symbole wie in Anspruch 1 definiert sind und, wenn $R_{17}$ und $R_{19}$ Wasserstoff sind, $R_{18}$

zusätzlich einen Rest der Formel (10)

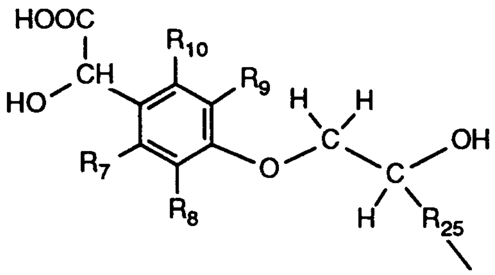
(10)

bedeutet, dadurch gekennzeichnet, dass eine 4-Hydroxymandelsäure der Formel (11) mit einem Epoxid der Formel (12),

(11)
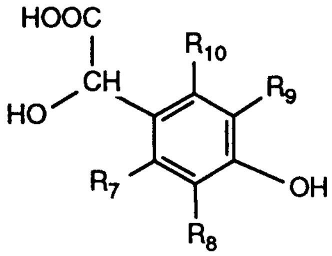

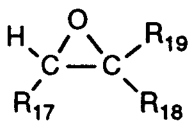
(12)

worin die Reste $R_7$, $R_8$, $R_9$, $R_{10}$, $R_{17}$, $R_{18}$ und $R_{19}$ die gleiche Bedeutung haben wie für Formel (1) in Anspruch 1, und wenn $R_{17}$ und $R_{19}$ Wasserstoff sind, $R_{18}$ in Formel (12) zusätzlich einen Rest der Formel (10) oder einen Rest der Formel (16)

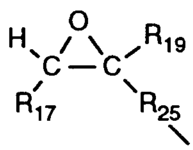
(16)

bedeutet, zu Verbindungen der Formel (6) umgesetzt wird.

**22.** Verfahren gemäss Anspruch 21, worin $R_{18}$ die gleiche Bedeutung hat wie für Formel (1) in Anspruch 1.

**23.** Verfahren gemäss Anspruch 21, worin das Epoxid Ethylenoxid ist.

**24.** Verfahren gemäss Anspruch 21, worin das Epoxid in einem molaren Ueberschuss von 1 bis 80 %, insbesondere 10 bis 60 %, bezüglich der eingesetzten 4-Hydroxymandelsäure der Formel (11) verwendet wird.

## Claims

**1.** A compound of the formula (1)

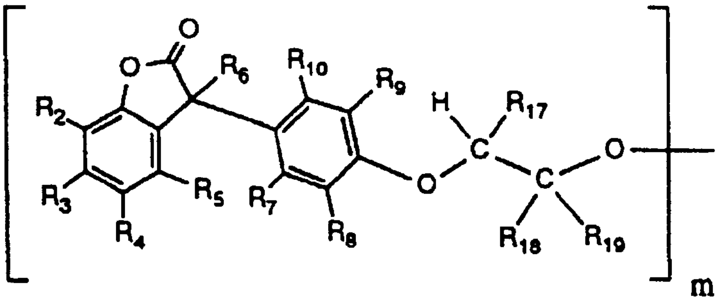 (1)

in which, if m is 1,

$R_1$ is hydrogen, $C_1$-$C_{25}$alkanoyl, $C_3$-$C_{25}$alkenoyl, $C_3$-$C_{25}$alkanoyl which is interrupted by oxygen, sulfur or $>$N-$R_6$ ; $C_2$-$C_{25}$alkanoyl substituted by a di($C_1$-$C_6$alkyl)phosphonate group; $C_6$-$C_9$cycloalkylcarbonyl, thenoyl, furoyl, benzoyl or $C_1$-$C_{12}$alkyl-substituted benzoyl;

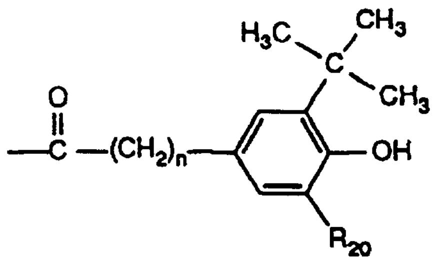 ,

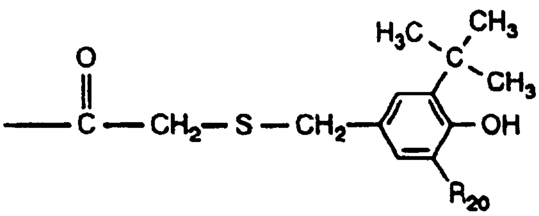 , 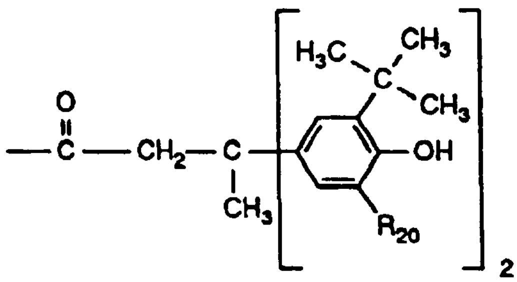 ,

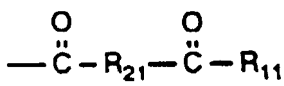

or

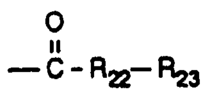

and, if m is 2,
$R_1$ is

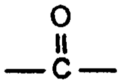 , 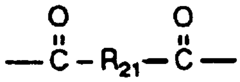

or

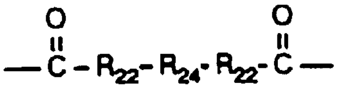

and, if m is 3,
$R_1$ is $C_4$-$C_{18}$alkanetricarbonyl, $C_9$-$C_{18}$aryltricarbonyl,

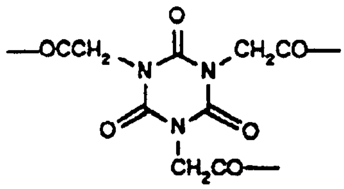

or

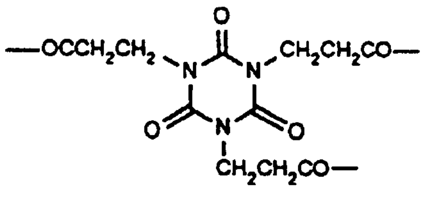

,

and, if m is 4,
$R_1$ is $C_6$-$C_{18}$alkanetetracarbonyl or $C_{10}$-$C_{18}$aryltetracarbonyl,
$R_2$, $R_3$, $R_4$ and $R_5$, independently of one another, are hydrogen, chlorine, $C_1$-$C_{25}$alkyl, $C_7$-$C_9$phenylalkyl, unsubstituted or $C_1$-$C_4$alkyl-substituted phenyl, unsubstituted or $C_1$-$C_4$alkyl-substituted $C_5$-$C_8$-cycloalkyl; $C_1$-$C_{18}$alkoxy, $C_1$-$C_{18}$alkylthio, hydroxyl, $C_1$-$C_4$alkylamino, di($C_1$-$C_4$alkyl)amino, $C_1$-$C_{25}$alkanoyloxy, $C_1$-$C_{25}$alkanoylamino, $C_3$-$C_{25}$alkenoyloxy, $C_3$-$C_{25}$alkanoyloxy which is interrupted by oxygen, sulfur or $>N-R_{16}$ ; $C_6$-$C_9$cycloalkylcarbonyloxy, benzoyloxy or $C_1$-$C_{12}$alkyl-substituted benzoyloxy, or, furthermore, the radicals $R_2$ and $R_3$ or the radicals $R_3$ and $R_4$ or the radicals $R_4$ and $R_5$ together with the carbon atoms to which they are attached form a phenyl ring, $R_4$ is additionally $-(CH_2)_n-COR_{11}$, or, if $R_3$, $R_5$, $R_6$, $R_7$ and $R_{10}$ are hydrogen, $R_4$ is additionally a radical of the formula (2)

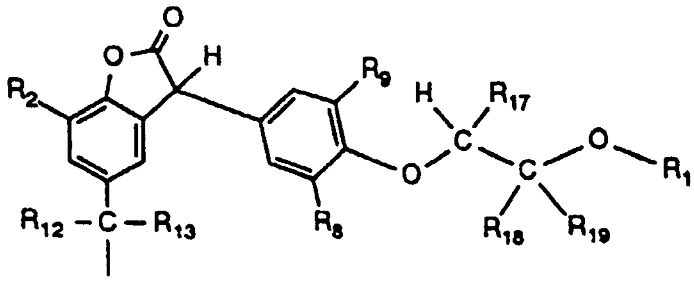

(2)

in which $R_1$ is as defined above for m = 1,
$R_6$ is hydrogen or a radical of the formula (3)

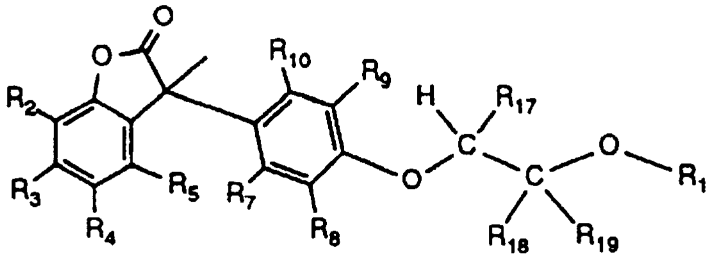

(3)

in which $R_4$ is not a radical of the formula (2) and $R_1$ is as defined above for m = 1,
$R_7$, $R_8$, $R_9$ and $R_{10}$, independently of one another, are hydrogen, $C_1$-$C_4$alkyl or $C_1$-$C_4$alkoxy, on the condition that at least one of the radicals $R_7$, $R_8$, $R_9$ and $R_{10}$ is hydrogen,
$R_{11}$ is hydroxyl,

$$\left[-O^{\ominus}\ \frac{1}{r}M^{r+}\right],$$

$C_1$-$C_{18}$alkoxy or

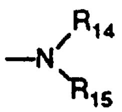

,

$R_{12}$ and $R_{13}$, independently of one another, are hydrogen, $CF_3$, $C_1$-$C_{12}$alkyl or phenyl, or $R_{12}$ and $R_{13}$ together with the C atom to which they are attached form a $C_5$-$C_8$cycloalkylidene ring which is unsubstituted or substituted by 1 to 3 $C_1$-$C_4$alkyl groups;
$R_{14}$ and $R_{15}$, independently of one another, are hydrogen or $C_1$-$C_{18}$alkyl,
$R_{16}$ is hydrogen or $C_1$-$C_8$alkyl,
$R_{17}$ is hydrogen or $C_1$-$C_4$alkyl,
$R_{18}$ is hydrogen, unsubstituted or $C_1$-$C_4$alkyl-substituted phenyl; $C_1$-$C_{25}$alkyl, $C_2$-$C_2$salkyl which is interrupted by oxygen, sulfur or $>N-R_{16}$ , $C_7$-$C_9$phenylalkyl which is unsubstituted or substituted on the phenyl radical by 1 to 3 $C_1$-$C_4$alkyl groups; $C_7$-$C_{25}$phenylalkyl which is interrupted by oxygen, sulfur or $>N-R_{16}$ and unsubstituted or substituted on the phenyl radical by 1 to 3 $C_1$-$C_4$alkyl groups, or, furthermore, the radicals $R_{17}$ and $R_{18}$ together with the carbon atoms to which they are attached form a $C_5$-$C_{12}$cycloalkylene ring which is unsubsti-

tuted or substituted by 1 to 3 $C_1$-$C_4$alkyl groups; or, if $R_6$, $R_{17}$ and $R_{19}$ are hydrogen, $R_4$ is not a radical of the formula (2), m is 1 and $R_1$ is as defined above for m = 1, $R_{18}$ is additionally a radical of the formula (4)

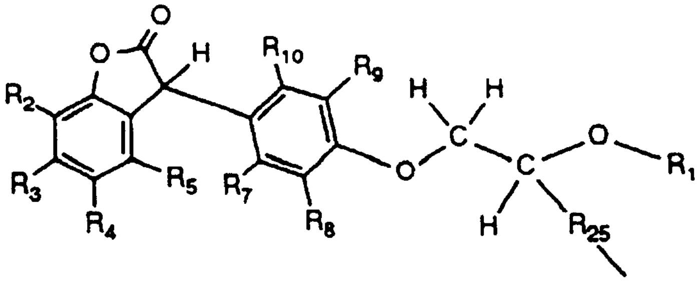

(4)

$R_{19}$ is hydrogen or $C_1$-$C_4$alkyl,
$R_{20}$ is hydrogen or $C_1$-$C_4$alkyl,
$R_{21}$ is a direct bond, $C_1$-$C_{18}$alkylene, $C_2$-$C_{18}$alkylene which is interrupted by oxygen, sulfur or >N-$R_{16}$ ; $C_2$-$C_{18}$alkenylene, $C_2$-$C_{20}$alkylidene, $C_7$-$C_{20}$phenylalkylidene, $C_5$-$C_8$cycloalkylene, $C_7$-$C_8$bicycloalkylene, unsubstituted or $C_1$-$C_4$alkyl-substituted phenylene,

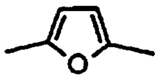

or

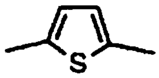

,

$R_{22}$ is oxygen, -NH- or

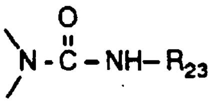

,

$R_{23}$ is $C_1$-$C_{18}$alkyl or phenyl,
$R_{24}$ is $C_2$-$C_{18}$alkylene, $C_5$-$C_8$cycloalkylene or phenylene,
$R_{25}$ is a direct bond, $C_1$-$C_{18}$alkylene or $C_2$-$C_{18}$alkylene which is interrupted by oxygen, sulfur or >N-$R_{16}$,
M is an r-valent metal cation,
m is 1, 2, 3 or 4, $R_6$ being hydrogen if m is 2, 3 or 4;
n is 0, 1 or 2 and
r is 1, 2 or 3.

**2.** A compound according to claim 1, in which, if m is 1,

$R_1$ is hydrogen, $C_1$-$C_{18}$alkanoyl, $C_3$-$C_{18}$alkenoyl, $C_3$-$C_{18}$alkanoyl which is interrupted by oxygen, sulfur or >N-$R_{16}$ ; $C_2$-$C_{18}$alkanoyl which is substituted by a di($C_1$-$C_6$alkyl)-phosphonate group; $C_6$-$C_9$cycloalkylcarbonyl, thenoyl, furoyl, benzoyl or $C_1$-$C_8$alkyl-substituted benzoyl;

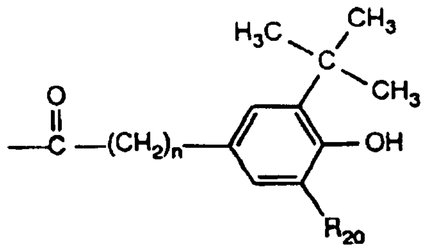,

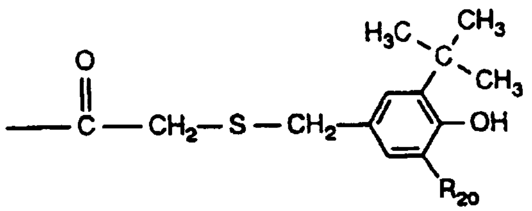, 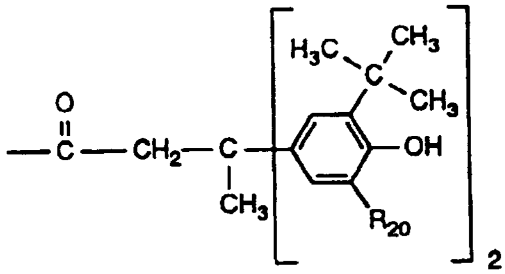,

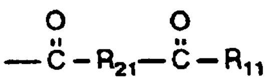 or 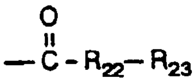,

$R_2$, $R_3$, $R_4$ and $R_5$, independently of one another, are hydrogen, chlorine, $C_1$-$C_{18}$alkyl, benzyl, phenyl, $C_5$-$C_8$cycloalkyl, $C_1$-$C_{18}$-alkoxy, $C_1$-$C_{18}$alkylthio, $C_1$-$C_{18}$alkanoyloxy, $C_1$-$C_{18}$alkanoylamino, $C_3$-$C_{18}$alkenoyloxy or benzoyloxy, or, furthermore, the radicals $R_2$ and $R_3$ or the radicals $R_4$ and $R_5$ together with the carbon atoms to which they are attached form a phenyl ring, or, if $R_3$, $R_5$, $R_6$, $R_7$, and $R_{10}$ are hydrogen, $R_4$ is additionally a radical of the formula (2), $R_7$, $R_8$, $R_9$ and $R_{10}$, independently of one another, are hydrogen or $C_1$-$C_4$alkyl, on the condition that at least one of the radicals $R_7$, $R_8$, $R_9$ and $R_{10}$ is hydrogen, $R_{12}$ and $R_{13}$ are methyl groups or together with the C atom to which they are attached form a $C_5$-$C_8$cycloalkylidene ring which is unsubstituted or subtituted by 1 to 3 $C_1$-$C_4$alkyl groups,

$R_{18}$ is hydrogen, phenyl, $C_1$-$C_{18}$alkyl, $C_2$-$C_{18}$alkyl which is interrupted by oxygen, sulfur or $>N-R_{16}$ ; benzyl, $C_7$-$C_{18}$phenylalkyl which is interrupted by oxygen, sulfur or $>N-R_{16}$ ; or, furthermore, the radicals $R_{17}$ and $R_{18}$ together with the carbon atoms to which they are attached form a $C_5$-$C_8$cycloalkylene ring which is unsubstituted or substituted by 1 to 3 $C_1$-$C_4$alkyl groups,

$R_{21}$ is a direct bond, $C_1$-$C_{12}$alkylene, $C_2$-$C_{12}$alkylene which is interrupted by oxygen, sulfur or $>N-R_{16}$ ; $C_2$-$C_{12}$alkenylene, $C_2$-$C_{12}$alkylidene, $C_7$-$C_{12}$phenylalkylidene, $C_5$-$C_8$cycloalkylene, $C_7$-$C_8$bicycloalkylene or phenylene, $R_{24}$ is $C_2$-$C_{12}$alkylene, $C_5$-$C_8$cycloalkylene or phenylene, and $R_{25}$ is a direct bond, $C_1$-$C_{12}$alkylene or $C_2$-$C_{12}$alkylene which is interrupted by oxygen, sulfur or $>N-R_{16}$.

3. A compound according to claim 1, in which at least two of the radicals $R_2$, $R_3$, $R_4$ and $R_5$ are hydrogen.

4. A compound according to claim 1, in which $R_3$ and $R_5$ are hydrogen.

5. A compound according to claim 1, in which $R_2$, $R_3$, $R_4$ and $R_5$, independently of one another, are hydrogen, chlorine, $C_1$-$C_{18}$alkyl, $C_5$-$C_6$cycloalkyl or $C_1$-$C_4$alkoxy, or, furthermore, the radicals $R_2$ and $R_3$ together with the carbon atoms to which they are attached form a phenyl ring.

6. A compound according to claim 1, in which m is 1 or 2.

7. A compound according to claim 1, in which $R_{18}$ is hydrogen, $C_1$-$C_{12}$alkyl, $C_2$-$C_{12}$alkyl which is interrupted by oxygen or sulfur; $C_7$-$C_{12}$phenylalkyl which is interrupted by oxygen or sulfur, or, furthermore, the radicals $R_{17}$ and $R_{18}$ together with the carbon atoms to which they are attached form a $C_5$-$C_8$cycloalkylene ring.

8. A compound according to claim 1, in which,
if m is 1,

$R_1$ is hydrogen, $C_1$-$C_{18}$alkanoyl, $C_3$-$C_{12}$alkenoyl, $C_3$-$C_{12}$alkenoyl which is interrupted by oxygen; $C_2$-$C_{12}$alkanoyl which is substituted by a di($C_1$-$C_6$-alkyl)phosphonate group; $C_6$-$C_9$cycloalkylcarbonyl, benzoyl,

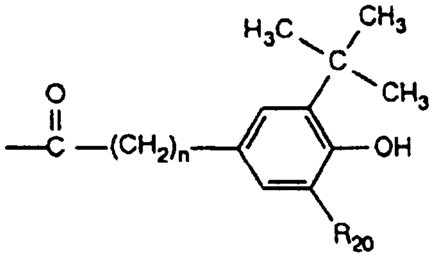
,
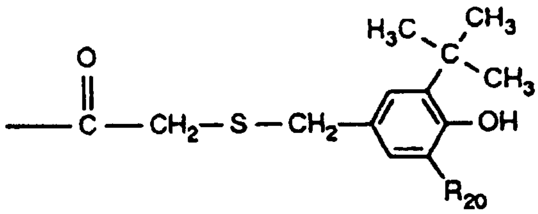
,

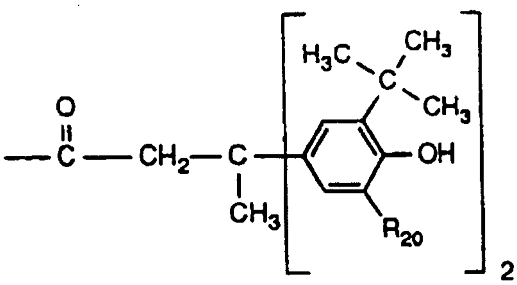
,
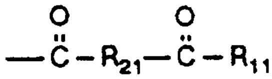

or

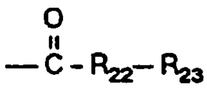
,

$R_2$, $R_3$, $R_4$ and $R_5$ independently of one another, are hydrogen, $C_1$-$C_{18}$alkyl, $C_5$-$C_7$cycloalkyl, $C_1$-$C_{12}$alkoxy, $C_1$-$C_{12}$alkanoyloxy or benzoyloxy, or, furthermore, the radicals $R_2$ and $R_3$ together with the carbon atoms to which they are attached form a phenyl ring, or, if $R_3$, $R_5$, $R_6$, $R_7$ and $R_{10}$ are hydrogen, $R_4$ is additionally a radical of the formula (2),
$R_{12}$ and $R_{13}$ are methyl groups, together with the C atom to which they are attached, form a $C_5$-$C_8$cycloalkylidene ring, $R_{18}$ is hydrogen, $C_1$-$C_{12}$alkyl, $C_2$-$C_{12}$alkyl which is interrupted by oxygen or sulfur; $C_7$-$C_{12}$phenylalkyl which is interrupted by oxygen or sulfur, or, furthermore, the radicals $R_{17}$ and $R_{18}$ together with the carbon atoms to which they are attached form a $C_5$-$C_8$cycloalkylene ring,
$R_{21}$ is $C_1$-$C_{12}$alkylene, phenylene or $C_2$-$C_{12}$alkylene which is interrupted by oxygen or sulfur,
$R_{23}$ is $C_1$-$C_{12}$alkyl,
$R_{24}$ is $C_2$-$C_{12}$alkylene or phenylene,
$R_{25}$ is $C_1$-$C_8$alkylene or $C_2$-$C_8$alkylene which is interrupted by oxygen, and
m is 1, 2 or 3.

**9.** A compound according to claim 1,
in which
if m is 1,

$R_1$ is hydrogen, $C_1$-$C_{18}$alkanoyl, $C_3$-$C_4$alkenoyl, $C_2$-$C_4$alkanoyl which is substituted by a di($C_1$-$C_4$alkyl)phosphonate group; cyclohexylcarbonyl, benzoyl,

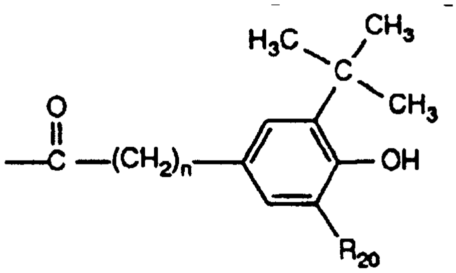 , 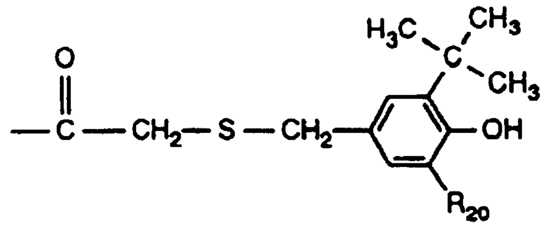

or

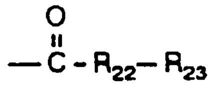 ,

and is m is 2,

$R_1$ is

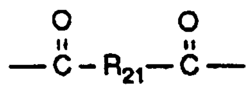

or

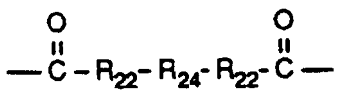 ,

$R_2$ is hydrogen, $C_1$-$C_{18}$alkyl or cyclohexyl,

$R_3$ is hydrogen, or, furthermore, the radicals $R_2$ and $R_3$ together with the carbon atoms to which they are attached form a phenyl ring,

$R_4$ is $C_1$-$C_4$alkyl or cyclohexyl, or, if $R_3$, $R_5$, $R_6$, $R_7$ and $R_{10}$ are hydrogen, $R_4$ is additionally a radical of the formula (2), in which $R_1$ is as defined above for m = 1,

$R_5$ is hydrogen,

$R_6$ is hydrogen or a radical of the formula (3), $R_4$ not being a radical of the formula (2) and $R_1$ being as defined above for m = 1,

$R_7$ is hydrogen,

$R_8$ and $R_9$, independently of one another, are hydrogen, $C_1$-$C_4$alkyl or $C_1$-$C_4$alkoxy,

$R_{10}$ is hydrogen,

$R_{12}$ and $R_{13}$ are methyl groups or, together with the C atom to which they are attached, form a cyclohexylidene ring,

$R_{17}$ is hydrogen,

$R_{18}$ is hydrogen, $C_1$-$C_4$alkyl, $C_2$-$C_8$alkyl which is interrupted by oxygen; or $C_7$-$C_9$phenylalkyl which is interrupted by oxygen, or, furthermore, the radicals $R_{17}$ and $R_{18}$ together with the carbon atoms to which they are attached form a cyclohexylene ring,

$R_{19}$ is hydrogen,

$R_{20}$ is hydrogen or $C_1$-$C_4$alkyl,

$R_{21}$ is $C_1$-$C_8$alkylene, $C_2$-$C_6$alkylene which is interrupted by sulfur; or phenylene,

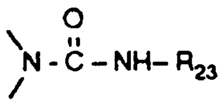

,

$R_{22}$ is -NH- or
$R_{23}$ is $C_1$-$C_4$alkyl,
$R_{24}$ is $C_4$-$C_8$alkylene,
m is 1 or 2, and
n is 0 or 2.

**10.** A composition comprising

a) an organic material subject to oxidative, thermal or light-induced degradation and

b) at least one compound of the formula (1) according to claim 1.

**11.** A composition according to claim 10, in which component a) is a synthetic polymer.

**12.** A composition according to claim 10, which component b) is present in an amount of 0.0005 to 5%, relative to the weight of component a).

**13.** A composition accoridng to claim 10, further comprising an organic phosphite or phosphonite.

**14.** Use of a compound of the formula (1) defined in claim 1 as stabilizer for organic materials against oxidative, thermal or light-induced degradation.

**15.** A process for stabilizing an organic material against oxidative, thermal or light-induced degradation, which comprises incorporating therein or applying thereto at least one compound of the formula (1) defined in claim 1.

**16.** A compound of the formula (9)

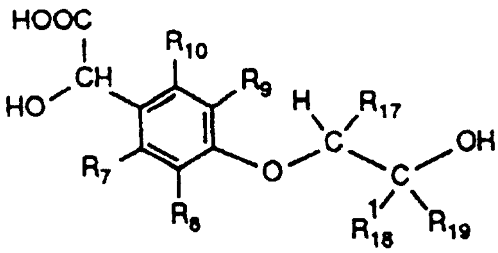

(9)

in which $R_7$, $R_8$, $R_9$ and $R_{10}$, independently of one another, are hydrogen, $C_1$-$C_4$alkyl or $C_1$-$C_4$alkoxy, on the condition that at least one of the radicals $R_7$, $R_8$, $R_9$ and $R_{10}$ is hydrogen and, if $R_7$, $R_8$, $R_9$ and $R_{10}$ are simultaneously hydrogen, either $R_{17}$, $R^{1}_{18}$ or $R_{19}$ is different from hydrogen,

$R_{16}$ is hydrogen or $C_1$-$C_8$alkyl,
$R_{17}$ is hydrogen or $C_1$-$C_4$alkyl,
$R^{1}_{18}$ is hydrogen, unsubstituted or $C_1$-$C_4$alkyl-substituted phenyl; $C_1$-$C_{25}$alkyl, $C_2$-$C_{25}$alkyl which is interrupted by oxygen or sulfur; $C_7$-$C_9$phenylalkyl which is unsubstituted or substituted on the phenyl ring by 1 to 3 $C_1$-$C_4$alkyl groups; $C_7$-$C_{25}$ phenylalkyl which is interrupted by oxygen, sulfur or >N-$R_{16}$ and unsubstituted or substituted on the phenyl ring by 1 to 3 $C_1$-$C_4$alkyl groups, or, furthermore, the radicals $R_{17}$ and $R^{1}_{18}$ together with the carbon atoms to which they are attached form a $C_5$-$C_{12}$cycloalkylene ring which is unsubstituted or substituted by 1 to 3 $C_1$-$C_4$alkyl groups; or, if $R_{17}$ and $R_{19}$ are hydrogen, $R^{1}_{18}$ is additionally a radical of the

formula (10)

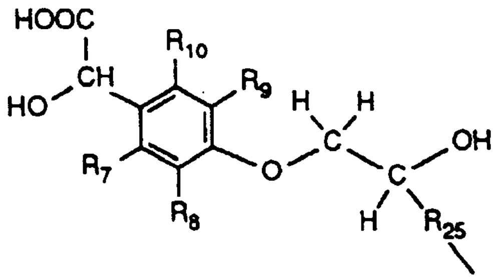

(10)

$R_{19}$ is hydrogen or $C_1$-$C_4$alkyl, and
$R_{25}$ is a direct bond, $C_1$-$C_{18}$alkylene or $C_2$-$C_{18}$alkylene which is interrupted by oxygen, sulfur or >N-$R_{16}$.

**17.** A compound according to claim 16, in which

$R_{18}^{1}$ is hydrogen, phenyl, $C_1$-$C_{18}$alkyl, $C_2$-$C_{18}$alkyl which is interrupted by oxygen or sulfur; benzyl, $C_7$-$C_{18}$phenylalkyl which is interrupted by oxygen, sulfur or >N-$R_{16}$, or, furthermore, the radicals $R_{17}$ and $R_{18}^{1}$ together with the carbon atoms to which they are attached form a $C_5$-$C_8$cycloalkylene ring which is unsubstituted or substituted by 1 to 3 $C_1$-$C_4$alkyl groups, and
$R_{25}$ is a direct bond, $C_1$-$C_{12}$alkylene or $C_2$-$C_{12}$alkylene which is interrupted by oxygen, sulfur or >N-$R_{16}$.

**18.** A compound according to claim 16, in which $R_7$, and $R_{10}$ are hydrogen.

**19.** A compound according to claim 16, in which

$R_{17}$ is hydrogen,
$R_{18}^{1}$ is hydrogen, $C_1$-$C_{12}$alkyl, $C_2$-$C_{12}$alkyl which is interrupted by oxygen or sulfur; $C_7$-$C_{12}$phenylalkyl which is interrupted by oxygen or sulfur, or, furthermore, the radicals $R_{17}$ and
$R_{18}^{1}$ together with the carbon atoms to which they are attached form a $C_5$-$C_8$cycloalkylene ring, and
$R_{25}$ is $C_1$-$C_8$alkylene or $C_2$-$C_8$alkylene which is interrupted by oxygen.

**20.** A compound according to claim 16, in which $R_7$, $R_{10}$, $R_{17}$ and $R_{19}$ are hydrogen, and

$R_{18}^{1}$ is hydrogen, $C_1$-$C_4$alkyl, $C_2$-$C_8$alkyl which is interrupted by oxygen;
$C_7$-$C_9$phenylalkyl which is interrupted by oxygen, or, furthermore, the radicals $R_{17}$ and $R_{18}^{1}$ together with the carbon atoms to which they are attached form a cyclohexylene ring.

**21.** A process for preparing compounds of the formula (6)

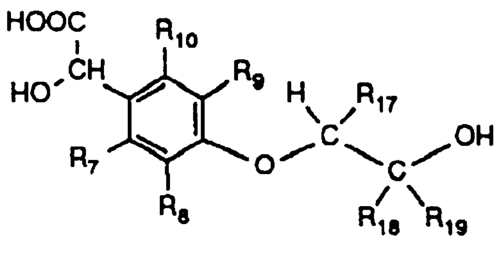

(6)

in which the general symbols are as claimed in claim 1 and, if $R_{17}$ and $R_{19}$ are hydrogen, $R_{18}$ is additionally a radical of the formula (10)

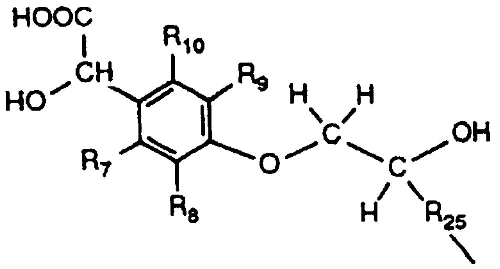 (10)

which comprises reacting a 4-hydroxymandelic acid of the formula (11) with an epoxide of the formula (12),

(11) 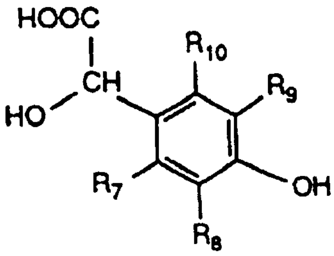 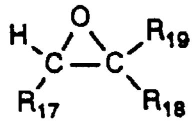 (12)

in which the radicals $R_7$, $R_8$, $R_9$, $R_{10}$, $R_{17}$, $R_{18}$ and $R_{19}$ have the same meaning as in formula (1) in claim 1, and, if $R_{17}$ and $R_{19}$ are hydrogen, $R_{18}$ in formula (12) is additionally a radical of the formula (10) or a radical of the formula (16)

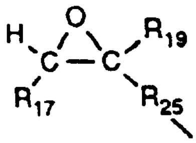 (16)

to give compounds of the formula (6).

**22.** A process according to claim 21, in which $R_{18}$ has the same meaning as in formula (1) in claim 1.

**23.** A process according to claim 21, in which the epoxide is ethylene oxide.

**24.** A process according to claim 21, in which the epoxide is used in a molar excess of 1 to 80%, in particular 10 to 60%, relative to the 4-hydroxymandelic acid of the formula (11) used.

**Revendications**

**1.** Composés de formule (1)

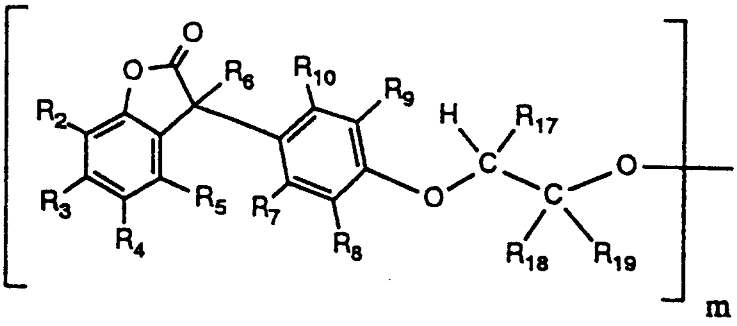

(1)

où, lorsque m vaut 1,

$R_1$ représente un atome d'hydrogène, des groupes alcanoyle en $C_1$-$C_{25}$, alcénoyle en $C_2$-$C_{25}$, alcanoyle en $C_3$-$C_{25}$ interrompu par des atomes d'oxygène, de soufre ou un groupe >N-$R_{16}$ ; alcanoyle en $C_2$-$C_{25}$ substitué par un groupe di-(alkyl en $C_1$-$C_6$)-phosphonato ; cyclo(alkyl en $C_6$-$C_9$)-carbonyle, thénoyle, furoyle, benzoyle ou benzoyle substitué par un substituant alkyle en $C_1$-$C_{12}$ ;

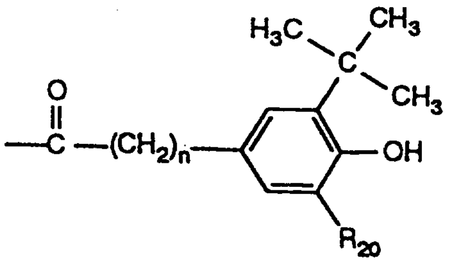 , 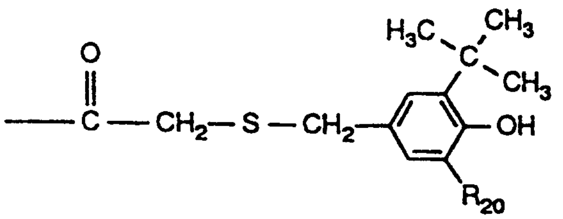 ,

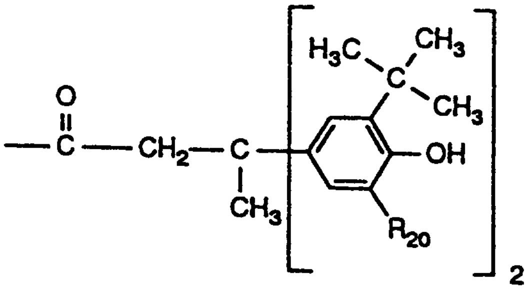 , 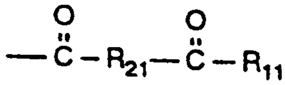

ou

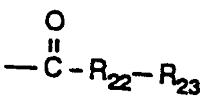 ,

et lorsque m vaut 2,

$R_1$ représente

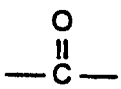 , 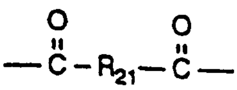

ou

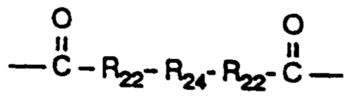

et lorsque m vaut 3

$R_1$ représente des groupes (alcane en $C_4$-$C_{18}$)-tricarbonyle, (aryl en $C_9$-$C_{18}$)tricarbonyle

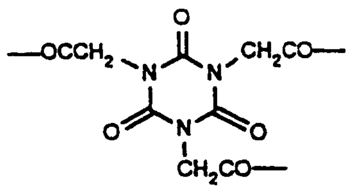

ou

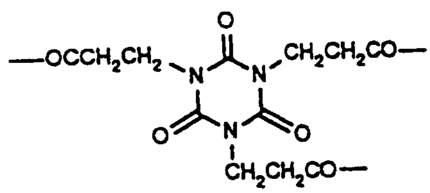

et lorsque m vaut 4,

$R_1$ représente des groupes (alcane en $C_6$-$C_{18}$)-tétracarbonyle ou (aryl en $C_6$-$C_{18}$)tétracarbonyle,

$R_2$, $R_3$, $R_4$ et $R_5$ représentent, indépendamment les uns des autres, des atomes d'hydrogène, de chlore, des groupes alkyle en $C_1$-$C_{25}$, phénylalkyle en $C_7$-$C_9$, phényle non substitué ou substiué par un substituant alkyle en $C_1$-$C_4$, cycloalkyle en $C_5$-$C_8$ non substitué ou substitué par un substituant alkyle en $C_1$-$C_4$ ; alkoxy en $C_1$-$C_{18}$, (alkyle en $C_1$-$C_{18}$)-thio, hydroxy, (alkyle en $C_1$-$C_4$)amino, di(alkyle en $C_1$-$C_4$)amino, (alcanoyl en $C_1$-$C_{25}$)oxy, (alcanoyl en $C_1$-$C_{25}$)amino, (alcénoyl en $C_3$-$C_{25}$)oxy, (alcanoyl en $C_3$-$C_{25}$)oxy interrompu des atomes d'oxygène, de soufre ou un groupe >N-$R_{16}$ ; (cycloalkyl en $C_6$-$C_9$)carbonyloxy, benzoyloxy ou benzoyloxy substitué par un substituant alkyle en $C_1$-$C_{12}$, ou de plus, les restes $R_2$ et $R_3$ ou les restes $R_3$ et $R_4$ ou les restes $R_4$ et $R_5$ foment, conjointement avec les atomes de carbone auxquels ils sont liés, un cycle phényle, $R_4$ représente de plus un groupe (-$CH_2$)$_n$-$COR_{11}$ ou lorsque $R_3$, $R_5$, $R_6$, $R_7$ et $R_{10}$ représentent des atomes d'hydrogène, $R_4$ représente de plus un reste de formule (2)

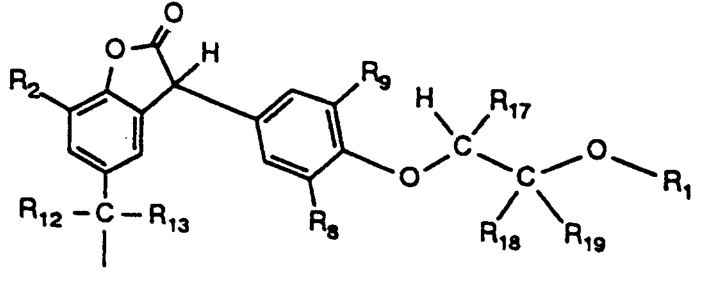

(2)

où $R_1$ est défini comme ci-dessus pour le cas où m = 1

$R_6$ représente un atome d'hydrogène ou un reste de formule (3)

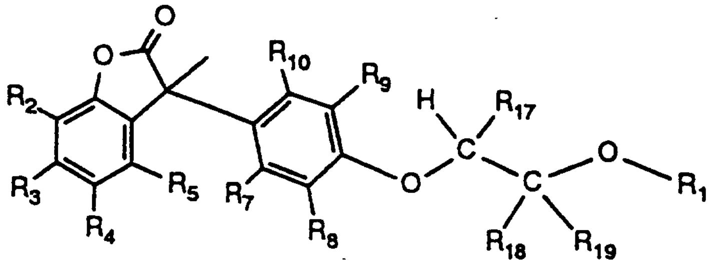

(3)

$R_4$ ne représentant pas un reste de formule (2) et $R_1$ étant défini conne donné ci-dessus pour m = 1,

$R_7$, $R_8$, $R_9$ et $R_{10}$ représentent, indépendamment les uns des autres, des atomes d'hydrogène, des groupes alkyle en $C_1$-$C_4$ ou alkoxy en $C_1$-$C_4$, à condition qu'au moins l'un des restes $R_7$, $R_8$, $R_9$ et $R_{10}$ représente un atome d'hydrogène,

$R_{11}$ représente des groupes hydroxy,

$$\left[-O^{\ominus}\ \frac{1}{r}M^{r+}\right] ,$$

alkoxy en $C_1$-$C_{18}$ ou

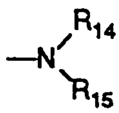

,

$R_{12}$ et $R_{13}$ représentent, indépendamment l'un de l'autre, des atomes d'hydrogène, des groupes $CF_3$, alkyle en $C_1$-$C_{12}$ ou phényle, ou $R_{12}$ et $R_{13}$ forment, conjointement avec l'atome de carbone auquel ils sont liés, un cycle cycloalkylidène en $C_5$-$C_8$ non substitué ou substitué par 1 à 3 substituants alkyle en $C_1$-$C_4$ ;

$R_{14}$ et $R_{15}$ représentent, indépendamment l'un de l'autre, des atomes d'hydrogène ou des groupes alkyle en

$R_{16}$ représente un atome d'hydrogène ou un groupe alkyle en $C_1$-$C_8$,

$R_{17}$ représente un atome d'hydrogène ou un groupe alkyle en $C_1$-$C_4$,

$R_{18}$ représente un atome d'hydrogène ou un groupe phényle non substitué ou substitué par un substituant alkyle en $C_1$-$C_4$; des groupes alkyle $C_1$-$C_{25}$, alkyle $C_2$-$C_{25}$ interrompu par des atomes d'oxygène, de soufre ou un groupe >N-$R_{16}$ ; un groupe phénylalkyle $C_7$-$C_9$ non substitué ou substitué sur le reste phényle par 1 à 3 substituants alkyle $C_1$-$C_4$; un groupe phénylalkyle $C_7$-$C_{25}$ interrompu par des atomes d'oxygène, de soufre ou un groupe >N-$R_{16}$, non substitué ou substitué sur le reste phényle par 1 à 3 substituants alkyle $C_1$-$C_4$ ou, de plus, les restes $R_{17}$ et $R_{18}$ forment, conjointement avec les atomes de carbone auxquels ils sont liés, un cycle cycloalkylène en $C_5$-$C_{12}$ non substitué ou substitué par 1 à 3 substituants alkyle en $C_1$-$C_4$ ; ou lorsque $R_6$, $R_{17}$ et $R_{19}$ représentent des atomes d'hydrogène, $R_4$ ne représente par le reste de formule (2), m vaut 1 et $R_1$ est défini conne donné ci-dessus pour m = 1, $R_{18}$ représente, de plus, un reste de formule (4)

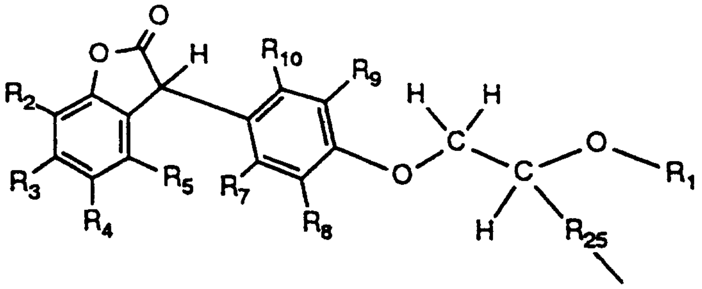

(4)

| | |
|---|---|
| $R_{19}$ | représente un atome d'hydrogène ou un groupe alkyle en $C_1$-$C_4$, |
| $R_{20}$ | représente un atome d'hydrogène ou un groupe alkyle en $C_1$-$C_4$, |
| $R_{21}$ | représente une liaison directe, des groupes alkylène en $C_1$-$C_{18}$, alkylène en $C_2$-$C_{18}$ interrompu par des atomes d'oxygène, de soufre ou un groupe >N-$R_{16}$ ; alkylène en $C_2$-$C_{18}$, alkylidène en $C_2$-$C_{20}$, phénylalkylidène en $C_7$-$C_{20}$, cycloalkylène en $C_5$-$C_8$, bicycloalkylène en $C_7$-$C_8$, phénylène non substitué ou substitué par un groupe alkyle en $C_1$-$C_4$, |

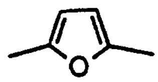

ou

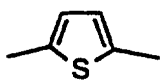

,

| | |
|---|---|
| $R_{22}$ | représente un atome d'oxygène, des groupes -NH- ou |

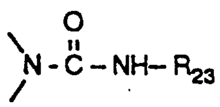

,

| | |
|---|---|
| $R_{23}$ | représente des groupes alkyle en $C_1$-$C_{18}$ ou phényle, |
| $R_{24}$ | représente des groupes alkylène en $C_2$-$C_{18}$, cycloalkylène en $C_5$-$C_8$ ou phénylène, |
| $R_{25}$ | représente une liaison directe, des groupes alkylène en $C_1$-$C_{18}$ ou alkylène en $C_2$-$C_{18}$ interrompu par des atomes d'oxygène de soufre ou un groupe |
| M | représente un cation métallique à fonctionnalité r, |
| m | vaut 1, 2, 3 ou 4, où, lorsque m vaut 2, 3 ou 4, $R_6$ représente un atome d'hydrogène ; |
| n | vaut 0, 1 ou 2 et |
| r | vaut 1, 2 ou 3. |

**2.** Composés selon la revendication 1, où lorsque m vaut 1

| | |
|---|---|
| $R_1$ | représente un atome d'hydrogène, des groupes alcanoyle en $C_1$-$C_{18}$, alcénoyle en $C_3$-$C_{18}$, alcanoyle en $C_3$-$C_{18}$ interrompu par des atomes d'oxygène, de soufre ou un groupe >N-$R_{16}$ ; alcanoyle en $C_2$-$C_{18}$ substitué par un groupe di(alkyl en $C_1$-$C_6$)-phosphonato ; (cycloalkyl en $C_6$-$C_9$)carbonyle, thénoyle, furoyle, benzoyle ou benzoyle substitué par des substituants alkyle en $C_1$-$C_{18}$ ; |

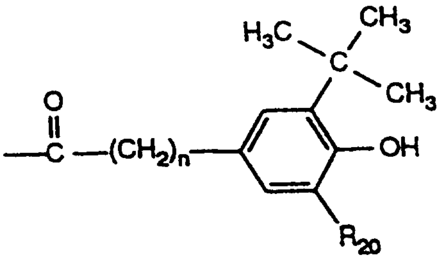 , 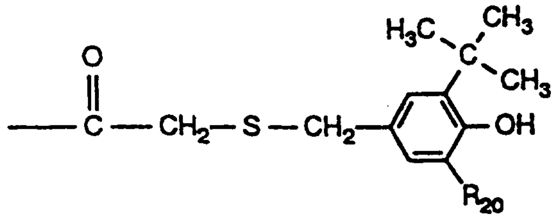 ,

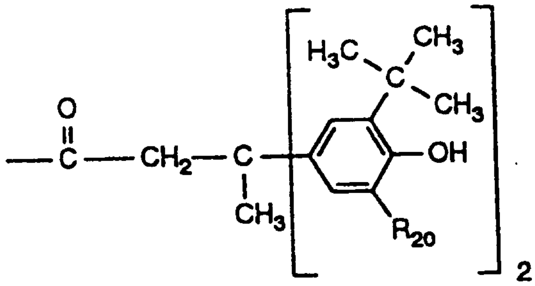 , 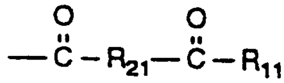

ou

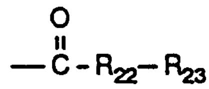 ,

et

$R_2$, $R_3$, $R_4$ et $R_5$ représentent, indépendamment les uns des autres, des atomes d'hydrogène, de chlore, des groupes alkyle en $C_1$-$C_{18}$, benzyle, phényle, cycloalkyle en $C_5$-$C_8$, alkoxy en $C_1$-$C_{18}$, (alkyl en $C_1$-$C_{18}$)thio, (alcanoyl en $C_1$-$C_{18}$)oxy, (alcanoyl en $C_1$-$C_{18}$)amino, (alcénoyl en $C_3$-$C_{18}$)oxy ou benzoyloxy, ou de plus, les restes $R_2$ et $R_3$ ou les restes $R_4$ et $R_5$ forment, conjointement avec les atomes de carbone auxquels ils sont liés, un cycle phényle, ou lorsque $R_3$, $R_5$, $R_6$, $R_7$ et $R_{10}$ représentent des atomes d'hydrogène, $R_4$ représente de plus un reste de formule (2),

$R_7$, $R_8$, $R_9$ et $R_{10}$ représentent, indépendamment les uns des autres, des atomes d'hydrogène ou des groupes alkyle en $C_1$-$C_4$, à condition qu'au moins l'un des restes $R_7$, $R_8$, $R_9$ et $R_{10}$ représente un atome d'hydrogène,

$R_{12}$ et $R_{13}$ représentent des groupes méthyle ou forment, conjointement avec l'atome de carbone auquel ils sont liés, un cycle cycloalkylidène en $C_5$-$C_8$ non substitué ou substitué par 1 à 3 substituants alkyle en $C_1$-$C_4$,

$R_{18}$ représente un atome d'hydrogène, des groupes phényle, alkyle en $C_1$-$C_{18}$, alkyle en $C_2$-$C_{18}$ interrompu par des atomes d'oxygène, de soufre ou un groupe $>N-R_{16}$ ; benzyle, phénylalkyle en $C_7$-$C_{18}$ interrompu par des atomes d'oxygène, de soufre ou un groupe $>N-R_{16}$, ou de plus les restes $R_{17}$ et $R_{18}$ forment, conjointement avec les atomes de carbone auxquels ils sont liés, un cycle cycloalkylidène en $C_5$-$C_8$ non substitué ou substitué par 1 à 3 substituants alkyle en $C_1$-$C_4$,

$R_{21}$ représente une liaison directe, des groupes alkylène en $C_1$-$C_{12}$, alkylène en $C_2$-$C_{12}$ interrompu par des atomes d'oxygène, de soufre ou un groupe $>N-R_{16}$ ; alcénylène en $C_2$-$C_{12}$, alkylidène en $C_2$-$C_{12}$, phénylalkylidène en $C_7$-$C_{12}$, cycloalkylène en $C_5$-$C_8$, bicycloalkylène en $C_7$-$C_8$ ou phénylène,

$R_{24}$ représente des groupes alkylène en $C_2$-$C_{12}$, cycloalkylène en $C_5$-$C_8$ ou phénylène, et

$R_{25}$ représente une liaison directe, des groupes alkylène en $C_1$-$C_{12}$ ou alkylène en $C_2$-$C_{12}$ interrompu par des atomes d'oxygène, de soufre ou un groupe $>N-R_{16}$.

**3.** Composés selon la revendication 1, où au moins deux des restes $R_2$, $R_3$, $R_4$ et $R_5$ représentent des atomes d'hydrogène.

**4.** Composés selon la revendication 1, où $R_3$ et $R_5$ représentent des atomes d'hydrogène.

**5.** Composés selon la revendication 1, où $R_2$, $R_3$, $R_4$ et $R_5$ représentent, indépendamment les uns des autres, des atomes d'hydrogène, de chlore, des groupes alkyle en $C_1$-$C_{18}$, cycloalkyle en $C_5$-$C_6$ ou alkoxy en $C_1$-$C_4$, ou de plus les restes $R_2$ et $R_3$ forment, conjointement avec les atomes de carbone auxquels ils sont liés, un cycle phényle.

**6.** Composés selon la revendication 1, où m vaut 1 ou 2.

**7.** Composés selon la revendication 1, où $R_{18}$ représente un atome d'hydrogène, des groupes alkyle en $C_1$-$C_{12}$, alkyle en $C_2$-$C_{12}$ interrompu par des atomes d'oxygène ou de soufre ; phénylalkyle en $C_7$-$C_{12}$ interrompu par des atomes d'oxygène ou de soufre, ou de plus les restes $R_{17}$ et $R_{18}$ forment, conjointement avec les atomes de carbone auxquels ils sont liés, un cycle cycloalkylène en $C_5$-$C_8$.

**8.** Composés selon la revendication 1, où lorsque m vaut 1,

$R_1$ représente un atome d'hydrogène, des groupes alcanoyle en $C_1$-$C_{18}$, alcénoyle en $C_3$-$C_{12}$, alcanoyle en $C_3$-$C_{12}$ interrompu par des atomes d'oxygène ; alcanoyle en $C_2$-$C_{12}$ substitué par un groupe di(alkyl en $C_1$-$C_6$)-phosphonato ; cyclo(alkyl en $C_6$-$C_9$)carbonyle, benzoyle,

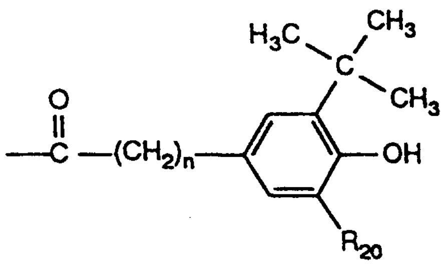 , 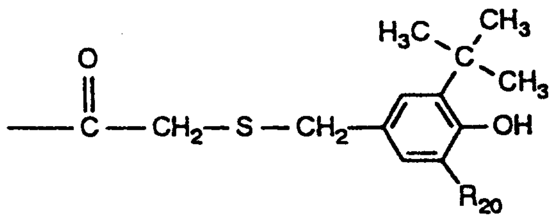 ,

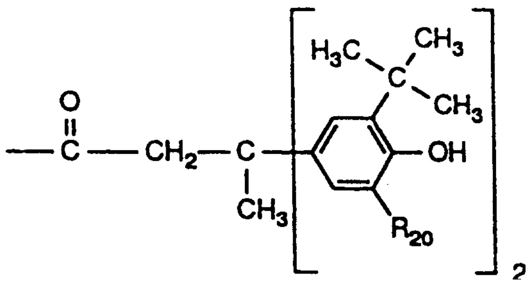 , 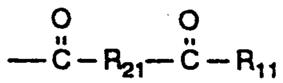

ou

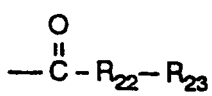

$R_2$, $R_3$, $R_4$ et $R_5$ représentent, indépendamment les uns des autres, des atomes d'hydrogène, des groupes alkyle en $C_1$-$C_{18}$, cycloalkyle en $C_5$-$C_7$, alkoxy en $C_1$-$C_{12}$, alcanoyloxy en $C_1$-$C_{12}$ ou benzoyloxy, ou de plus les restes $R_2$ et $R_3$ forment, conjointement avec les atomes de carbone auxquels ils sont liés, un cycle phényle, ou lorsque $R_3$, $R_5$, $R_6$, $R_7$ et $R_{10}$ représentent des atomes d'hydrogène, $R_4$ représente de plus un reste de formule (2),

$R_{12}$ et $R_{13}$ représentent des groupes méthyle ou forment, conjointement avec l'atome de carbone auquel ils sont liés, un cycle cycloalkylidène en $C_5$-$C_8$,

$R_{18}$ représente un atome d'hydrogène, des groupes alkyle en $C_1$-$C_{12}$, alkyle en $C_2$-$C_{12}$ interrompu

par des atomes d'oxygène ou de soufre ; phénylalkyle en $C_7$-$C_{12}$ interrompu par des atomes d'oxygène ou de soufre, ou de plus, les restes $R_{17}$ et $R_{18}$ forment, conjointement avec les atomes de carbone qui les relient, un cycle cycloalkylène en $C_5$-$C_8$,

$R_{21}$ représente des groupes alkylène en $C_1$-$C_{12}$, phénylène ou alkylène en $C_2$-$C_{12}$ interrompu par des atomes d'oxygène ou de soufre,

$R_{23}$ représente un groupe alkyle en $C_1$-$C_{12}$,

$R_{24}$ représente un groupe alkylène en $C_2$-$C_{12}$ ou phénylène,

$R_{25}$ représente un groupe alkylène en $C_1$-$C_8$, alkylène en $C_2$-$C_8$ interrompu par des atomes d'oxygène, et

m vaut 1, 2 ou 3.

9. Composés selon la revendication 1, où lorsque m vaut 1,

$R_1$ représente un atome d'hydrogène, des groupes alcanoyle en $C_1$-$C_{18}$, alcénoyle en $C_3$-$C_4$, alcanoyle en $C_2$-$C_4$ substitué par un groupe di-(alkyl en $C_1$-$C_6$)phosphonato ; cyclohexylcarbonyle, benzoyle,

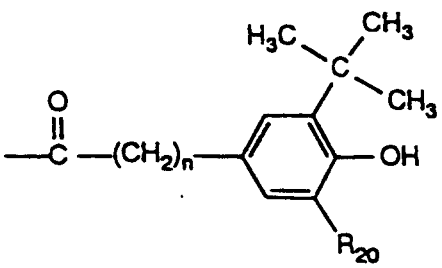
,
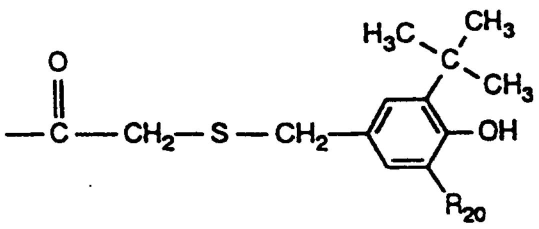

ou

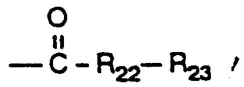

lorsque m vaut 2

$R_1$ représente

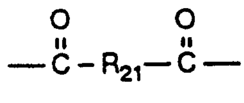

ou

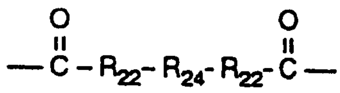

$R_2$ représente un atome d'hydrogène, des groupes alkyle en $C_1$-$C_{18}$ ou cyclohexyle,

$R_3$ représente un atome d'hydrogène, ou de plus les restes $R_2$ et $R_3$ forment, conjointement avec les atomes de carbone auxquels ils sont liés, un cycle phényle,

$R_4$ représente des groupes alkyle en $C_1$-$C_4$ ou cyclohexyle, ou lorsque $R_3$, $R_5$, $R_6$, $R_7$ et $R_{10}$ représentent des atomes d'hydrogène, $R_4$ représente de plus un reste de formule (2), où $R_1$ possède la définition donnée ci-dessus pour m = 1,

$R_5$ représente un atome d'hydrogène,

$R_6$ représente un atome d'hydrogène ou un reste de formule (3), où $R_4$ ne représente pas un reste de formule (2) et $R_1$ possède la définition donnée ci-dessus pour m = 1,

$R_7$ représente un atome d'hydrogène,

$R_8$ et $R_9$ représentent, indépendamment l'un de l'autre, des atomes d'hydrogène, des groupes alkyle en $C_1$-$C_4$ ou alkoxy en $C_1$-$C_4$,

$R_{10}$ représente un atome d'hydrogène,

$R_{12}$ et $R_{13}$ représentent des groupes méthyle ou forment, conjointement avec l'atome de carbone qui les relie, un cycle cyclohexylidène,

$R_{17}$ représente un atome d'hydrogène,

$R_{18}$ représente un atome d'hydrogène, des groupes alkyle en $C_1$-$C_4$, alkyle en $C_2$-$C_8$ interrompu par des atomes d'oxygène ; ou phénylalkyle en $C_7$-$C_9$ interrompu par des atomes d'oxygène, ou de plus, les restes $R_{17}$ et $R_{18}$ forment, conjointement avec les atomes de carbone qui les relient, un cycle cyclohexylène,

$R_{19}$ représente un atome d'hydrogène,

$R_{20}$ représente un atome d'hydrogène ou un groupe alkyle en $C_1$-$C_4$,

$R_{21}$ représente des groupes alkylène en $C_1$-$C_8$, alkylène en $C_2$-$C_6$ interrompu par des atomes d'oxygène ; ou phénylalkyle en $C_7$-$C_9$ interrompu par des atomes de soufre ; ou phénylène,

$R_{22}$ représente des groupes -NH- ou

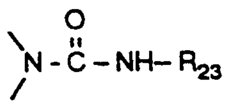 ,

$R_{23}$ représente un groupe alkyle en $C_1$-$C_4$,

$R_{24}$ représente un groupe alkylène en $C_4$-$C_8$, et

m vaut 1 ou 2,

n vaut 0 ou 2.

**10.** Composition contenant

a) une matière organique soumise à la dégradation oxydative, thermique ou induite par la lumière et
b) au moins un composé de formule (1) selon la revendication 1.

**11.** Composition selon la revendication 10, où le constituant a) est un polymère synthétique.

**12.** Composition selon la revendication 10, où le constituant b) est présent en une quantité de 0,0005 à 5 % par rapport aux poids du constituant a).

**13.** Composition selon la revendication 10 contenant de plus un phosphite ou phosphonite organique.

**14.** Utilisation des composés de formule (1) définis à la revendication 1, en tant que stabilisants de matières organiques contre la dégradation oxydative, thermique ou induite par la lumière.

**15.** Procédé pour la stabilisation d'une matière organique contre la dégradation oxydative, thermique ou induite par la lumière, caractérisé en ce qu'on incorpore à celle-ci ou applique sur celle-ci au moins un composé de formule (1) définie à la revendication 1.

**16.** Composés de formule (9)

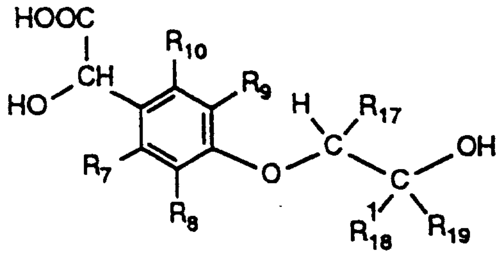

(9)

où

$R_7$, $R_8$, $R_9$ et $R_{10}$ représentent, indépendamment les uns des autres, des atomes d'hydrogène, des groupes alkyle en $C_1$-$C_4$ ou alkoxy en $C_1$-$C_4$, à condition qu'au moins l'un des restes $R_7$, $R_8$, $R_9$ et $R_{10}$ soit un atome d'hydrogène et, lorsque $R_7$, $R_8$, $R_9$ et $R_{10}$ représentent simultanément un atome d'hydrogène, soit $R_{17}$, $R^1_{18}$ , soit $R_{19}$ soient différents d'un atome d'hydrogène,

$R_{16}$ représente un atome d'hydrogène ou un groupe alkyle en $C_1$-$C_8$,

$R_{17}$ représente un atome d'hydrogène ou un groupe alkyle en $C_1$-$C_4$,

$R^1_{18}$ représente un atome d'hydrogène ou un groupe phényle substitué par un substituant alkyle en $C_1$-$C_4$ ; des groupes alkyle en $C_1$-$C_{25}$, alkyle en $C_2$-$C_{25}$ interrompu par des atomes d'oxygène ou de soufre ; phénylalkyle en $C_7$-$C_9$ non substitué ou substitué sur le cycle phényle par 1 à 3 substituants alkyle en $C_1$-$C_4$ ; phénylalkyle en $C_7$-$C_{25}$ interrompu par des atomes d'oxygène, de soufre ou un groupe $>$N-$R_{16}$, non substitué ou substitué sur le cycle phényle par 1 à 3 substituants alkyle en $C_1$-$C_4$, ou de plus les restes $R_{17}$ et $R^1_{18}$ forment, conjointement avec les atomes de carbone auxquels ils sont liés, un cycle cycloalkylidène en $C_5$-$C_{12}$ non substitué ou substitué par 1 à 3 groupes alkyle en $C_1$-$C_4$ ; ou lorsque $R_{17}$ et $R_{19}$ représentent un atome d'hydrogène, $R^1_{18}$ représente de plus un reste de formule (10)

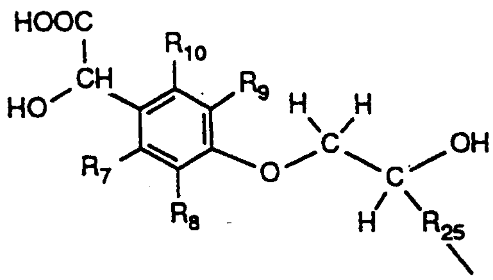

(10)

$R_{19}$ représente un atome d'hydrogène ou un groupe alkyle en $C_1$-$C_4$,

$R_{25}$ représente une liaison directe, des groupes alkylène en $C_1$-$C_{18}$ ou alkylène en $C_2$-$C_{18}$ interrompu par des atomes d'oxygène de soufre ou un groupe $>$N-$R_{16}$.

**17.** Composés selon la revendication 16, où

$R^1_{18}$ représente un atome d'hydrogène des groupes phényle, alkyle en $C_1$-$C_{18}$, alkyle en $C_2$-$C_{18}$ interrompu par des atomes d'oxygène ou de soufre ; benzyle, phénylalkyle en $C_7$-$C_{18}$ interrompu par des atomes d'oxygène, de soufre ou un groupe $>$N-$R_{16}$, ou de plus les restes $R_{17}$ et $R^1_{18}$ forment, conjointement avec les atomes de carbone auxquels ils sont liés, un cycle cycloalkylène en $C_5$-$C_8$ non substitué ou substitué par 1 à 3 groupes alkyle en $C_1$-$C_4$, et

$R_{25}$ représente une liaison directe, des groupes alkylène en $C_1$-$C_{12}$ ou alkylène en $C_2$-$C_{12}$ interrompu par des atomes d'oxygène, de soufre ou un groupe $>$N-$R_{16}$.

**18.** Composé selon la revendication 16, où $R_7$ et $R_{10}$ représentent des atomes d'hydrogène.

**19.** Composé selon la revendication 16, où

$R_{17}$ représente un atome d'hydrogène,

$R^1_{18}$ représente un atome d'hydrogène des groupes alkyle en $C_1$-$C_{12}$, alkyle en $C_2$-$C_{12}$ interrompu par des atomes d'oxygène ou de soufre ; phénylalkyle en $C_7$-$C_{12}$ interrompu par des atomes d'oxygène ou de soufre, ou de plus les restes $R_{17}$ et $R^1_{18}$ forment, conjointement avec les atomes de carbone auxquels ils sont liés, un cycle cycloalkylène en $C_5$-$C_8$, et

$R_{25}$ représente des groupes alkylène en $C_1$-$C_8$ ou alkylène en $C_2$-$C_8$ interrompu par des atomes d'oxygène.

**20.** Composé selon la revendication 16, où

$R_7$, $R_{10}$, $R_{17}$ et $R_{19}$ représentent des atomes d'hydrogène, et

$R^1_{18}$ représente un atome d'hydrogène, des groupes alkyle en $C_1$-$C_4$, alkyle en $C_2$-$C_8$ interrompu par des atomes d'oxygène ; phénylalkyle en $C_7$-$C_9$ interrompu par des atomes d'oxygène, ou de plus les restes $R_{17}$ et $R^1_{18}$ forment, conjointement avec les atomes de carbone auxquels ils sont liés, un cycle cyclohexylène.

**21.** Procédé de préparation de composés de formule (6)

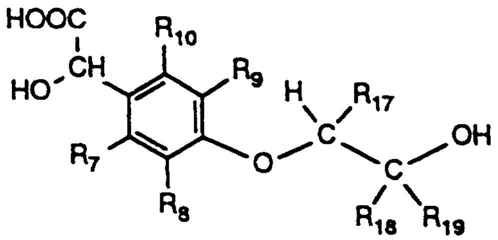

(6)

où les symboles généraux sont définis comme à la formule (1) et, lorsque $R_{17}$ et $R_{19}$ représentent un atome d'hydrogène, $R_{18}$ représente de plus un reste de formule (10)

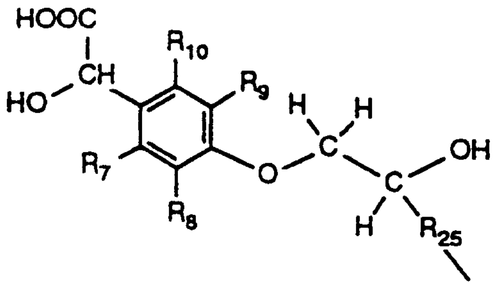

(10),

caractérisé en ce qu'on fait réagir un acide 4-hydroxymandélique de formule (11) sur un époxyde de formule (12),

(11)

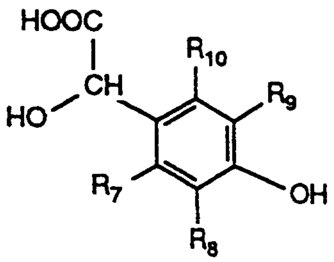

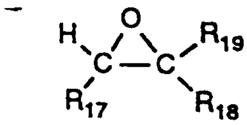

(12),

où les restes $R_7$, $R_8$, $R_9$, $R_{10}$, $R_{17}$, $R_{18}$ et $R_{19}$ possèdent la même signification que celle décrite à la formule (1), et

lorsque $R_{17}$ et $R_{19}$ représentent un atome d'hydrogène $R_{18}$ à la formule (12) représente de plus un reste de formule (10) ou un reste de formule (16)

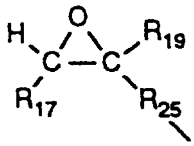

(16),

pour obtenir des composés de formule (6).

**22.** Procédé selon la revendication 21, où $R_{18}$ possède la même signification que celle donnée pour la formule (1) à la revendication 1.

**23.** Procédé selon la revendication 21, où l'époxyde est l'oxyde d'éthylène.

**24.** Procédé selon la revendication 21, où l'on utilise l'époxyde en un excès molaire de 1 à 80 %, en particulier de 10 à 60 %, par rapport à l'acide 4-hydroxymandélique de formule (11).